(19) Europäisches Patentamt
European Patent Office
Office européen des brevets

(11) Veröffentlichungsnummer: **0 384 211 B1**

# (12) EUROPÄISCHE PATENTSCHRIFT

(45) Veröffentlichungstag der Patentschrift: **07.09.94**

(21) Anmeldenummer: **90102336.6**

(22) Anmeldetag: **07.02.90**

(51) Int. Cl.$^5$: **C07D 277/38**, C07D 263/34, A01N 43/74, C07D 277/32, C07D 277/56

## (54) Substituierte Acrylsäureester.

(30) Priorität: **20.02.89 DE 3905119**

(43) Veröffentlichungstag der Anmeldung:
**29.08.90 Patentblatt 90/35**

(45) Bekanntmachung des Hinweises auf die Patenterteilung:
**07.09.94 Patentblatt 94/36**

(84) Benannte Vertragsstaaten:
**BE CH DE DK ES FR GB IT LI NL**

(56) Entgegenhaltungen:
**EP-A- 0 206 523**
**EP-A- 0 331 966**

**JOURNAL OF THE CHEMICAL SOCIETY, PERKIN TRANSACTIONS I, 1986, Seiten 39-59, London, GB; D.H.R. BARTON et al.: "The invention of new radical chain reactions. Part 9. Further radical chemistry of thiohydroxamic esters; formation of carbon-carbon bonds"**

(73) Patentinhaber: **BAYER AG**

**D-51368 Leverkusen (DE)**

(72) Erfinder: **Klausener, Alexander, Dr.**
**Bendenstrasse 16**
**D-5190 Stolberg (DE)**
Erfinder: **Kleefeld, Gerd, Dr.**
**Otto-Hahn-Strasse 87**
**D-4000 Düsseldorf 13 (DE)**
Erfinder: **Brandes, Wilhelm, Dr.**
**Eichendorffstrasse 3**
**D-5653 Leichlingen (DE)**
Erfinder: **Dutzmann, Stefan, Dr.**
**Leinenweberweg 33**
**D-4000 Düsseldorf 13 (DE)**
Erfinder: **Hänssler, Gerd, Dr.**
**Am Arenzberg 58a**
**D-5090 Leverkusen 3 (DE)**

EP 0 384 211 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte Acrylsäureester, mehrere Verfahren zu ihrer Herstellung, ihre Verwendung in Schädlingsbekämpfungsmittel und neue Zwischenprodukte.

Es ist bekannt, daß bestimmte substituierte Acrylsäureester, wie beispielsweise die Verbindung 3-Methoxy-2-(2-methylphenyl)-acrylsäuremethylester fungizide Eigenschaften besitzen (vgl. z.B. EP 178 826).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte Acrylsäureester der allgemeinen Formel (I),

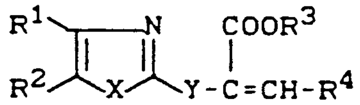

(I)

in welcher

$R^1$ für Wasserstoff, für geradkettiges oder verzweigtes Alkyl mit 1 bis 8 Kohlenstoffatomen, für geradkettiges oder verzweigtes Alkenyl mit 2 bis 8 Kohlenstoffatomen, für jeweils gegebenenfalls im Arylteil einfach bis mehrfach, gleich oder verschieden substituiertes Aralkyl mit 1 bis 6 Kohlenstoffatomen im geradkettigen oder verzweigten Alkylteil, Aralkenyl mit 2 bis 6 Kohlenstoffatomen im geradkettigen oder verzweigten Alkenylteil oder Aryl mit 6 bis 10 Kohlenstoffatomen im jeweiligen Arylteil steht, wobei als Arylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach bis mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen, - insbesondere Stickstoff, Sauerstoff und/oder Schwefel - im Heteroarylteil und gegebenenfalls 1 bis 4 Kohlenstoffatomen im geradkettigen oder verzweigten Alkylteil;

$R^1$ außerdem für einen gegebenenfalls einfach bis mehrfach, gleich oder verschieden substituierten Heteroarylrest mit 2 bis 9 Kohlenstoffatomen und 1 bis 4 gleichen oder verschiedenen Heteroatomen - insbesondere Stickstoff, Sauerstoff und/oder Schwefel- steht, wobei als Substituenten die oben genannten Arylsubstituenten infrage kommen,

$R^2$ für Fluor, Chlor, Brom, Iod, Cyano, Nitro, Formyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor, Brom oder Jod, für jeweils geradkettiges oder verzweigtes Alkoxyalkyl oder Alkylthioalkyl mit jeweils 1 bis 4 Kohlenstoffatomen in den einzelnen Alkylteilen, für jeweils geradkettiges oder verzweigtes Hydroximinoalkyl, Alkoximinoalkyl, N-Alkyliminoalkyl oder N,N-Dialkylhydrazonoalkyl mit jeweils 1 bis 4 Kohlenstoffatomen in den einzelnen Alkylteilen, für jeweils geradkettiges oder verzweigtes Alkoxy oder Alkylthio mit jeweils 1 bis 4 Kohlenstoffatomen, für jeweils geradkettiges oder verzweigtes Halogenalkoxy oder Halogenalkylthio mit jeweils 1 bis 4 Kohlenstoffatomen und jeweils 1 bis 9 gleichen oder verschiedenen Halogenatomen, insbesondere Fluor, Chlor oder Brom, für jeweils geradkettiges oder verzweigtes Alkanoyl, Alkoxycarbonyl oder N,N-Dialkylcarbamoyl mit jeweils 1 bis 4 Kohlenstoffatomen in den einzelnen Alkylteilen oder für Heterocyclylcarbonyl steht, wobei als Heterocyclylrest ein gesättigter fünf- bis siebengliedriger N-verknüpfter Heterocyclus infrage kommt, der gegebenenfalls ein weiteres

Heteroatom, insbesondere Sauerstoff, Schwefel oder Stickstoff, enthalten kann und der gegebenenfalls ein- bis vierfach durch Methyl und/oder Ethyl substituiert sein kann;

$R^2$ außerdem für jeweils gegebenenfalls einfach bis mehrfach, gleich oder verschieden im Arylteil mit jeweils 6 bis 10 Kohlenstoffatomen substituiertes N-Aryliminoalkyl, Aryloxy oder Arylthio steht, wobei der geradkettige oder verzweigte Alkylteil 1 bis 4 Kohlenstoffatome hat und wobei als Arylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen und/oder geradkettiges oder verzweigtes Alkyl mit 1 bis 4 Kohlenstoffatomen substituiertes Phenyl,

$R^3$ für geradkettiges oder verzweigtes Alkyl mit 1 bis 6 Kohlenstoffatomen oder für gegebenenfalls im Arylteil einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ genannten infrage kommen,

$R^4$ für Dialkylamino mit jeweils 1 bis 6 Kohlenstoffatomen in den einzelnen geradkettigen oder verzweigten Alkylteilen steht oder für einen Rest -Z-$R^5$ steht,

X für Sauerstoff oder Schwefel steht und

Y für Sauerstoff, Schwefel oder für einen Rest

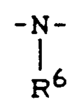

steht,

wobei

$R^5$ für geradkettiges oder verzweigtes Alkyl mit 1 bis 6 Kohlenstoffatomen oder für gegebenenfalls im Arylteil einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ genannten infrage kommen,

$R^6$ für Wasserstoff, für geradkettiges oder verzweigtes Alkyl mit 1 bis 6 Kohlenstoffatomen, für geradkettiges oder verzweigtes Alkanoyl mit 1 bis 6 Kohlenstoffatomen im Alkylteil oder für jeweils gegebenenfalls im Arylteil einfach bis mehrfach, gleich oder verschieden substituiertes Aralkyl mit 1 bis 6 Kohlenstoffatomen im geradkettigen oder verzweigten Alkylteil oder Aryl mit jeweils 6 bis 10 Kohlenstoffatomen im jeweiligen Arylteil steht, wobei als Substituenten im Arylteil jeweils die bei $R^1$ genannten infrage kommen und

Z für Sauerstoff oder Schwefel steht,

gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Acrylsäureester der allgemeinen Formel (I),

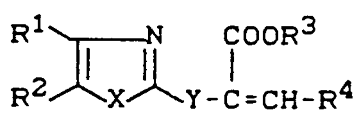

(I)

in welcher

$R^1$, $R^2$, $R^3$, $R^4$, X und Y die oben angegebene Bedeutung haben

nach einem der im Folgenden beschriebenen Verfahren erhält:

(a) Man erhält substituierte Acrylsäureester der Formel (Ia),

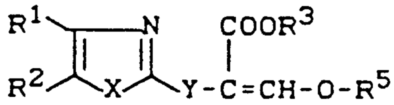

(Ia)

in welcher
$R^1$, $R^2$, $R^3$, $R^5$, X und Y die oben angegebene Bedeutung haben,
wenn man Hydroxyacrylsäureester oder deren Alkalimetallsalze der Formel (II),

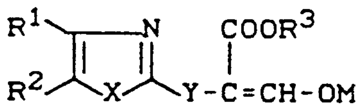

(II)

in welcher
M für Wasserstoff oder für ein Alkalimetallkation steht und
$R^1$, $R^2$, $R^3$, X und Y die oben angegebene Bedeutung haben,
mit Alkylierungsmitteln der Formel (III),

$R^5$-$E^1$ (III)

in welcher
$E^1$ für eine elektronenanziehende Abgangsgruppe steht und
$R^5$ die oben angegebene Bedeutung hat,
gegebenenfalls in Gegenwart eines Verdünnungsmittels und gegebenenfalls in Gegenwart eines Reaktionshilfsmittels umsetzt;
(b) man erhält substituierte Acrylsäureester der Formel (Ib),

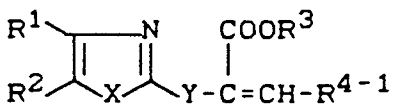

(Ib)

in welcher
$R^{4-1}$ für Dialkylamino steht und
$R^1$, $R^2$, $R^3$, X und Y die oben angegebene Bedeutung haben,
wenn man substituierte Essigsäureester der Formel (IV),

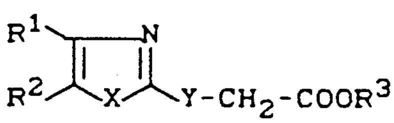

(IV)

in welcher
$R^1$, $R^2$, $R^3$, X und Y die oben angegebene Bedeutung haben,
mit Formamiden der Formel (Va),

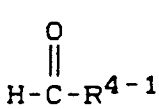

(Va)

in welcher
$R^{4-1}$ die oben angegebene Bedeutung hat,
oder mit deren Derivaten der Formel (Vb),

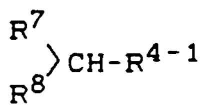

(Vb)

in welcher
$R^7$ und $R^8$ unabhängig voneinander jeweils für Alkoxy oder Dialkylamino stehen und
$R^{4-1}$ die oben angegebene Bedeutung hat,
gegebenenfalls in Gegenwart eines Verdünnungsmittels umsetzt;
(c) man erhält substituierte Acrylsäureester der Formel (Ic),

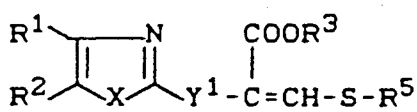

(Ic)

in welcher
$Y^1$ für Schwefel oder für einen Rest

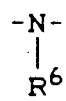

steht, und
$R^1$, $R^2$, $R^3$, $R^5$, $R^6$ und X die oben angegebene Bedeutung haben,
wenn man Oxalsäurederivate der Formel (VI),

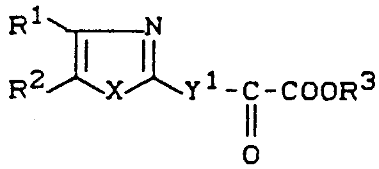

(VI)

in welcher
$R^1$, $R^2$, $R^3$, X und $Y^1$ die oben angegebene Bedeutung haben,
mit metallorganischen Verbindungen der Formel (VII),

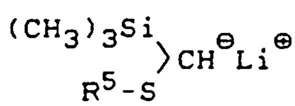

(VII)

in welcher
$R^5$ die oben angegebene Bedeutung hat,
gegebenenfalls in Gegenwart eines Verdünnungsmittels umsetzt;

(d) man erhält substituierte Acrylsäureester der Formel (Id),

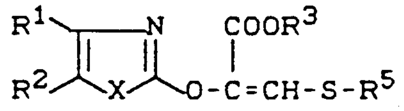
(Id)

in welcher
$R^1$, $R^2$, $R^3$, $R^5$ und X die oben angegebene Bedeutung haben,
wenn man substituierte Acrylsäureester der Formel (VIII),

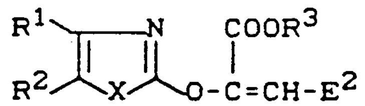
(VIII)

in welcher
$E^2$ für eine elektronenanziehende Abgangsgruppe steht und
$R^1$, $R^2$, $R^3$ und X die oben angegebene Bedeutung haben,
mit Thiolen der Formel (IX),

$R^5$-SH (IX)

in welcher
$R^5$ die oben angegebene Bedeutung hat,
gegebenenfalls in Gegenwart eines Verdünnungsmittels und gegebenenfalls in Gegenwart eines Reaktionshilfsmittels umsetzt.

Schließlich wurde gefunden, daß die neuen substituierten Acrylsäureester der allgemeinen Formel (I) eine gute Wirkung gegen Schädlinge besitzen.

Überraschenderweise zeigen die erfindungsgemäßen substituierten Acrylsäureester der allgemeinen Formel (I) z.B. eine erheblich bessere fungizide Wirksamkeit als die aus dem Stand der Technik bekannten Acrylsäureester, wie beispielsweise die Verbindung 3-Methoxy-2-(2-methylphenyl)-acrylsäuremethylester, welche chemisch und wirkungsmäßig naheliegende Verbindungen sind.

Besonders bevorzugt sind Verbindungen der Formel (I), bei welchen

$R^1$ für Wasserstoff, Methyl, Ethyl, n- oder i-Propyl, n-, i-, s- oder t-Butyl, für Allyl, n- oder i-Butenyl, für jeweils gegebenenfalls im Arylteil oder im Heteroarylteil ein- bis vierfach, gleich oder verschieden substituiertes Benzyl, Phenylethyl, Phenylethenyl, Phenyl, Naphthyl, Pyridyl, Thienyl oder Furyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy oder Benzyloxy,

$R^2$ für Chlor, Brom, Iod, Cyano, Nitro, Formyl, Chlormethyl, Brommethyl, Iodmethyl, Methoxymethyl, Ethoxymethyl, n- oder i-Propoxymethyl, Methylthiomethyl, Ethylthiomethyl, n- oder i-Propylthiomethyl, für Hydroximinomethyl, Hydroximinoethyl, Hydroximinopropyl, für Methoximinomethyl, Ethoximinomethyl, n- oder i-Propoximinomethyl, Methoximinoethyl, Ethoximinoethyl, n- oder i-Propoximinoethyl, für Methyliminomethyl, Ethyliminomethyl, n- oder i-Propyliminomethyl, für Methyliminoethyl, Ethyliminoethyl, n- oder i-Propyliminoethyl, für N,N-Dimethylhydrazonomethyl, N,N-Diethylhydrazonomethyl, N-Methyl-N-ethylhydrazonomethyl, N-Methyl-N-propylhydrazonomethyl, N,N-Dipropylhydrazonomethyl, N,N-Dimethylhydrazonoethyl, N,N-Diethylhydrazonoethyl, für Methoxy, Ethoxy, n- oder i-Propoxy, Methylthio, Ethylthio, n- oder i-Propylthio, für Trifluormethoxy, Difluormethoxy, Dichlorfluormethoxy, Difluorchlormethoxy, Trichlormethoxy, Trifluormethylthio, Trichlormethylthio, Difluormethylthio, Dichlorfluormethylthio, Difluorchlormethylthio, für Acetyl,

Propionyl, Butyryl, Methoxycarbonyl, Ethoxycarbonyl, n- oder i-Propoxycarbonyl, N,N-Dimethylaminocarbonyl, N,N-Diethylaminocarbonyl, N,N-Dipropylaminocarbonyl, N-Methyl-N-ethylaminocarbonyl, N-Methyl-N-propylaminocarbonyl, für 1-Piperidinylcarbonyl, 4-Morpholinylcarbonyl oder für jeweils gegebenenfalls im Phenylteil ein- bis dreifach, gleich oder verschieden substituiertes N-Phenyliminomethyl, N-Phenyliminoethyl, Phenoxy oder Phenylthio steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/oder Ethyl substituiertes Phenyl,

$R^3$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^1$ genannten infrage kommen,

$R^4$ für Dialkylamino mit jeweils 1 bis 4 Kohlenstoffatomen in den einzelnen geradkettigen oder verzweigten Alkylteilen steht oder für einen Rest -Z-$R^5$ steht,

X für Sauerstoff oder Schwefel steht und

Y für Sauerstoff, Schwefel oder für einen Rest

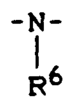

steht,

wobei

$R^5$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^1$ genannten infrage kommen;

$R^6$ für Wasserstoff, Methyl, Ethyl, n- oder i-Propyl, n-, i-, s- oder t-Butyl, für Acetyl, Propionyl, n- oder i-Butyryl oder für jeweils gegebenenfalls ein- bis dreifach, gleich oder verschieden substituiertes Benzyl oder Phenyl steht, wobei als Substituenten die bei $R^1$ genannten infrage kommen und

Z für Sauerstoff oder Schwefel steht.

Ganz besonders bevorzugt sind Verbindungen der Formel (I), bei welchen

$R^1$ für Wasserstoff, Methyl, Ethyl, n- oder i-Propyl oder für jeweils gegebenenfalls ein- bis vierfach, gleich oder verschieden substituiertes Phenyl oder Naphthyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls ein- oder zweifach, gleich oder verschieden durch Fluor, Chlor, Brom, Methyl und/oder Ethyl substituiertes Phenyl, Phenoxy, Benzyl oder Benzyloxy,

$R^2$ für Chlor, Brom, Cyano, Nitro, Formyl, Methoxymethyl, Ethoxymethyl, Methylthiomethyl, Hydroximinomethyl, Methoximinomethyl, Ethoximinomethyl, Methyliminomethyl, Ethyliminomethyl, N,N-Dimethylhydrazonomethyl, N,N-Diethylhydrazonomethyl, für Methoxy, Ethoxy, Methylthio, Ethylthio, Trifluormethylthio, Dichlorfluormethylthio, Difluorchlormethylthio, Trichlormethylthio, für Acetyl, Methoxycarbonyl, Ethoxycarbonyl, N,N-Dimethylaminocarbonyl, N,N-Diethylaminocarbonyl, N-Methyl-N-ethylaminocarbonyl oder für gegebenenfalls einfach oder zweifach, gleich oder verschieden substituiertes Phenylthio steht, wobei als Substituenten infrage kommen: Fluor, Chlor, Brom, Cyano, Nitro, Methyl, Ethyl, n- oder i-Propyl n-, i-, s- oder t-Butyl, Methoxy, Ethoxy, n- oder i-Propoxy, Methylthio, Trifluormethyl, Trifluormethoxy, Trifluormethylthio,

$R^3$ für Methyl, Ethyl oder Benzyl steht,

$R^4$ für Dimethylamino, Diethylamino oder für einen Rest -Z-$R^5$ steht,

X für Sauerstoff oder Schwefel steht und

Y für Sauerstoff, Schwefel oder für einen Rest

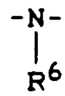

steht,
wobei

$R^5$ für Methyl, Ethyl, n- oder i-Propyl oder Benzyl steht,

$R^6$ für Wasserstoff, Methyl, Ethyl, Acetyl, Propionyl oder für jeweils gegebenenfalls einfach oder zweifach, gleich oder verschieden durch Fluor, Chlor, Brom, Methyl, Ethyl und/oder Trifluormethyl substituiertes Benzyl oder Phenyl steht und

Z für Sauerstoff oder Schwefel steht.

Insbesondere bevorzugt sind Verbindungen der Formel (I), bei welchen

$R^1$ für Wasserstoff, Methyl, Ethyl, n- oder i-Propyl oder für gegebenenfalls einfach oder zweifach, gleich oder verschieden substituiertes Phenyl steht, wobei als Substituenten infrage kommen: Fluor, Chlor, Brom, Cyano, Nitro, Methyl, Ethyl, Methoxy, Ethoxy, Methylthio, Trifluormethyl, Trifluormethoxy, Trifluormethylthio, Methoxycarbonyl, Ethoxycarbonyl, Methoximinomethyl, Cyclopentyl, 1,3-Propandiyl, Methoximinoethyl oder gegebenenfalls ein- oder zweifach, gleich oder verschieden durch Fluor, Chlor, Brom oder Methyl substituiertes Phenyl, Phenoxy, Benzyl oder Benzyloxy,

$R^2$ für Chlor, Brom, Cyano, Nitro, Formyl, Methoxymethyl, Methylthiomethyl, Methoxycarbonyl, Ethoxycarbonyl, Methylthio, Trifluormethylthio oder Methoximinomethyl steht,

$R^3$ für Methyl oder Ethyl steht,

$R^4$ für Methoxy, Ethoxy, Methylthio oder Dimethylamino steht,

X für Sauerstoff oder Schwefel steht und

Y für einen N-Methyl-Rest steht.

Im einzelnen seien außer den bei den Herstellungsbeispielen genannten Verbindungen die folgenden substituierten Acrylsäureester der allgemeinen Formel (I) genannt:

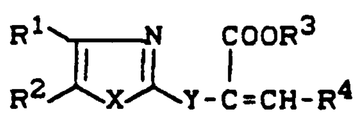

(I)

| $R^1$ | $R^2$ | $R^3$ | $R^4$ | X | Y |
|---|---|---|---|---|---|
| 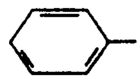 | CN | $CH_3$ | $OCH_3$ | S | $N-CH_3$ |
| 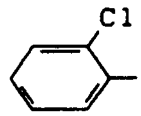 | CN | $CH_3$ | $OCH_3$ | S | $N-CH_3$ |
| 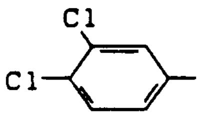 | CN | $CH_3$ | $OCH_3$ | S | $N-CH_3$ |
| 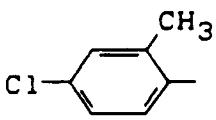 | CN | $CH_3$ | $OCH_3$ | S | $N-CH_3$ |
| 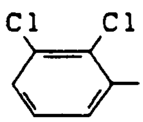 | CN | $CH_3$ | $OCH_3$ | S | $N-CH_3$ |
| 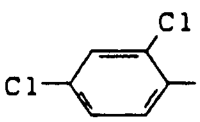 | CN | $CH_3$ | $OCH_3$ | S | $N-CH_3$ |
| 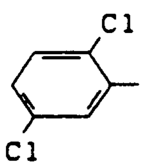 | CN | $CH_3$ | $OCH_3$ | S | $N-CH_3$ |
| 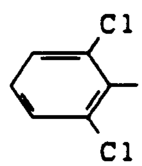 | CN | $CH_3$ | $OCH_3$ | S | $N-CH_3$ |

| $R^1$ | $R^2$ | $R^3$ | $R^4$ | X | Y |
|---|---|---|---|---|---|
| 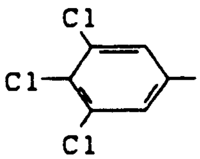 | CN | $CH_3$ | $OCH_3$ | S | $N-CH_3$ |
| 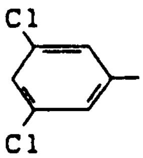 | CN | $CH_3$ | $OCH_3$ | S | $N-CH_3$ |
| 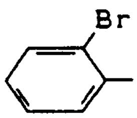 | CN | $CH_3$ | $OCH_3$ | S | $N-CH_3$ |
| 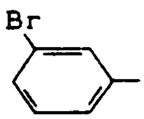 | CN | $CH_3$ | $OCH_3$ | S | $N-CH_3$ |
| 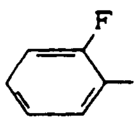 | CN | $CH_3$ | $OCH_3$ | S | $N-CH_3$ |
| 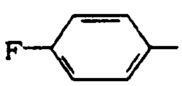 | CN | $CH_3$ | $OCH_3$ | S | $N-CH_3$ |
| 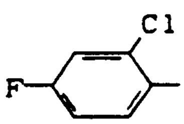 | CN | $CH_3$ | $OCH_3$ | S | $N-CH_3$ |
| 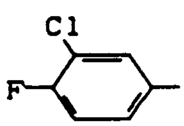 | CN | $CH_3$ | $OCH_3$ | S | $N-CH_3$ |

| $R^1$ | $R^2$ | $R^3$ | $R^4$ | X | Y |
|---|---|---|---|---|---|
| 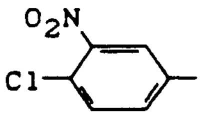 | CN | $CH_3$ | $OCH_3$ | S | $N-CH_3$ |
| 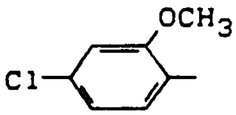 | CN | $CH_3$ | $OCH_3$ | S | $N-CH_3$ |
| 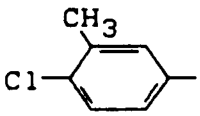 | CN | $CH_3$ | $OCH_3$ | S | $N-CH_3$ |
| 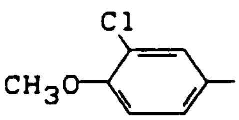 | CN | $CH_3$ | $OCH_3$ | S | $N-CH_3$ |
| 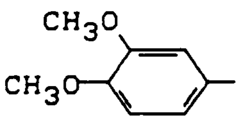 | CN | $CH_3$ | $OCH_3$ | S | $N-CH_3$ |
| 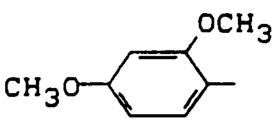 | CN | $CH_3$ | $OCH_3$ | S | $N-CH_3$ |
| 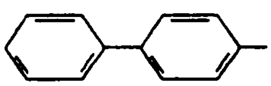 | CN | $CH_3$ | $OCH_3$ | S | $N-CH_3$ |
| 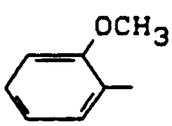 | CN | $CH_3$ | $OCH_3$ | S | $N-CH_3$ |

| $R^1$ | $R^2$ | $R^3$ | $R^4$ | X | Y |
|---|---|---|---|---|---|
| 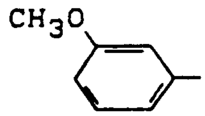 | -CHO | $CH_3$ | $OCH_3$ | S | $N-CH_3$ |
| 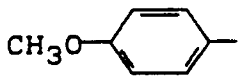 | -CHO | $CH_3$ | $OCH_3$ | S | $N-CH_3$ |
| 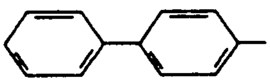 | -CHO | $CH_3$ | $OCH_3$ | S | $N-CH_3$ |
| 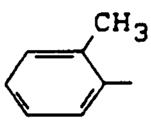 | -CHO | $CH_3$ | $OCH_3$ | S | $N-CH_3$ |
| 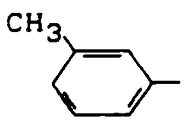 | -CHO | $CH_3$ | $OCH_3$ | S | $N-CH_3$ |
| 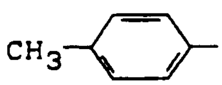 | -CHO | $CH_3$ | $OCH_3$ | S | $N-CH_3$ |
| 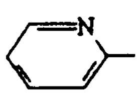 | -CHO | $CH_3$ | $OCH_3$ | S | $N-CH_3$ |
| 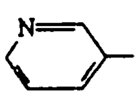 | -CHO | $CH_3$ | $OCH_3$ | S | $N-CH_3$ |
| 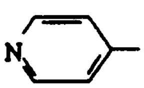 | -CHO | $CH_3$ | $OCH_3$ | S | $N-CH_3$ |
| 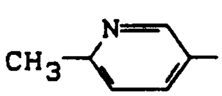 | -CHO | $CH_3$ | $OCH_3$ | S | $N-CH_3$ |

| $R^1$ | $R^2$ | $R^3$ | $R^4$ | X | Y |
|---|---|---|---|---|---|
| 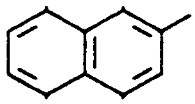 | $-COOCH_3$ | $CH_3$ | $OCH_3$ | S | $N-CH_3$ |
| 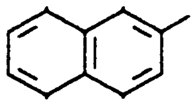 | $-COOCH_3$ | $CH_3$ | $OCH_3$ | O | $N-CH_3$ |
| 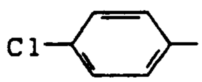 | -CHO | $CH_3$ | $OCH_3$ | S | 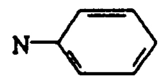 |
| 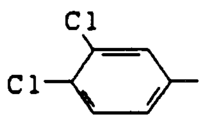 | -CHO | $CH_3$ | $OCH_3$ | S | $N-CH_3$ |
| 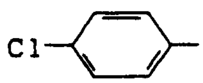 | Cl | $CH_3$ | $OCH_3$ | O | 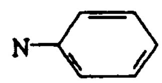 |
| 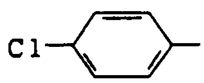 | Cl | $CH_3$ | $OCH_3$ | O | $N-CH_3$ |
| 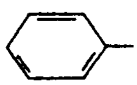 | Cl | $CH_3$ | $OCH_3$ | O | $N-CH_3$ |
| 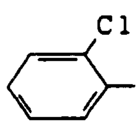 | Cl | $CH_3$ | $OCH_3$ | O | $N-CH_3$ |
| 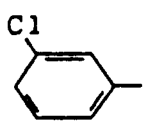 | Cl | $CH_3$ | $OCH_3$ | O | $N-CH_3$ |
| 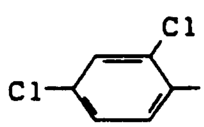 | Cl | $CH_3$ | $OCH_3$ | O | $N-CH_3$ |

| $R^1$ | $R^2$ | $R^3$ | $R^4$ | X | Y |
|---|---|---|---|---|---|
| 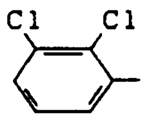 | Cl | $CH_3$ | $OCH_3$ | O | $N-CH_3$ |
| 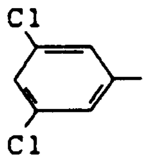 | Br | $CH_3$ | $OCH_3$ | O | $N-CH_3$ |
| 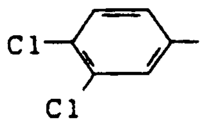 | Br | $CH_3$ | $OCH_3$ | O | $N-CH_3$ |
| 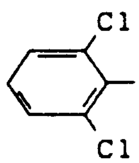 | Br | $CH_3$ | $OCH_3$ | O | $N-CH_3$ |
| 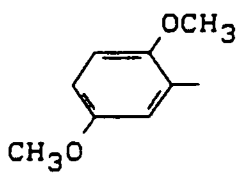 | Br | $CH_3$ | $OCH_3$ | O | $N-CH_3$ |
| 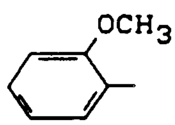 | Br | $CH_3$ | $OCH_3$ | O | $N-CH_3$ |
| 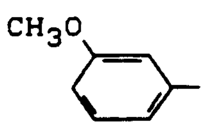 | Br | $CH_3$ | $OCH_3$ | O | $N-CH_3$ |
| 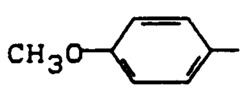 | Br | $CH_3$ | $OCH_3$ | O | $N-CH_3$ |

| $R^1$ | $R^2$ | $R^3$ | $R^4$ | X | Y |
|---|---|---|---|---|---|
| 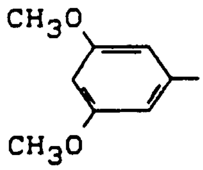 | $NO_2$ | $CH_3$ | $OCH_3$ | O | $N-CH_3$ |
| 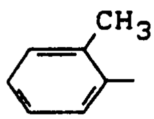 | $NO_2$ | $CH_3$ | $OCH_3$ | O | $N-CH_3$ |
| 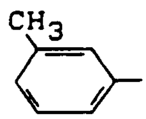 | $NO_2$ | $CH_3$ | $OCH_3$ | O | $N-CH_3$ |
| 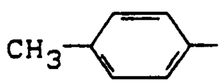 | $NO_2$ | $CH_3$ | $OCH_3$ | O | $N-CH_3$ |
| 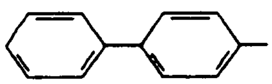 | $-COOCH_3$ | $CH_3$ | $OCH_3$ | O | $N-CH_3$ |
| 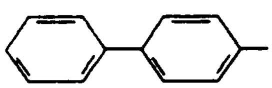 | Br | $CH_3$ | $OCH_3$ | O | $N-CH_3$ |
| 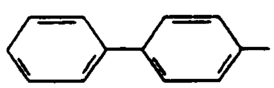 | Cl | $CH_3$ | $OCH_3$ | O | $N-CH_3$ |
| 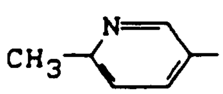 | $-COOCH_3$ | $CH_3$ | $OCH_3$ | O | $N-CH_3$ |
| 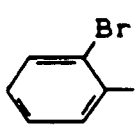 | $-CH=N-OCH_3$ | $CH_3$ | $OCH_3$ | O | $N-CH_3$ |

| $R^1$ | $R^2$ | $R^3$ | $R^4$ | X | Y |
|---|---|---|---|---|---|
| 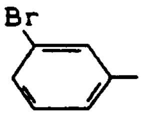 | $-CH=N-OCH_3$ | $CH_3$ | $OCH_3$ | O | $N-CH_3$ |
| 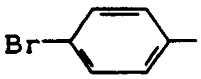 | $-CH=N-OCH_3$ | $CH_3$ | $OCH_3$ | O | $N-CH_3$ |
| 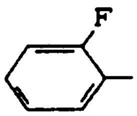 | $-CH_2-OCH_3$ | $CH_3$ | $OCH_3$ | O | $N-CH_3$ |
| 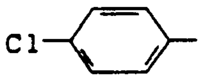 | $-CH_2-OCH_3$ | $CH_3$ | $OCH_3$ | O | $N-CH_3$ |
| 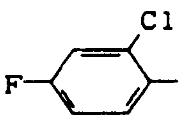 | $-CH_2-OCH_3$ | $CH_3$ | $OCH_3$ | O | $N-CH_3$ |
| 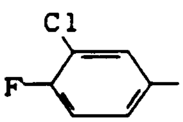 | $-CH_2-OCH_3$ | $CH_3$ | $OCH_3$ | O | $N-CH_3$ |
| 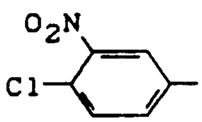 | $-CH_2-OCH_3$ | $CH_3$ | $OCH_3$ | O | $N-CH_3$ |
| 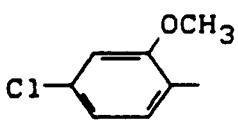 | $-CH_2-OCH_3$ | $CH_3$ | $OCH_3$ | O | $N-CH_3$ |
| 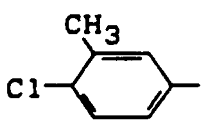 | $-CH_2-OCH_3$ | $CH_3$ | $OCH_3$ | O | $N-CH_3$ |

| $R^1$ | $R^2$ | $R^3$ | $R^4$ | X | Y |
|---|---|---|---|---|---|
| 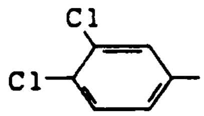 | Br | $CH_3$ | $OCH_3$ | S | $N-CH_3$ |
| 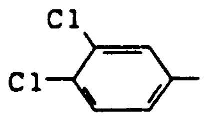 | $-SCH_3$ | $CH_3$ | $OCH_3$ | S | $N-CH_3$ |
| 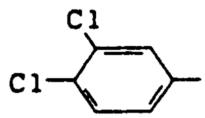 | $-COOCH_3$ | $CH_3$ | $OCH_3$ | S | $N-CH_3$ |
| 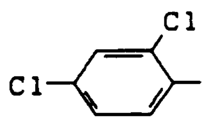 | $-OCH_3$ | $CH_3$ | $SCH_3$ | S | $N-CH_3$ |
| 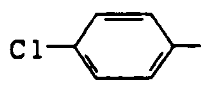 | $-OCH_3$ | $CH_3$ | $SCH_3$ | S | $N-CH_3$ |
| 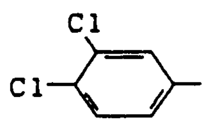 | $NO_2$ | $CH_3$ | $OCH_3$ | S | $N-CH_3$ |
| 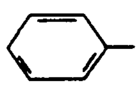 | $-COOCH_3$ | $CH_3$ | $OCH_3$ | S | $N-CH_3$ |
| 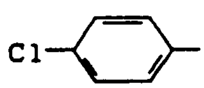 | CN | $CH_3$ | $-N\left\langle\begin{matrix}CH_3\\CH_3\end{matrix}\right.$ | S | $N-CH_3$ |
| 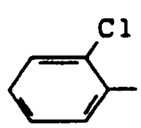 | $-CHO$ | $CH_3$ | $-N\left\langle\begin{matrix}CH_3\\CH_3\end{matrix}\right.$ | O | $N-CH_3$ |

| $R^1$ | $R^2$ | $R^3$ | $R^4$ | X | Y |
|---|---|---|---|---|---|
| 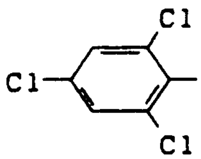 | CN | $CH_3$ | $OCH_3$ | S | S |
| 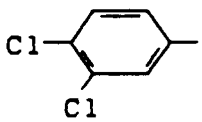 | Br | $CH_3$ | $OCH_3$ | O | S |
| 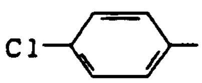 | $-COOCH_3$ | $C_2H_5$ | $OCH_3$ | S | $N-CH_3$ |
| 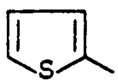 | $-COOCH_3$ | $CH_3$ | $OCH_3$ | S | $N-CH_3$ |
| 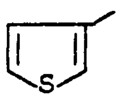 | Br | $CH_3$ | $OCH_3$ | S | $N-CH_3$ |
| 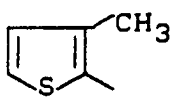 | Cl | $CH_3$ | $OCH_3$ | S | $N-CH_3$ |
| 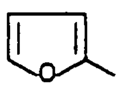 | Br | $CH_3$ | $OCH_3$ | S | $N-CH_3$ |
| 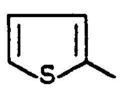 | Cl | $CH_3$ | $OCH_3$ | O | $N-CH_3$ |
| 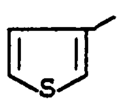 | $NO_2$ | $CH_3$ | $OCH_3$ | O | $N-CH_3$ |
| 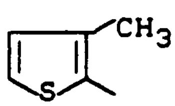 | $-COOCH_3$ | $CH_3$ | $OCH_3$ | O | $N-CH_3$ |
| 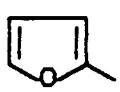 | $-CH_2-S-CH_3$ | $CH_3$ | $OCH_3$ | O | $N-CH_3$ |

Verwendet man beispielsweise 2-{N-[4-(3,4-Dichlorphenyl)-5-bromthiazol-2-yl]-N-methylamino}-3-hydroxyacrylsäuremethylester und Dimethylsulfat als Ausgangsstoffe, so läßt sich der Reaktionsablauf des erfindungsgemäßen Verfahrens (a) durch das folgende Formelschema darstellen:

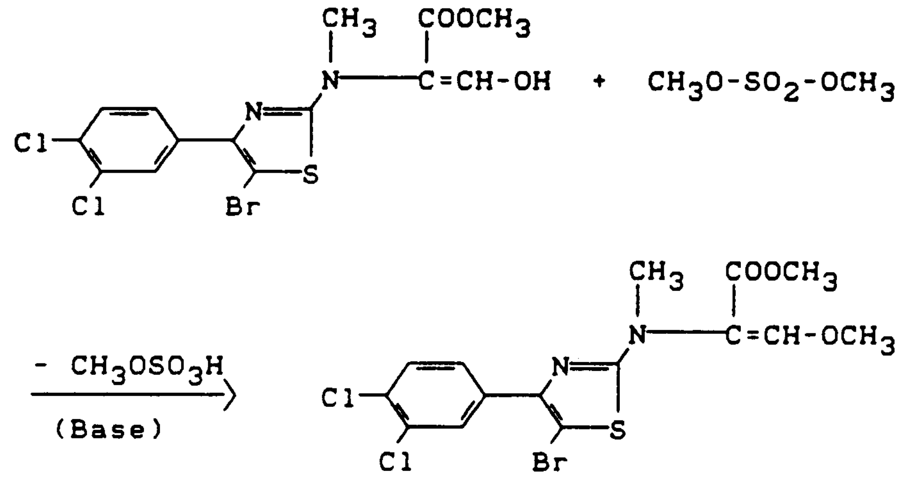


Verwendet man beispielsweise 2-[4-(4-Chlorphenyl)-5-methoxycarbonylthiazol-2-yl-oxy]-essigsäuremethylester und Dimethylformamiddimethylacetal als Ausgangsstoffe, so läßt sich der Reaktionsablauf des erfindungsgemäßen Verfahrens (b) durch das folgende Formelschema darstellen:

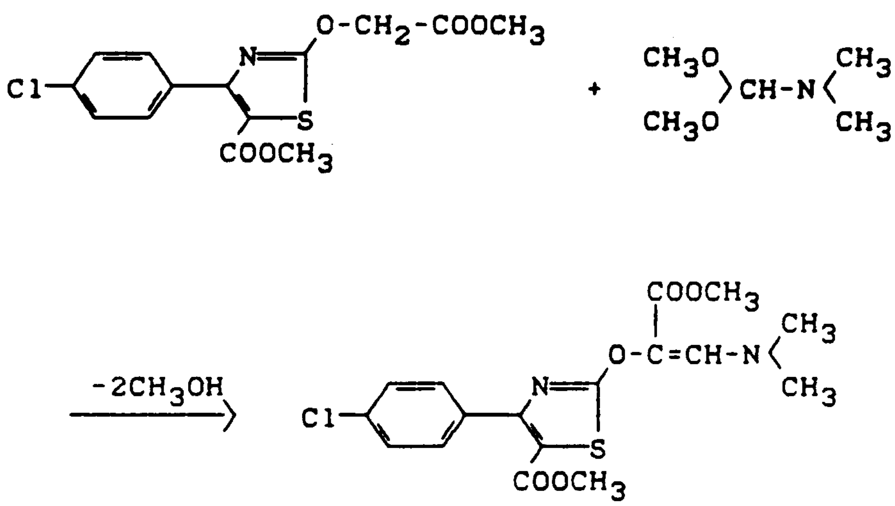


Verwendet man beispielsweise 2-[4-(2,4-Dichlorphenyl)-5-chlorthiazol-2-yl-thio]-2-oxo-essigsäuremethylester und (Methylthio)-(trimethylsilyl)-methylenlithium als Ausgangsstoffe, so läßt sich der Reaktionsablauf des erfindungsgemäßen Verfahrens (c) durch das folgende Formelschema darstellen:

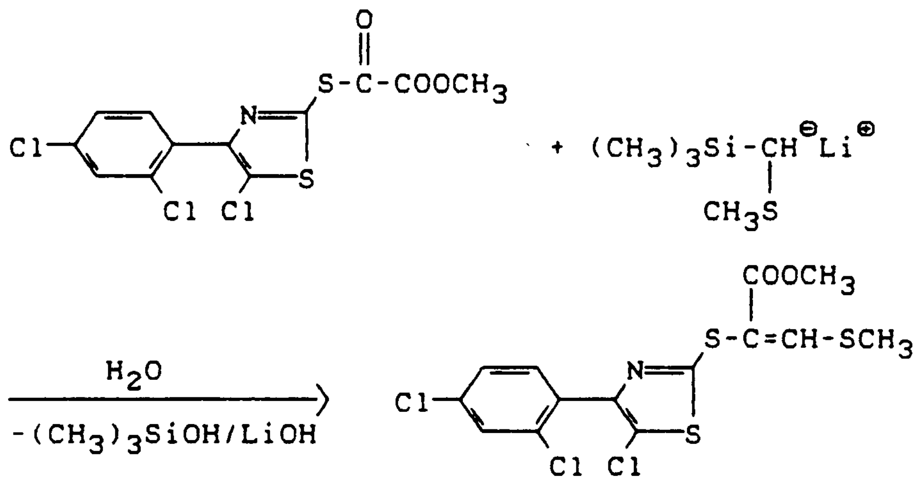


Verwendet man beispielsweise 2-[4-(4-Chlorphenyl)-5-cyanooxazol-2-yl-oxy]-3-methylsulfonyloxy-acrylsäuremethylester und Methylmercaptan als Ausgangsstoffe, so läßt sich der Reaktionsablauf des erfindungsgemäßen Verfahrens (d) durch das folgende Formelschema darstellen:

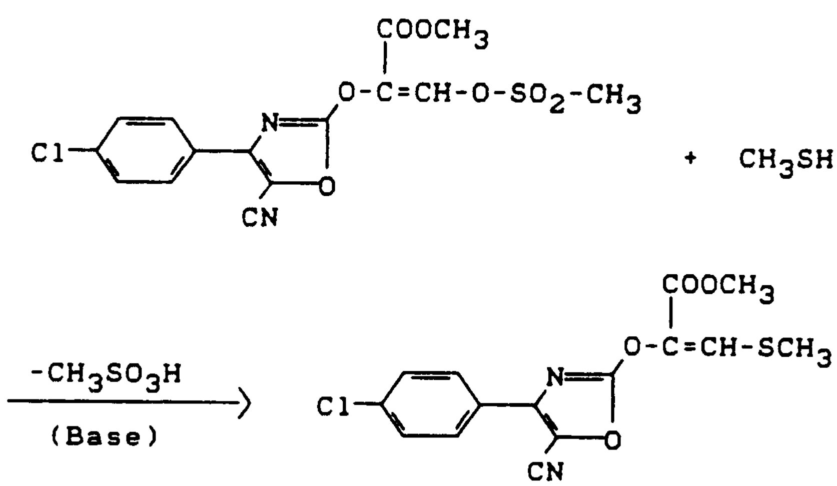


Die zur Durchführung des erfindungsgemäßen Verfahrens (a) als Ausgangsstoffe benötigten Hydroxyacrylsäureester oder deren Alkalimetallsalze sind durch die Formel (II) allgemein definiert. In dieser Formel (II) stehen $R^1$, $R^2$, $R^3$, X und Y vorzugsweise für diejenigen Reste, die bereits im Zusammenhang mit der Beschreibung der erfindungsgemäßen Stoffe der Formel (I) als bevorzugt für diese Substituenten genannt wurden.

M steht vorzugsweise für Wasserstoff oder für ein Natrium- oder Kaliumkation.

Die Hydroxyacrylsäureester der Formel (II) sind noch nicht bekannt und ebenfalls Gegenstand der Erfindung.

Man erhält sie, wenn man substituierte Essigsäureester der Formel (IV),

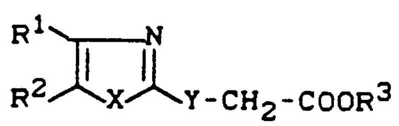 (IV)

in welcher

$R^1$, $R^2$, $R^3$, X und Y die oben angegebene Bedeutung haben,

mit Ameisensäureestern der Formel (X),

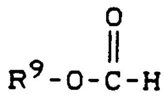

(X)

in welcher

$R^9$ für Alkyl, insbesondere für Methyl oder Ethyl steht,

gegebenenfalls in Gegenwart eines Verdünnungsmittels wie beispielsweise Dimethylformamid und gegebenenfalls in Gegenwart eines basischen Reaktionshilfsmittels wie beispielsweise Natriumhydrid bei Temperaturen zwischen - 20 °C und + 50 °C umsetzt.

Ameisensäureester der Formel (X) sind allgemein bekannte Verbindungen der organischen Chemie.

Die zur Durchführung des erfindungsgemäßen Verfahrens (a) weiterhin als Ausgangsstoffe benötigten Alkylierungsmittel sind durch die Formel (III) allgemein definiert. In dieser Formel (III) steht $R^5$ vorzugsweise für diejenigen Reste, die bereits im Zusammenhang mit der Beschreibung der erfindungsgemäßen Stoffe der Formel (I) als bevorzugt für diesen Substituenten genannt wurden.

$E^1$ steht für eine bei Alkylierungsmitteln übliche Abgangsgruppe, vorzugsweise für einen gegebenenfalls substituierten Alkyl-, Alkoxy- oder Arylsulfonyloxyrest, wie beispielsweise ein Methoxysulfonyloxyrest, ein Ethoxysulfonyloxyrest oder ein p-Toluolsulfonyloxyrest oder für Halogen, insbesondere für Chlor, Brom oder Iod.

Die Alkylierungsmittel der Formel (III) sind allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten substituierten Essigsäureester sind durch die Formel (IV) allgemein definiert. In dieser Formel (IV) stehen $R^1$, $R^2$, $R^3$, X und Y vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Essigsäureester der Formel (IV) sind teilweise bekannt (vgl. z.B. J. Med. Chem. 15, 951-954 [1972]; J. Med. Chem. 14, 10-16 [1971]; J. Med. Chem. 16, 1030-1034 [1973]).

Noch nicht bekannt und ebenfalls Gegenstand der Erfindung sind substituierte Essigsäureester der Formel (IVa),

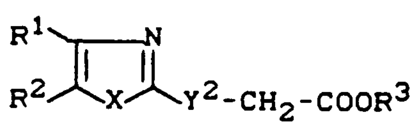

(IVa)

in welcher

$Y^2$ für Sauerstoff, Schwefel oder für einen N-Alkylrest, vorzugsweise mit 1 bis 4 Kohlenstoffatomen, insbesondere für einen -N-Methylrest oder für einen -N-Ethylrest steht und

$R^1$, $R^2$, $R^3$ und X die oben angegebene Bedeutung haben.

Man erhält sie in Analogie zu bekannten Verfahren, beispielsweise, wenn man Thiazol- oder Oxazolderivate der Formel (XIa),

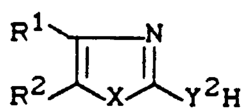

(XIa)

in welcher

$R^1$, $R^2$,X und $Y^2$ die oben angegebene Bedeutung haben,

oder Thiazol- oder Oxazolderivate der Formel (XIb),

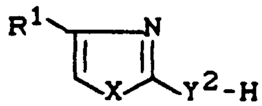

(XIb)

in welcher

$R^1$, X und $Y^2$ die oben angegebene Bedeutung haben,

mit Bromessigestern der Formel (XII),

$Br\text{-}CH_2\text{-}COOR^3$ (XII)

in welcher

$R^3$ die oben angegebene Bedeutung hat,

gegebenenfalls in Gegenwart eines Verdünnungsmittels wie beispielsweise Dimethylformamid und gegebenenfalls in Gegenwart eines Reaktionshilfsmittels wie beispielsweise Natriumhydrid bei Temperaturen zwischen - 20 °C und + 150 °C umsetzt und gegebenenfalls anschließend in 5-Position des Thiazol- bzw. Oxazolringes mit üblichen elektrophilen Substitutionsreaktionen substituiert oder bereits vorhandene funktionelle Gruppen in dieser Position mit Hilfe allgemein bekannter Verfahren derivatisiert (vgl. auch die Herstellungsbeispiele). So lassen sich beispielsweise durch übliche elektrophile Substitutionen die Nitrogruppe mit Salpetersäure, die Halogenreste mit elementarem Chlor oder Brom oder anderen üblichen Halogenierungsmitteln, die Formylgruppe durch die Vilsmeier Reaktion mit Phosphoroxychlorid und Dimethylformamid, Alkanoylgruppen durch Friedel-Crafts-Acylierungen mit Alkanoylchloriden in Gegenwart von Aluminiumtrichlorid, Alkylthio-, Halogenalkylthio- und Arylthioreste durch direkte Sulfenylierung mit den entsprechenden Sulfenylchloriden oder durch Rhodanierung mit Kaliumthiocyanat oder Ammoniumthiocyanat, anschließende Reduktion zur Mercaptogruppe und Alkylierung derselben einführen.

Iminoalkylsubstituenten in 5-Position des Heterocyclus erhält man durch Umsetzen von Formyl- oder Alkanoylsubstituenten mit aliphatischen oder aromatischen Aminen, Hydroxylaminen oder Hydrazinen in üblicher Art und Weise. Amidsubstituenten erhält man aus Methoxy- oder Ethoxycarbonylgruppen durch Umsetzung mit Aminen, die Cyanogruppe durch Dehydratisierung der so erhältlichen N-unsubstituierten Carbamoylgruppe oder durch Dehydratisierung der Hydroximinomethylgruppe.

Alkoxymethylgruppen, Alkylthiomethylgruppen und Halogenmethylgruppen lassen sich einführen durch Reduktion der Formylgruppe beispielsweise mit Natriumborhydrid und anschließende Alkylierung der so erhältlichen Hydroxymethylgruppe oder anschließende nukleophile Substitution mit Halogen oder anschließende Sulfonylierung mit einem üblichen Alkyl- oder Arylsulfonsäurechlorid und anschließende Substitution mit Alkoholen oder Thiolen unter Austritt von Alkyl- oder Arylsulfonyloxyresten.

Es kann gegebenenfalls auch von Vorteil sein, die oben beschriebenen Substitutions- und/oder Derivatisierungsreaktionen bereits auf der Stufe der Thiazol- oder Oxazolderivate der Formeln (XIa) bzw. (XIb) vor der Umsetzung mit den Bromessigestern der Formel (XII) durchzuführen (vgl. auch die Herstellungsbeispiele).

Thiazol- und Oxazolderivate der Formeln (XIa) und (XIb) sind bekannt oder erhältlich in Analogie zu bekannten Verfahren (vgl. z.B. Heterocycles 23, 2645-2649 [1985]; GB 20 20 661; Indian J. Chem. Sect. B, 16B, 749-751 [1978]; J. org. Chem. 32, 3132-3134 (1967]; J. Heterocycl. Chem. 22, 1621-1630 [1985]; JP 63/112 572; JP 63/112 573; JP 63/112 574; JP 62/132 871). Bromessigester der Formel (XII) sind allgemein bekannte Verbindungen der organischen Chemie.

Die zur Durchführung des erfindungsgemäßen Verfahrens (b) weiterhin als Ausgangsstoffe benötigten Formamide und deren Derivate sind durch die Formeln (Va) und (Vb) allgemein definiert. In diesen Formeln (Va) und (Vb) steht $R^{4-1}$ vorzugsweise für Dialkylamino mit jeweils 1 bis 6, insbesondere mit 1 bis 4 Kohlenstoffatomen in den einzelnen geradkettigen oder verzweigten Alkylteilen. $R^{4-1}$ steht ganz besonders bevorzugt für Dimethylamino oder Diethylamino.

$R^7$ und $R^8$ stehen vorzugsweise unabhängig voneinander jeweils für geradkettiges oder verzweigtes Alkoxy mit 1 bis 4 Kohlenstoffatomen insbesondere für Methoxy oder Ethoxy oder für einen Dialkylaminorest mit jeweils 1 bis 6, insbesondere mit 1 bis 4 Kohlenstoffatomen in den einzelnen geradkettigen oder verzweigten Alkylteilen.

Die Formamide der Formel (Va) und deren Derivate der Formel (Vb) sind allgemein bekannte Verbindungen der organischen Chemie.

Die zur Durchführung des erfindungsgemäßen Verfahrens (c) als Ausgangsstoffe benötigten Oxalsäurederivate sind durch die Formel (VI) allgemein definiert. In dieser Formel (VI) stehen $R^1$, $R^2$, $R^3$ und X

vorzugsweise für diejenigen Reste, die bereits im Zusammenhang mit der Beschreibung der erfindungsgemäßen Stoffe der Formel (I) als bevorzugt für diese Substituenten genannt wurden.

$Y^1$ steht vorzugsweise für Schwefel oder für einen Rest

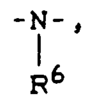

wobei $R^6$ vorzugsweise für diejenigen Reste steht, die bereits im Zusammenhang mit der Beschreibung der erfindungsgemäßen Stoffe der Formel (I) als bevorzugt für diesen Substituenten genannt wurden.

Die Oxalsäurederivate der Formel (VI) sind entweder bekannt (vgl. z.B. FR 13 06 603) oder erhältlich in Analogie zu bekannten Verfahren (vgl. z.B. US 4 321 372; Synthetic Communications 11, 943 [1981] oder Organic Reactions 26, 1 [1979]), beispielsweise wenn man Oxalester der Formel (XIII),

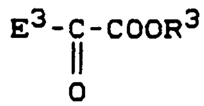

(XIII)

in welcher

$E^3$ für Alkoxy oder Halogen insbesondere für Methoxy, Ethoxy oder Chlor steht und

$R^3$ die oben angegebene Bedeutung hat,

mit Heterocyclen der Formel (XIc),

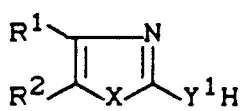

(XIc)

in welcher

$R^1$, $R^2$, X und $Y^1$ die oben angegebene Bedeutung haben,

gegebenenfalls in Gegenwart eines Verdünnungsmittels, wie beispielsweise Dichlormethan oder Tetrahydrofuran und gegebenenfalls in Gegenwart einer Base, wie beispielsweise n-Butyllithium, Natriumhydrid, Kalium-t-butylat, Triethylamin oder Pyridin bei Temperaturen zwischen - 80 °C und + 80 °C umsetzt.

Oxalester der Formel (XIII) sind allgemein bekannte Verbindungen der organischen Chemie.

Heterocyclen der Formel (XIc) sind ebenfalls allgemein bekannt oder erhältlich in Analogie zu allgemein bekannten Verfahren (vgl. z.B. Organic Reactions 6, 367 ff. sowie die Herstellungsbeispiele).

Die zur Durchführung des erfindungsgemäßen Verfahrens (c) weiterhin als Ausgangsstoffe benötigten metallorganischen Verbindungen sind durch die Formel (VII) allgemein definiert. In dieser Formel (VII) steht $R^5$ vorzugsweise für diejenigen Reste, die bereits im Zusammenhang mit der Beschreibung der erfindungsgemäßen Stoffe der Formel (I) als bevorzugt für diesen Substituenten genannt wurden.

Die metallorganischen Verbindungen der Formel (VII) sind bekannt (vgl. z.B. J. org. Chem. 33, 780 [1968]; J.org. Chem. 37, 939 [1972]).

Die zur Durchführung des erfindungsgemäßen Verfahrens (d) als Ausgangsstoffe benötigten substituierten Acrylsäureester sind durch die Formel (VIII) allgemein definiert. In dieser Formel (VIII) stehen $R^1$, $R^2$, $R^3$ und X vorzugsweise für diejenigen Reste, die bereits im Zusammenhang mit der Beschreibung der erfindungsgemäßen Stoffe der Formel (I) als bevorzugt für diese Substituenten genannt wurden.

$E^2$ steht vorzugsweise für einen geeigneten Acyloxy- oder Sulfonyloxyrest, insbesondere für einen Acetoxy-, einen Methansulfonyloxy- oder einen p-Toluolsulfonyloxyrest.

Die substituierten Acrylsäureester der Formel (VIII) sind noch nicht bekannt.

Man erhält sie, wenn man Hydroxyacrylsäureester der Formel (IIb),

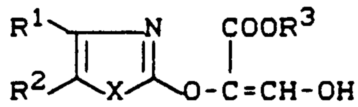

(IIb)

in welcher

$R^1$, $R^2$, $R^3$ und X die oben angegebene Bedeutung haben,

mit Säurechloriden der Formel (XIV),

$R^{10}$-Cl (XIV)

in welcher

$R^{10}$ für einen Acyl- oder Sulfonylrest, insbesondere für einen Acetyl-, einen Methansulfonyl- oder ein p-Toluolsulfonylrest steht,

gegebenenfalls in Gegenwart eines Verdünnungsmittels wie beispielsweise Dichlormethan und gegebenenfalls in Gegenwart eines Säurebindemittels wie beispielsweise Triethylamin oder Pyridin bei Temperaturen zwischen - 20 °C und + 120 °C umsetzt.

Säurechloride der Formel (XIV) sind allgemein bekannte Verbindungen der organischen Chemie.

Die zur Durchführung des erfindungsgemäßen Verfahrens (d) weiterhin als Ausgangsstoffe benötigten Thiole sind durch die Formel (IX) allgemein definiert. In dieser Formel (IX) steht $R^5$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Thiole der Formel (IX) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyldimethyl-ammoniummethylsulfat, Dimethyl-$C_{12}$/$C_{14}$-alkylbenzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Das erfindungsgemäße Verfahren (a) wird vorzugsweise in Gegenwart eines geeigneten basischen Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen - 30 °C und + 120 °C, vorzugsweise bei Temperaturen zwischen - 20 °C und + 60 °C.

Zur Durchführung des erfindungsgemäßen Verfahrens (a) setzt man pro Mol an 3-Hydroxyacrylsäureester oder eines entsprechenden Alkalimetallsalzes der Formel (II) im allgemeinen 1.0 bis 10.0 Mol, vorzugsweise 1.0 bis 5.0 Mol an Alkylierungsmittel der Formel (III) und gegebenenfalls 1.0 bis 5.0 Mol, vorzugsweise 1.0 bis 2.0 Mol an Reaktionshilfsmittel ein.

Dabei ist es auch möglich, die als Ausgangsverbindungen zur Durchführung des erfindungsgemäßen Verfahrens (a) benötigten 3-Hydroxyacrylsäureester oder deren Alkalimetallsalze der Formel (II) in einer vorgelagerten Reaktion direkt im Reaktionsgefäß herzustellen und direkt aus dem Reaktionsgemisch heraus ohne Isolierung mit dem Alkylierungsmittel der Formel (III) gemäß dem erfindungsgemäßen Verfahren (a)

weiter umzusetzen ("Eintopfverfahren").
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff oder Ether, wie Diethylether, Dioxan, Tetrahydrofuran oder Ethylenglykoldimethyl- oder -diethylether.

Es ist jedoch auch möglich, das erfindungsgemäße Verfahren (b) ohne Zusatz eines Verdünnungsmittels durchzuführen.

Die Reaktionstemperaturen können bei der Durchführung des erfindungsgemäßen Verfahrens (b) in einem größeren Bereich variiert werden. Im allgemeinen arbeitet man bei Temperaturen zwischen - 20 °C und + 200 °C, vorzugsweise bei Temperaturen zwischen 0 °C und 150 °C.

Zur Durchführung des erfindungsgemäßen Verfahrens (b) setzt man pro Mol an substituiertem Essigsäureester der Formel (IV) im allgemeinen 1.0 bis 30.0 Mol, vorzugsweise 1.0 bis 15.0 Mol an Formamid der Formel (Va) oder eines entsprechenden Derivates der Formel (Vb) ein. Die Reaktionsdurchführung, Aufarbeitung und Isolierung der Reaktionsprodukte erfolgt nach allgemein üblichen Methoden (vgl. hierzu auch G. Mathieu; J. Weill-Raynal "Formation of C-C-Bonds", Vol I; p. 229-244; Thieme Verlag Stuttgart 1973).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan oder Cyclohexan oder Ether, wie Diethylether, Dioxan, Tetrahydrofuran oder Ethylenglykoldimethyl- oder -diethylether.

Die Reaktionstemperaturen können bei der Durchführung des erfindungsgemäßen Verfahrens (c) in einem größeren Bereich variiert werden. Im allgemeinen arbeitet man bei Temperaturen zwischen - 100 °C und + 100 °C, vorzugsweise bei Temperaturen zwischen - 80 °C und + 50 °C.

Zur Durchführung des erfindungsgemäßen Verfahrens (c) setzt man pro Mol an Oxalsäurederivat der Formel (VI) im allgemeinen 1.0 bis 1.5 Mol, vorzugsweise 1.0 bis 1.2 Mol an metallorganischer Verbindung der Formel (VII) ein.
Die Reaktionsdurchführung, Aufarbeitung und Isolierung der Reaktionsprodukte erfolgt nach bekannten Verfahren (vgl. z.B. J. org. Chem. 33, 780 [1968]; J. org. Chem. 37; 939 [1972]).

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (d) wird vorzugsweise in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, oder tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (d) in einem größeren Bereich variiert werden. Im allgemeinen arbeitet man bei Temperaturen zwischen - 20 °C und 180 °C, vorzugsweise bei Temperaturen zwischen 0 °C und 150 °C.

Das erfindungsgemäße Verfahren kann in Abhängigkeit vom Siedepunkt der verwendeten Reaktionspartner, beispielsweise beim Einsatz von niedrigsiedenden Thiolen der Formel (IX) gegebenenfalls auch unter Druck durchgeführt werden.
Vorzugsweise arbeitet man dann bei dem Druck, der sich beim Erhitzen auf die erforderliche Reaktionstemperatur unter den Reaktionsbedingungen einstellt.

Zur Durchführung des erfindungsgemäßen Verfahrens (d) setzt man pro Mol an substituiertem Acrylsäureester der Formel (VIII) im allgemeinen 1.0 bis 20.0 Mol, vorzugsweise 1.0 bis 5.0 Mol an Thiol der Formel (IX) und gegebenenfalls 1.0 bis 5.0 Mol, vorzugsweise 1.0 bis 1.5 Mol an Reaktionshilfsmittel ein.
Die Reaktionsdurchführung, Aufarbeitung und Isolierung der Reaktionsprodukte erfolgt nach allgemein üblichen Methoden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch z.B. als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis) oder gegen den Erreger der Netzfleckenkrankheit der Gerste (Pyrenophora teres) oder gegen den Erreger der Halmbruchkrankheit an Getreide (Pseudocercosporella herpotrichoides) oder gegen den Erreger der Braunspelzigkeit des Weizens (Leptosphaeria nodorum) oder zur Bekämpfung von Reiskrankheiten, wie beispielsweise gegen den Erreger der Reisfleckenkrankheit (Pyricularia oryzae) oder gegen den Erreger der Reisstengelkrankheit (Pellicularia sasakii) oder zur Bekämpfung von Krankheiten im Obst- und Gemüseanbau, wie beispielsweise gegen den Erreger des Apfelschorfes (Venturia inaequalis) einsetzen.

Darüberhinaus zeigen die erfindungsgemäßen Wirkstoffe eine breite in-vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen,

Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1

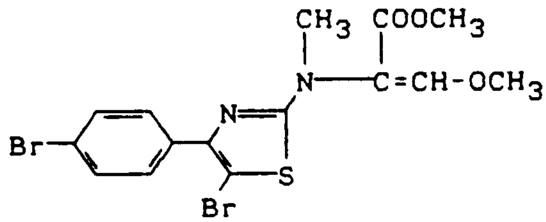

Verfahren (a) - Eintopfvariante

Eine Lösung von 5,4 g (0,013 Mol) N-[5-Brom-4-(4-bromphenyl)-thiazol-2-yl]-N-methylaminoessigsäuremethylester in einem Gemisch aus 25 ml Dimethylformamid und 25 ml Ameisensäuremethylester gibt man tropfenweise unter Rühren und Kühlung sowie unter sorgfältigem Feuchtigkeitsausschluß zu einer Suspension von 0,8 g (0,0335 Mol) Natriumhydrid in 75 ml Dimethylformamid, wobei die Temperatur der Reaktionsmischung 0 °C nicht übersteigen soll. Nach beendeter Zugabe rührt man 15 Stunden bei Raumtemperatur, gibt dann 4,9 g (0,039 Mol) Dimethylsulfat zu, rührt weitere 15 Stunden bei Raumtemperatur, gibt dann 300 ml Wasser zu, extrahiert mehrfach mit Essigester, trocknet die vereinigten organischen Phasen über Natriumsulfat, engt unter vermindertem Druck ein und reinigt den Rückstand durch Chromatographie an Kieselgel.

Man erhält 3,0 g (51 % der Theorie) an N-[5-Brom-4-(4-bromphenyl)-thiazol-3-yl]-N-methyl-2-methoxymethylidenglycinmethylester vom Schmelzpunkt 112 °C.

Beispiel 2

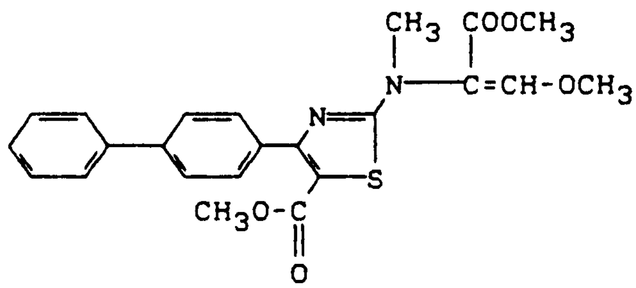

Verfahren (a)-Eintopfvariante

Eine Lösung aus 4 g (0,01 Mol) N-[5-Methoxycarbonyl-4-(4-biphenylyl)-thiazol-2-yl]-N-methylaminoessigsäuremethylester in einem Gemisch aus 10 ml Dimethylformamid und 10 ml Ameisensäuremethylester gibt man tropfenweise unter Rühren und Kühlung sowie unter sorgfältigem Feuchtigkeitsausschuß und einer Argonschutzgasatmosphäre zu einer Suspension von 0,66 g (0,022 Mol) Natriumhydrid in 10 ml Dimethylformamid, wobei die Temperatur der Reaktionsmischung 0 °C nicht übersteigen soll. Nach beendeter Zugabe rührt man weitere vier bis fünf Stunden bei 0 °C, gibt dann tropfenweise unter Rühren 3 g (0,02 Mol) Dimethylsulfat zu und läßt die Reaktionsmischung anschließend langsam auf Raumtemperatur kommen. Man rührt weitere 12 Stunden bei Raumtemperatur, gibt dann die Reaktionsmischung in Eis, extrahiert mehrfach mit Dichlormethan, trocknet die vereinigten organischen Phasen über Natriumsulfat, engt unter vermindertem Druck ein und reinigt den Rückstand durch Chromatographie an Kieselgel (Laufmittel: Dichlormethan/Essigester 20:1).

Man erhält als 1. Fraktion

2,5 g (56,4 % der Theorie) an N-[5-Methoxycarbonyl-4-(4-biphenylyl)-thiazol-2-yl]-N-methyl-2-methoxymethylidenglycinmethylester als Z-Isomeres vom Schmelzpunkt 132 °C

und als 2. Fraktion
0,6 g (13,5 % der Theorie) an N-[5-Methoxycarbonyl-4-(4-biphenylyl)-thiazol-2-yl]-N-methyl-2-methoxymethylidenglycinmethylester als E-Isomeres vom Schmelzpunkt 223 °C.

In entsprechender Weise und gemäß den allgemeinen Angaben zur Herstellung erhält man die folgenden substituierten Acrylsäureester der allgemeinen Formel (I):

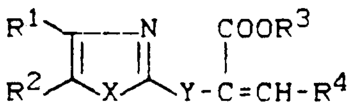

(I)

| Bsp. Nr. | $R^1$ | $R^2$ | $R^3$ | $R^4$ | X | Y | physikalische Eigenschaften |
|---|---|---|---|---|---|---|---|
| 3 | 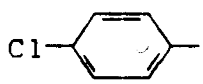 | Br | $CH_3$ | $OCH_3$ | S | $-\underset{CH_3}{\underset{\mid}{N}}-$ | $^1$H-NMR*): 3,26; 3,78; 3,97; 7,46; 7,5; 7,9 |
| 4 | 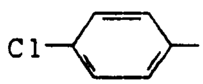 | $-\overset{\overset{O}{\|}}{C}-H$ | $CH_3$ | $OCH_3$ | S | $-\underset{CH_3}{\underset{\mid}{N}}-$ | $^1$H-NMR*): 3,37; 3,8; 4,0; 7,45; 7,5; 7,66; 9,68 |
| 5 | 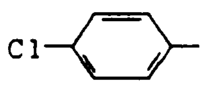 | -CN | $CH_3$ | $OCH_3$ | S | $-\underset{CH_3}{\underset{\mid}{N}}-$ | Fp. 129°C |

| Bsp. Nr. | $R^1$ | $R^2$ | $R^3$ | $R^4$ | X | Y | physikalische Eigenschaften |
|---|---|---|---|---|---|---|---|
| 6 | 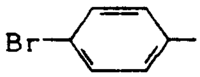 | 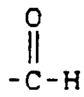 | $CH_3$ | $OCH_3$ | S | -N-<br>$CH_3$ | $^1H$-NMR*): 3,4; 3,8; 3,99; 7,2-7,7; 9,68 |
| 7 | 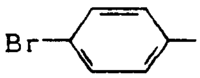 | -CN | $CH_3$ | $OCH_3$ | S | -N-<br>$CH_3$ | Fp.151-152° C |
| 8 | 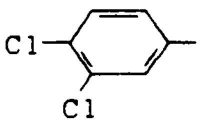 | -Br | $CH_3$ | $OCH_3$ | S | -N-<br>$CH_3$ | Fp.137° C |
| 9 | 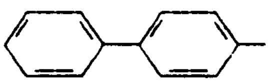 | Br | $CH_3$ | $OCH_3$ | S | -N-<br>$CH_3$ | |
| 10 | 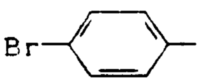 | Cl | $CH_3$ | $OCH_3$ | S | -N-<br>$CH_3$ | Fp. 108-110° C |
| 11 | 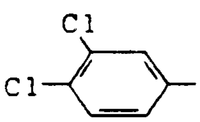 | Cl | $CH_3$ | $OCH_3$ | S | -N-<br>$CH_3$ | Fp. 136° C |
| 12 | 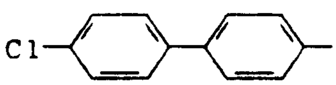 | Br | $CH_3$ | $OCH_3$ | S | -N-<br>$CH_3$ | |
| 13 | 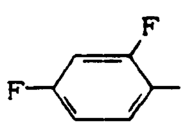 | Br | $CH_3$ | $OCH_3$ | S | -N-<br>$CH_3$ | Fp. 87-89° C |

| Bsp. Nr. | $R^1$ | $R^2$ | $R^3$ | $R^4$ | X | Y | physikalische Eigenschaften |
|---|---|---|---|---|---|---|---|
| 14 | 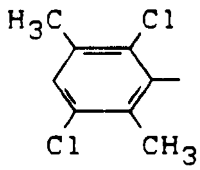 | Br | $CH_3$ | $OCH_3$ | S | -N-<br>\|<br>$CH_3$ | Fp. 111-112° C |
| 15 | 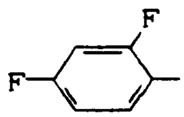 | Br | $CH_3$ | $OCH_3$ | S | -N-<br>\|<br>$CH_3$ | Fp. 77-79° C |
| 16 | 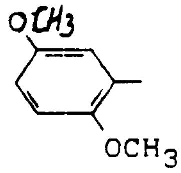 | Br | $CH_3$ | $OCH_3$ | S | -N-<br>\|<br>$CH_3$ | Fp. 87-88° C |
| 17 | 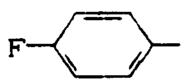 | Br | $CH_3$ | $OCH_3$ | S | -N-<br>\|<br>$CH_3$ | |
| 18 | 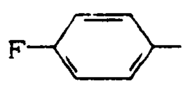 | Cl | $CH_3$ | $OCH_3$ | S | -N-<br>\|<br>$CH_3$ | |
| 19 | 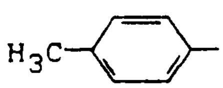 | Br | $CH_3$ | $OCH_3$ | S | -N-<br>\|<br>$CH_3$ | |
| 20 | 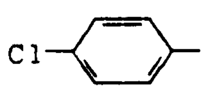 | Br | $CH_3$ | 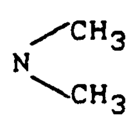 | S | -N-<br>\|<br>$CH_3$ | |

| Bsp. Nr. | $R^1$ | $R^2$ | $R^3$ | $R^4$ | X | Y | Physikalische Eigenschaften |
|---|---|---|---|---|---|---|---|
| 21 | 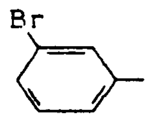 | Br | $CH_3$ | $OCH_3$ | S | -N-<br>$CH_3$ | Fp. 76-77° C |
| 22 | 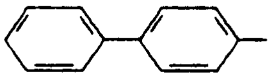 | $SCCl_2F$ | $CH_3$ | $OCH_3$ | S | -N-<br>$CH_3$ | |
| 23 | 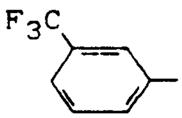 | Br | $CH_3$ | $OCH_3$ | S | -N-<br>$CH_3$ | Fp. 83-87° C |
| 24 | 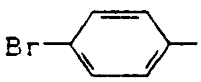 | $SCCl_2F$ | $CH_3$ | 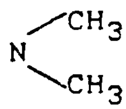 | S | -N-<br>$CH_3$ | Fp. 122-123° C |
| 25 | 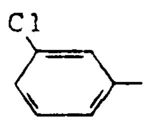 | Br | $CH_3$ | $OCH_3$ | S | -N-<br>$CH_3$ | |
| 26 | 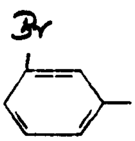 | Br | $CH_3$ | $OCH_3$ | S | -N-<br>$CH_3$ | Fp. 94-95° C |

*) Die $^{1}$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen

Beispiel IV-1

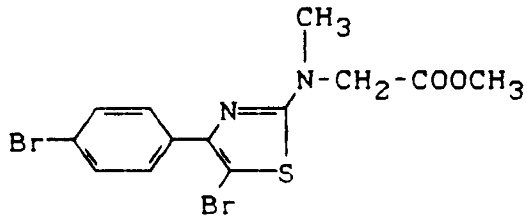

Zu einem Gemisch aus 4,5 g (0,013 Mol) N-[4-(4-Bromphenyl)-thiazol-2-yl]-N-methylaminoessigsäuremethylester und 2,5 g (0,03 Mol) Natriumacetat in 30 ml Eisessig gibt man unter Rühren bei Raumtemperatur tropfenweise im Verlauf von 45 Minuten eine Lösung von 2,34 g (0,015 Mol) Brom in 5 ml Eisessig, rührt anschließend mit überschüssiger wässriger gesättigter Natriumhydrogencarbonatlösung und saugt den ausgefallenen Niederschlag ab.

Nach dem Trocknen erhält man 5,4 g (97 % der Theorie) an N- [5-Brom-4-(4-bromphenyl)-thiazol-2-yl]-N-methylaminoessigsäuremethylester als Öl.

$^1$H-NMR ($CDCl_3$/Tetramethylsilan):

$\delta$ = 3,13 (s,3H); 3,75 (s,3H); 4,30 (s,2H); 7,51 (d,2H); 7,80 (d,2H) ppm.

Beispiel IV-2

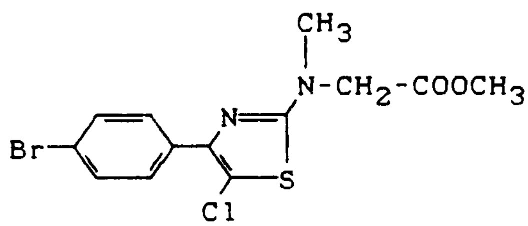

In eine Mischung aus 4,5 g (0,013 Mol) N-[4-(4-Bromphenyl)-thiazol-2-yl]-N-methylaminoessigsäuremethylester und 2,5 g (0,03 Mol) Natriumacetat in 30 ml Eisessig leitet man bei 35 °C unter Rühren so lange Chlorgas ein, bis im Dünnschichtchromatogramm des Reaktionsgemisches keine Ausgangsverbindung mehr nachweisbar ist. Danach rührt man die Mischung mit überschüssiger wässriger gesättigter Natriumhydrogencarbonatlösung, saugt den ausgefallenen Niederschlag ab und reinigt ihn durch Chromatographie an Kieselgel (Laufmittel: Dichlormethan/n-Hexan 1:1).

Man erhält 0,58 g (12 % der Theorie) an N-[5-Chlor-4-(4-bromphenyl)-thiazol-2-yl]-N-methylaminoessigsäuremethylester.

MS: m/e = 376 ($M^{\oplus}$); 317 (Basis-Peak; $M^{\oplus}$-$COOCH_3$).

Beispiel IV-3

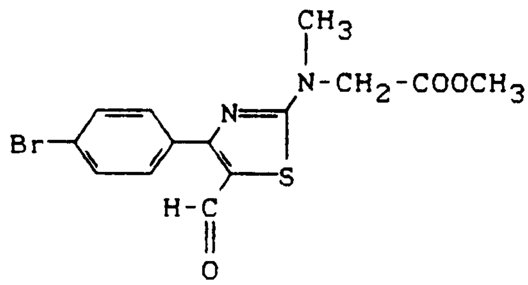

Zu einer Lösung aus 2,0 g (0,006 Mol) N-[4-(4-Bromphenyl)-thiazol-2-yl]-N-methylamino-essigsäuremethylester in 15 ml Dimethylformamid gibt man unter Rühren und Feuchtigkeitsausschluß, tropfenweise 5 g (0,03 Mol) Phosphoroxychlorid zu, so daß die Temperatur der Reaktionsmischung 45 °C nicht übersteigt. Nach beendeter Zugabe läßt man die Reaktionsmischung 2 Stunden bei Raumtemperatur stehen, gießt dann auf Eis, neutralisiert durch Zugabe von wässriger Natriumhydrogencarbonatlösung, saugt ab und trocknet.

Man erhält 1,62 g (71 % der Theorie) an N-[4-(4-Bromphenyl)-5-formylthiazol-2-yl]-N-methylaminoessigsäuremethylester als Monohydrat vom Schmelzpunkt 105-106 °C.

Beispiel IV-4

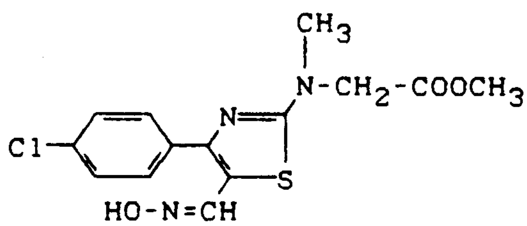

Ein Gemisch aus 5 g (0,0146 Mol) N-[4-(4-Chlorphenyl)-5-formylthiazol-2-yl)-N-methylaminoessigsäuremethylester und 1,6 g (0,023 Mol) Hydroxylaminhydrochlorid in 50 ml Ethanol und 5 ml Eisessig wird 45 Minuten lang unter Rühren auf Rückflußtemperatur erhitzt, abgekühlt, in Eis gegossen, neutralisiert durch Zugabe von Natriumhydrogencarbonat, mit Essigester extrahiert, die organische Phase über Natriumsulfat getrocknet und unter vermindertem Druck eingeengt.

Man erhält 4,56 g (92 % der Theorie) an N-[4-(4-Chlorphenyl)-5-hydroximinomethylthiazol-2-yl]-N-methylessigsäuremethylester vom Schmelzpunkt 147 °C bis 149 °C als Z/E-Isomerengemisch.

Beispiel IV-5

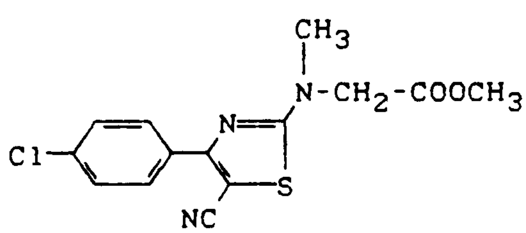

Ein Gemisch aus 4,16 g (0,0122 Mol) N-[4-(4-Chlorphenyl)-5-hydroximinomethylthiazol-2-yl]-N-methylaminoessigsäuremethylester und 30 ml Acetanhydrid wird 4 Stunden unter Rühren auf Rückflußtemperatur erhitzt, abgekühlt, in Eiswasser gegossen, in der Kälte mit gesättigter wässriger Natriumhydrogencarbonatlösung neutralisiert, mit Essigester extrahiert, die organische Phase über Natriumsulfat getrocknet, eingeengt und der kristalline Rückstand durch Verrühren mit einem Gemisch aus Diisopropylether und n-Hexan gereinigt.

Nach Absaugen und Trocknen erhält man 3,01 g (77 % der Theorie) an N-[4-(4-Chlorphenyl)-5-cyanothiazol-2-yl]-N-methylaminoessigsäuremethylester.

MS: m/e = 321 ($M^{\oplus}$); 262 (Basis-Peak; $M^{\oplus}$-$COOCH_3$).

Beispiel IV-6

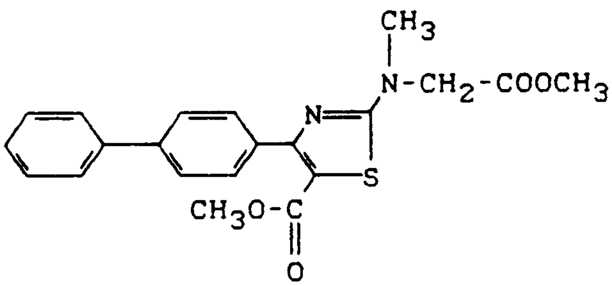

Zu einem Gemisch aus 8,1 g (0,025 Mol) 2-(N-Methylamino)-4-(4-biphenylyl)-thiazol-5-yl-carbonsäuremethylester und 30 ml Dimethylformamid gibt man unter Rühren und Kühlung portionsweise 0,9 g (0,03 Mol) Natriumhydrid, so daß die Temperatur der Reaktionsmischung 20 °C nicht übersteigt, rührt anschließend 45 Minuten bei Raumtemperatur und gibt dann tropfenweise unter Rühren bei Raumtemperatur im Verlauf von 30 Minuten 18,3 g (0,119 Mol) Bromessigsäuremethylester zu, rührt nach beendeter Zugabe weitere 12 Stunden bei Raumtemperatur, gießt dann das Reaktionsgemisch in Eiswasser, extrahiert mehrfach mit Dichlormethan, trocknet die vereinigten organischen Phasen über Natriumsulfat, engt unter vermindertem Druck ein und reinigt den kristallinen Rückstand durch Verrühren mit Diisopropylether.

Man erhält 5,1 g (52 % der Theorie) an N-[4-(4-Biphenylyl)-5-methoxycarbonylthiazol-2-yl)-N-methylaminoessigsäuremethylester vom Schmelzpunkt 141 °C.

In entsprechender Weise und gemäß den allgemeinen Angaben zur Herstellung erhält man die folgenden substituierten Essigsäureester der allgemeinen Formel (IV),

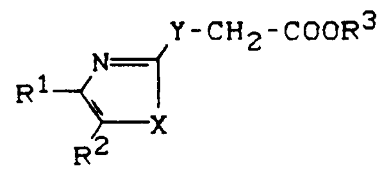

(IV)

| Bsp. Nr. | $R^1$ | $R^2$ | $R^3$ | X | Y | Schmelzpunkt/°C |
|---|---|---|---|---|---|---|
| IV-7 | 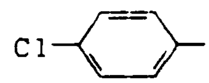 | Br | $CH_3$ | S | 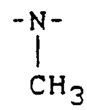 | 92-94 |
| IV-8 | 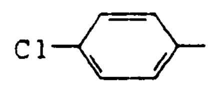 | 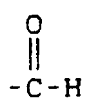 | $CH_3$ | S | 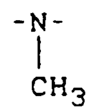 | 115-118 |
| IV-9 | 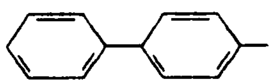 | Br | $CH_3$ | S | 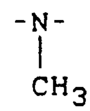 | 105-106 |
| IV-10 | 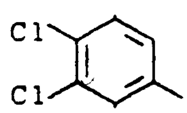 | Br | $CH_3$ | S | 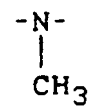 | 109 |
| IV-11 | 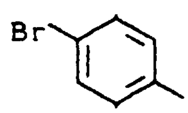 | CN | $CH_3$ | S | 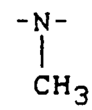 | 133-136 |

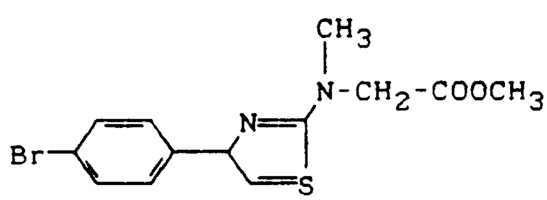

Zu 2,73 g (0,114 Mol) Natriumhydrid in 200 ml Dimethylformamid gibt man tropfenweise unter Rühren, sowie unter Feuchtigkeitsausschluß und Eiskühlung eine Lösung aus 9,0 g (0,036 Mol) 4-(4-Bromphenyl)-2-methylaminothiazol in 50 ml Dimethylformamid, so daß die Temperatur der Reaktionsmischung 10 °C nicht übersteigt. Nach beendeter Zugabe rührt man eine Stunde bei Raumtemperatur, kühlt dann auf 10 °C ab und gibt tropfenweise unter Rühren im Verlauf von einer Stunde 21,4 g (0,14 Mol) Bromessigsäuremethylester zu, rührt anschließend 15 Stunden bei Raumtemperatur, gibt dann die Reaktionsmischung in Eiswasser, extrahiert mehrfach mit Dichlormethan, trocknet die vereinigten organischen Phasen über Natriumsulfat, engt unter vermindertem Druck ein und reinigt den Rückstand durch Chromatographie an Kieselgel (Laufmittel:Dichlormethan).

Man erhält 10,0 g (42 % der Theorie) an N-[4-(4-Bromphenyl)-thiazol-2-yl]-N-methylaminoessigsäuremethylester vom Schmelzpunkt 95 °C.

In entsprechender Weise und gemäß den allgemeinen Angaben zur Herstellung erhält man

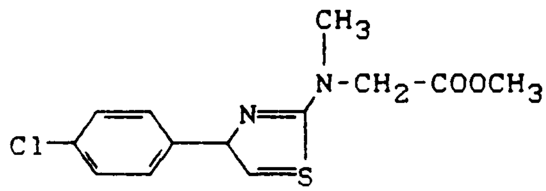

Schmelzpunkt 86 °C.

Beispiel XI-1:

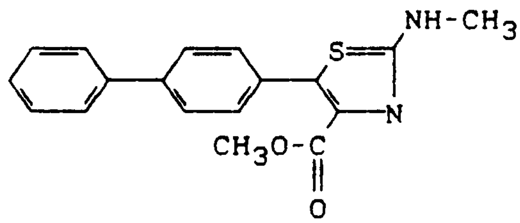

Eine Mischung von 17,1 g (0,06 Mol) 2-Chlor-2-(4-phenylbenzoyl)-essigsäuremethylester (durch Chlorierung mit Sulfurylchlorid nach Standardverfahren; vgl. auch DE-OS 23 43 974) 10,8 g (0,12 Mol) N-Methylthioharnstoff und 200 ml Methanol wird 3 Stunden auf Rückflußtemperatur erhitzt, abgekühlt, in überschüssige verdünnte wässrige Natriumhydrogencarbonatlösung eingerührt, der ausgefallene Niederschlag abgesaugt und getrocknet.

Man erhält 18,2 g (95 % der Theorie) 2-(N-Methylamino)-4-(4-biphenylyl)-thiazol-5-ylcarbonsäuremethylester vom Schmelzpunkt >230 °C.

Beispiel XI-2

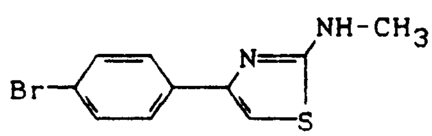

Ein Gemisch aus 27,8 g (0,1 Mol) 2,4'-Dibromacetophenon (vgl. z.B. Tetrahedron Lett. 1975, 373-376), 9,0 g (0,115 Mol) N-Methylthioharnstoff und 400 ml Ethanol wird 3 Stunden auf Rückflußtemperatur erhitzt, anschließend abgekühlt, in überschüssige verdünnte wässrige Natriumhydrogencarbonatlösung gegeben, mit Dichlormethan extrahiert, die vereinigten organischen Phasen über Natriumsulfat getrocknet und eingeengt.

Man erhält 20,8 g (78 % der Theorie) an 2-(N-Methylamino)-4-(4-bromphenyl)-thiazol vom Schmelzpunkt 147 °C.

In entsprechender Weise erhält man Beispiel XI-3:

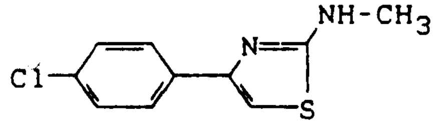

Schmelzpunkt 140 °C

Anwendungsbeispiele

In den folgenden Anwendungsbeispielen wurde die nachstehend aufgeführte Verbindung als Vergleichsubstanz eingesetzt:

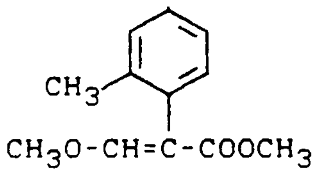

(A)

3-Methoxy-2-(2-methylphenyl)-acrylsäuremethylester
(bekannt aus EP 178 826)

Beispiel A

| Venturia-Test (Apfel) /protektiv | |
|---|---|
| Lösungsmittel:<br>Emulgator: | 4,7 Gewichtsteile Aceton<br>0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen 1 und 3.

Beispiel B

| Pyrenophora teres-Test (Gerste) / protektiv | |
|---|---|
| Lösungsmittel:<br>Emulgator: | 100 Gewichtsteile Dimethylformamid<br>0,25 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigt bei diesem Test z.B. die Verbindung gemäß Herstellungsbeispiel 3.

Beispiel C

| Pyricularia-Test (Reis) /protektiv | |
|---|---|
| Lösungsmittel: | 12,5 Gewichtsteile Aceton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen 1 und 3.

**Patentansprüche**

**Patentansprüche für folgende Vertragsstaaten : BE, CH, LI, DE, DK, FR, GB, IT, NL**

**1.** Substituierte Acrylsäureester der allgemeinen Formel (I),

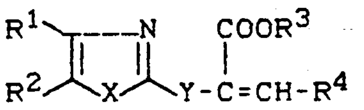

(I)

in welcher

$R^1$ für Wasserstoff, für geradkettiges oder verzweigtes Alkyl mit 1 bis 8 Kohlenstoffatomen, für geradkettiges oder verzweigtes Alkenyl mit 2 bis 8 Kohlenstoffatomen, für jeweils gegebenenfalls im Arylteil einfach bis mehrfach, gleich oder verschieden substituiertes Aralkyl mit 1 bis 6 Kohlenstoffatomen im geradkettigen oder verzweigten Alkylteil, Aralkenyl mit 2 bis 6 Kohlenstoffatomen im geradkettigen oder verzweigten Alkenylteil oder Aryl mit 6 bis 10 Kohlenstoffatomen im jeweiligen Arylteil steht, wobei als Arylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach bis mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach bis mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen im Heteroarylteil und gegebenenfalls 1 bis 4 Kohlenstoffatomen im geradketti-

gen oder verzweigten Alkylteil;

$R^1$ außerdem für einen gegebenenfalls einfach bis mehrfach, gleich oder verschieden substituierten Heteroarylrest mit 2 bis 9 Kohlenstoffatomen und 1 bis 4 gleichen oder verschiedenen Heteroatomen steht, wobei als Substituenten die oben genannten Arylsubstituenten infrage kommen,

$R^2$ für Fluor, Chlor, Brom, Iod, Cyano, Nitro, Formyl, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkoxyalkyl oder Alkylthioalkyl mit jeweils 1 bis 4 Kohlenstoffatomen in den einzelnen Alkylteilen, für jeweils geradkettiges oder verzweigtes Hydroximinoalkyl, Alkoximinoalkyl, N-Alkyliminoalkyl oder N,N-Dialkylhydrazonoalkyl mit jeweils 1 bis 4 Kohlenstoffatomen in den einzelnen Alkylteilen, für jeweils geradkettiges oder verzweigtes Alkoxy oder Alkylthio mit jeweils 1 bis 4 Kohlenstoffatomen, für jeweils geradkettiges oder verzweigtes Halogenalkoxy oder Halogenalkylthio mit jeweils 1 bis 4 Kohlenstoffatomen und jeweils 1 bis 9 gleichen oder verschiedenen Halogenatomen, für jeweils geradkettiges oder verzweigtes Alkanoyl, Alkoxycarbonyl oder N,N-Dialkylcarbamoyl mit jeweils 1 bis 4 Kohlenstoffatomen in den einzelnen Alkylteilen oder für Heterocyclylcarbonyl steht, wobei als Heterocyclylrest ein gesättigter fünf- bis siebengliedriger N-verknüpfter Heterocyclus infrage kommt, der gegebenenfalls ein weiteres Heteroatom enthalten kann und der gegebenenfalls ein- bis vierfach durch Methyl und/oder Ethyl substituiert sein kann;

$R^2$ außerdem für jeweils gegebenenfalls einfach bis mehrfach, gleich oder verschieden im Arylteil mit jeweils 6 bis 10 Kohlenstoffatomen substituiertes N-Aryliminoalkyl, Aryloxy oder Arylthio steht, wobei der geradkettige oder verzweigte Alkylteil 1 bis 4 Kohlenstoffatome hat und wobei als Arylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen und/oder geradkettiges oder verzweigtes Alkyl mit 1 bis 4 Kohlenstoffatomen substituiertes Phenyl,

$R^3$ für geradkettiges oder verzweigtes Alkyl mit 1 bis 6 Kohlenstoffatomen oder für gegebenenfalls im Arylteil einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ genannten infrage kommen,

$R^4$ für Dialkylamino mit jeweils 1 bis 6 Kohlenstoffatomen in den einzelnen geradkettigen oder verzweigten Alkylteilen steht oder für einen Rest -Z-$R^5$ steht,

X für Sauerstoff oder Schwefel steht und

Y für Sauerstoff, Schwefel oder für einen Rest

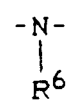

steht,

wobei

$R^5$ für geradkettiges oder verzweigtes Alkyl mit 1 bis 6 Kohlenstoffatomen oder für gegebenenfalls im Arylteil einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ genannten infrage kommen,

$R^6$ für Wasserstoff, für geradkettiges oder verzweigtes Alkyl mit 1 bis 6 Kohlenstoffatomen, für geradkettiges oder verzweigtes Alkanoyl mit 1 bis 6 Kohlenstoffatomen im Alkylteil oder für jeweils gegebenenfalls im Arylteil einfach bis mehrfach, gleich oder verschieden substituiertes Aralkyl mit 1 bis 6 Kohlenstoffatomen im geradkettigen oder verzweigten Alkylteil oder Aryl mit jeweils 6 bis 10 Kohlenstoffatomen im jeweiligen Arylteil steht, wobei als Substituenten im Arylteil jeweils die bei $R^1$ genannten infrage kommen und

Z für Sauerstoff oder Schwefel steht,

deren geometrische Isomere und deren Gemische.

**2.** Substituierte Acrylsäureester der Formel (I) gemäß Anspruch 1, worin

$R^1$ für Wasserstoff, Methyl, Ethyl, n- oder i-Propyl,n-, i-, s- oder t-Butyl, für Allyl, n- oder i-Butenyl, für jeweils gegebenenfalls im Arylteil oder im Heteroarylteil ein- bis vierfach, gleich oder verschieden substituiertes Benzyl, Phenylethyl, Phenylethenyl, Phenyl, Naphthyl, Pyridyl, Thienyl oder Furyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy oder Benzyloxy,

$R^2$ für Chlor, Brom, Iod, Cyano, Nitro, Formyl, Chlormethyl, Brommethyl, Iodmethyl, Methoxymethyl, Ethoxymethyl, n- oder i-Propoxymethyl, Methylthiomethyl, Ethylthiomethyl, n- oder i-Propylthiomethyl, für Hydroximinomethyl, Hydroximinoethyl, Hydroximinopropyl, für Methoximinomethyl, Ethoximinomethyl, n- oder i-Propoximinomethyl, Methoximinoethyl, Ethoximinoethyl, n- oder i-Propoximinoethyl, für Methyliminomethyl, Ethyliminomethyl, n-oder i-Propyliminomethyl, für Methyliminoethyl, Ethyliminoethyl, n- oder i-Propyliminoethyl, für N,N-Dimethylhydrazonomethyl, N,N-Diethylhydrazonomethyl, N-Methyl-N-ethylhydrazonomethyl, N-Methyl-N-propylhydrazonomethyl, N,N-Dipropylhydrazonomethyl, N,N-Dimethylhydrazonoethyl, N,N-Diethylhydrazonoethyl, für Methoxy, Ethoxy, n- oder i-Propoxy, Methylthio, Ethylthio, n- oder i-Propylthio, für Trifluormethoxy, Difluormethoxy, Dichlorfluormethoxy, Difluorchlormethoxy, Trichlormethoxy, Trifluormethylthio, Trichlormethylthio, Difluormethylthio, Dichlorfluormethylthio, Difluorchlormethylthio, für Acetyl, Propionyl, Butyryl, Methoxycarbonyl, Ethoxycarbonyl, n-oder i-Propoxycarbonyl, N,N-Dimethylaminocarbonyl, N,N-Diethylaminocarbonyl, N,N-Dipropylaminocarbonyl, N-Methyl-N-ethylaminocarbonyl, N-Methyl-N-propyl-aminocarbonyl, für 1-Piperidinylcarbonyl, 4-Morpholinylcarbonyl oder für jeweils gegebenenfalls im Phenylteil ein- bis dreifach, gleich oder verschieden substituiertes N-Phenyliminomethyl, N-Phenyliminoethyl, Phenoxy oder Phenylthio steht,
wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/oder Ethyl substituiertes Phenyl,

$R^3$ für Methyl, Ethyl, n- oder i-Propyl, n-, i-, s-oder t-Butyl oder für gegebenenfalls ein-bis dreifach, gleich oder verschieden substituiertes Benzyl steht, wobei als Substituenten die bei $R^1$ genannten infrage kommen,

$R^4$ für Dialkylamino mit jeweils 1 bis 4 Kohlenstoffatomen in den einzelnen geradkettigen oder verzweigten Alkylteilen steht oder für einen Rest -Z-$R^5$ steht,

X für Sauerstoff oder Schwefel steht und

Y für Sauerstoff, Schwefel oder für einen Rest

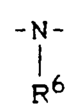

steht,
wobei

$R^5$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^1$ genannten infrage kommen;

$R^6$ für Wasserstoff, Methyl, Ethyl, n- oder i-Propyl, n-, i-, s- oder t-Butyl, für Acetyl, Propionyl, n- oder i-Butyryl oder für jeweils gegebenenfalls ein- bis dreifach, gleich oder verschieden substituiertes Benzyl oder Phenyl steht, wobei als Substituenten die bei $R^1$ genannten infrage kommen und

Z für Sauerstoff oder Schwefel steht.

**3.** Substituierte Acrylsäureester der Formel (I) gemäß Anspruch 1, worin

$R^1$ für Wasserstoff, Methyl, Ethyl, n- oder i-Propyl oder für jeweils gegebenenfalls ein- bis vierfach, gleich oder verschieden substituiertes Phenyl oder Naphthyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls ein- oder zweifach, gleich oder verschieden durch Fluor, Chlor, Brom, Methyl und/oder Ethyl substituiertes Phenyl, Phenoxy, Benzyl oder Benzyloxy,

$R^2$ für Chlor, Brom, Cyano, Nitro, Formyl, Methoxymethyl, Ethoxymethyl, Methylthiomethyl, Hydroximinomethyl, Methoximinomethyl, Ethoximinomethyl, Methyliminomethyl, Ethyliminomethyl, N,N-Dimethylhydrazonomethyl, N,N-Diethylhydrazonomethyl, für Methoxy, Ethoxy, Methylthio, Ethylthio, Trifluormethylthio, Dichlorfluormethylthio, Difluorchlormethylthio, Trichlormethylthio, für Acetyl, Methoxycarbonyl, Ethoxycarbonyl, N,N-Dimethylaminocarbonyl, N,N-Diethylaminocarbonyl, N-Methyl-N-ethylaminocarbonyl oder für gegebenenfalls einfach oder zweifach, gleich oder verschieden substituiertes Phenylthio steht,

wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio,

$R^3$ für Methyl, Ethyl oder Benzyl steht,

$R^4$ für Dimethylamino, Diethylamino oder für einen Rest -Z-$R^5$ steht,

X für Sauerstoff oder Schwefel steht und

Y für Sauerstoff, Schwefel oder für einen Rest

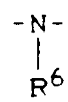

steht,

wobei

$R^5$ für Methyl, Ethyl, n- oder i-Propyl oder Benzyl steht,

$R^6$ für Wasserstoff, Methyl, Ethyl, Acetyl, Propionyl oder für jeweils gegebenenfalls einfach oder zweifach, gleich oder verschieden durch Fluor, Chlor, Brom, Methyl, Ethyl und/oder Trifluormethyl substituiertes Benzyl oder Phenyl steht und

Z für Sauerstoff oder Schwefel steht.

**4.** Substituierte Acrylsäureester der Formel (I) gemäß Anspruch 1, worin

$R^1$ für Wasserstoff, Methyl, Ethyl, n- oder i-Propyl oder für gegebenenfalls einfach oder zweifach, gleich oder verschieden substituiertes Phenyl steht, wobei als Substituenten infrage kommen:

Fluor, Chlor, Brom, Cyano, Nitro, Methyl, Ethyl, Methoxy, Ethoxy, Methylthio, Trifluormethyl, Trifluormethoxy, Trifluormethylthio, Methoxycarbonyl, Ethoxycarbonyl, Methoximinomethyl, Cyclopentyl, 1,3-Propandiyl, Methoximinoethyl oder gegebenenfalls ein- oder zweifach, gleich oder verschieden durch Fluor, Chlor, Brom oder Methyl substituiertes Phenyl, Phenoxy, Benzyl oder Benzyloxy,

$R^2$ für Chlor, Brom, Cyano, Nitro, Formyl, Methoxymethyl, Methylthiomethyl, Methoxycarbonyl, Ethoxycarbonyl, Methylthio, Trifluormethylthio oder Methoximinomethyl steht,

$R^3$ für Methyl oder Ethyl steht,

$R^4$ für Methoxy, Ethoxy, Methylthio oder Dimethylamino steht,

X für Sauerstoff oder Schwefel steht und

Y für einen N-Methyl-Rest steht.

**5.** Verfahren zur Herstellung von substituierten Acrylsäureestern der allgemeinen Formel (I),

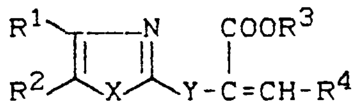

(I)

in welcher

$R^1$, $R^2$, $R^3$, $R^4$, X und Y  die in Anspruch 1 angegebene Bedeutung haben

dadurch gekennzeichnet, daß man

(a) substituierte Acrylsäureester der Formel (Ia),

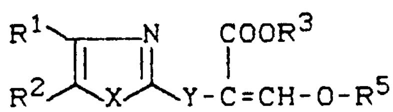

(Ia)

in welcher

$R^1$, $R^2$, $R^3$, $R^5$, X und Y  die oben angegebene Bedeutung haben, erhält,

indem man Hydroxyacrylsäureester oder deren Alkalimetallsalze der Formel (II),

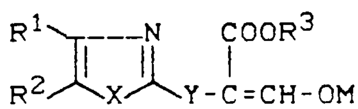

(II)

in welcher

M  für Wasserstoff oder für ein Alkalimetallkation steht und

$R^1$, $R^2$, $R^3$, X und Y  die oben angegebene Bedeutung haben,

mit Alkylierungsmitteln der Formel (III),

$R^5$-$E^1$  (III)

in welcher

$E^1$  für eine elektronenanziehende Abgangsgruppe steht und

$R^5$  die oben angegebene Bedeutung hat,

gegebenenfalls in Gegenwart eines Verdünnungsmittels und gegebenenfalls in Gegenwart eines Reaktionshilfsmittels umsetzt oder

(b) substituierte Acrylsäureester der Formel (Ib),

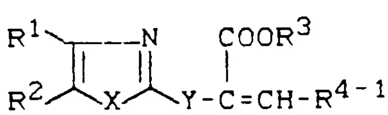

(Ib)

in welcher

$R^{4-1}$  für Dialkylamino steht und

$R^1$, $R^2$, $R^3$, X und Y  die oben angegebene Bedeutung haben, erhält,

indem man substituierte Essigsäureester der Formel (IV),

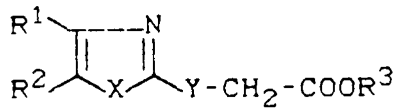 (IV)

in welcher

$R^1$, $R^2$, $R^3$, X und Y die oben angegebene Bedeutung haben,

mit Formamiden der Formel (Va),

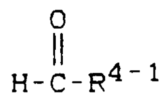 (Va)

in welcher

$R^{4-1}$ die oben angegebene Bedeutung hat,

oder mit deren Derivaten der Formel (Vb),

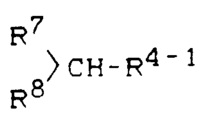 (Vb)

in welcher

$R^7$ und $R^8$ unabhängig voneinander jeweils für Alkoxy oder Dialkylamino stehen und

$R^{4-1}$ die oben angegebene Bedeutung hat,

gegebenenfalls in Gegenwart eines Verdünnungsmittel umsetzt oder

(c) substituierte Acrylsäureester der Formel (Ic),

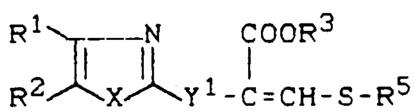 (Ic)

in welcher

$Y^1$ für Schwefel oder für einen Rest

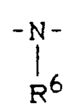

steht,

und

$R^1$, $R^2$, $R^3$, $R^5$, $R^6$ und X die oben angegebene Bedeutung haben, erhält,

indem man Oxalsäurederivate der Formel (VI),

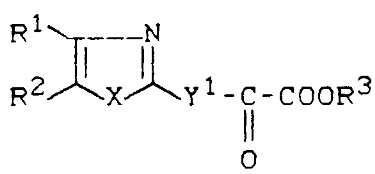 (VI)

in welcher
$R^1$, $R^2$, $R^3$, X und $Y^1$ die oben angegebene Bedeutung haben,
mit metallorganischen Verbindungen der Formel (VII),

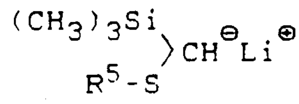

(VII)

in welcher
$R^5$ die oben angegebene Bedeutung hat,
gegebenenfalls in Gegenwart eines Verdünnungsmittels umsetzt oder
(d) substituierte Acrylsäureester der Formel (Id),

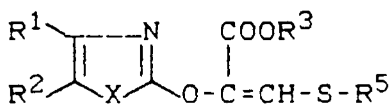

(Id)

in welcher
$R^1$, $R^2$, $R^3$, $R^5$ und X die oben angegebene Bedeutung haben, erhält,
indem man substituierte Acrylsäureester der Formel (VIII),

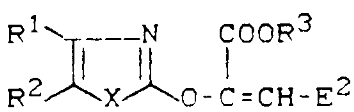

(VIII)

in welcher
$E^2$ für eine elektronenanziehende Abgangsgruppe steht und
$R^1$, $R^2$, $R^3$ und X die oben angegebene Bedeutung haben,
mit Thiolen der Formel (IX),

$R^5$-SH (IX)

in welcher
$R^5$ die oben angegebene Bedeutung hat,
gegebenenfalls in Gegenwart eines Verdünnungsmittels und gegebenenfalls in Gegenwart eines Reaktionshilfsmittels umsetzt.

**6.** Schädlingsbekämpfungsmittel, gekennzeichnet durch einen Gehalt an mindestens einem substituierten Acrylsäureester der Formel (I) nach den Ansprüchen 1 bis 4.

**7.** Verwendung von substituierten Acrylsäureestern der Formel (I) nach den Ansprüchen 1 bis 4 zur Bekämpfung von Schädlingen.

**8.** Verfahren zur Bekämpfung von Schädlingen, dadurch gekennzeichnet, daß man substituierte Acrylsäureester der Formel (I) nach den Ansprüchen 1 bis 4 auf Schädlinge und/oder ihren Lebensraum einwirken läßt.

**9.** Verfahren zur Herstellung von Schädlingsbekämpfungsmitteln, dadurch gekennzeichnet, daß man substituierte Acrylsäureester der Formel (I) nach den Ansprüchen 1 bis 4 mit Streckmitteln und/oder oberflächenaktiven Mitteln vermischt.

**10.** Hydroxyacrylsäureester oder deren Alkalimetallsalze der Formel (II),

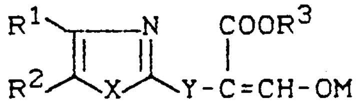

(II)

in welcher

| | |
|---|---|
| $R^1$, $R^2$, $R^3$, X und Y | die in Anspruch 1 angegebene Bedeutung haben und |
| M | für Wasserstoff oder für ein Alkalimetallkation steht. |

**11.** Verfahren zur Herstellung von Hydroxyacrylsäureestern oder deren Alkalimetallsalzen der Formel (II)

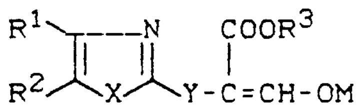

(II)

in welcher

$R^1$, $R^2$, $R^3$, X, Y und M die in Anspruch 10 angegebene Bedeutung haben,

dadurch gekennzeichnet, daß man substituierte Essigsäureester der Formel (IV),

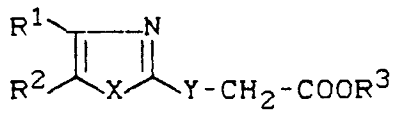

(IV)

in welcher

$R^1$, $R^2$, $R^3$, X und Y die oben angegebene Bedeutung haben,

mit Ameisensäureestern der Formel (X),

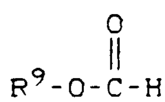

(X)

in welcher

$R^9$ für Alkyl steht,

gegebenenfalls in Gegenwart eines Verdünnungsmittels und gegebenenfalls in Gegenwart eines basischen Reaktionshilfsmittels bei Temperaturen zwischen -20 °C und +50 °C umsetzt.

**12.** Substituierte Essigsäureester der Formel (IVa),

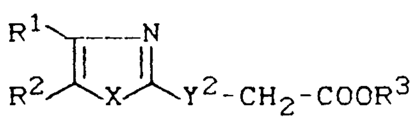

(IVa)

in welcher

| | |
|---|---|
| $R^1$, $R^2$, $R^3$, und X | die in Anspruch 1 angegebene Bedeutung haben und |
| $Y^2$ | für Sauerstoff, Schwefel oder für einen N-Alkylrest steht. |

**13.** Verfahren zur Herstellung von substituierten Essigsäureestern der Formel (IVa)

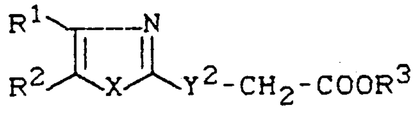

(IVa)

in welcher

$R^1$, $R^2$, $R^3$, $Y^2$ und X die in Anspruch 12 angegebene Bedeutung haben,

dadurch gekennzeichnet, daß man Thiazol- oder Oxazolderivate der Formel (XIa),

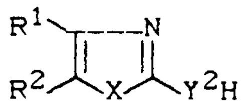

(XIa)

in welcher

$R^1$, $R^2$,X und $Y^2$ die oben angegebene Bedeutung haben,

oder Thiazol- oder Oxazolderivate der Formel (XIb),

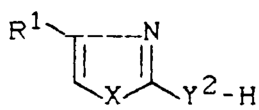

(XIb)

in welcher

$R^1$, X und $Y^2$ die oben angegebene Bedeutung haben,

mit Bromessigestern der Formel (XII),

$Br-CH_2-COOR^3$ (XII)

in welcher

$R^3$ die oben angegebene Bedeutung hat,

gegebenenfalls in Gegenwart eines Verdünnungsmittels und gegebenenfalls in Gegenwart eines Reaktionshilfsmittels bei Temperaturen zwischen - 20 °C und + 150 °C umsetzt und gegebenenfalls anschließend in 5-Position des Thiazol- bzw. Oxazolringes mit üblichen elektrophilen Substitutionsreaktionen substituiert oder bereits vorhandene funktionelle Gruppen in dieser Position mit Hilfe allgemein bekannter Verfahren derivatisiert.

**14.** Substituierte Acrylsäureester der Formel (VIII),

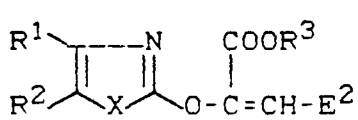

(VIII)

in welcher

$R^1$, $R^2$, $R^3$ und X die in Anspruch 1 angegebene Bedeutung haben, und

$E^2$ für eine elektronenanziehende Abgangsgruppe steht.

**15.** Verfahren zur Herstellung von substituierten Acrylsäureestern der Formel (VIII)

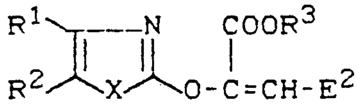

(VIII)

in welcher

$R^1$, $R^2$, $R^3$, X und $E^2$ die in Anspruch 14 angegebene Bedeutung haben,

dadurch gekennzeichnet, daß man Hydroxyacrylsäureester der Formel (IIb),

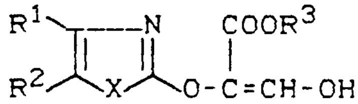

(IIb)

in welcher

$R^1$, $R^2$, $R^3$ und X die oben angegebene Bedeutung haben,

mit Säurechloriden der Formel (XIV),

$R^{10}$-Cl (XIV)

in welcher

$R^{10}$ für einen Acyl- oder Sulfonylrest steht,

gegebenenfalls in Gegenwart eines Verdünnungsmittels und gegebenenfalls in Gegenwart eines Säurebindemittels bei Temperaturen zwischen -20 °C und +120 °C umsetzt.

**16.** Verwendung von Verbindungen der Formeln (II), (IVa) und (VIII) gemäß den Ansprüchen 10, 12 und 14 als Zwischenprodukte.

**Patentansprüche für folgenden Vertragsstaat : ES**

**1.** Verfahren zur Herstellung von substituierten Acrylsäureestern der allgemeinen Formel (I),

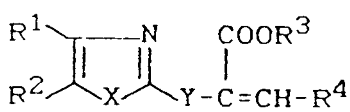

(I)

in welcher

$R^1$ für Wasserstoff, für geradkettiges oder verzweigtes Alkyl mit 1 bis 8 Kohlenstoffatomen, für geradkettiges oder verzweigtes Alkenyl mit 2 bis 8 Kohlenstoffatomen, für jeweils gegebenenfalls im Arylteil einfach bis mehrfach, gleich oder verschieden substituiertes Aralkyl mit 1 bis 6 Kohlenstoffatomen im geradkettigen oder verzweigten Alkylteil, Aralkenyl mit 2 bis 6 Kohlenstoffatomen im geradkettigen oder verzweigten Alkenylteil oder Aryl mit 6 bis 10 Kohlenstoffatomen im jeweiligen Arylteil steht, wobei als Arylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach bis mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach bis mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen im Heteroarylteil und gegebenenfalls 1 bis 4 Kohlenstoffatomen im geradkettigen oder verzweigten Alkylteil;

$R^1$ außerdem für einen gegebenenfalls einfach bis mehrfach, gleich oder verschieden substituierten Heteroarylrest mit 2 bis 9 Kohlenstoffatomen und 1 bis 4 gleichen oder verschiedenen Heteroatomen steht, wobei als Substituenten die oben genannten Arylsubstituenten infrage kommen,

$R^2$ für Fluor, Chlor, Brom, Iod, Cyano, Nitro, Formyl, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkoxyalkyl oder Alkylthioalkyl mit jeweils 1 bis 4 Kohlenstoffatomen in den einzelnen Alkylteilen, für jeweils geradkettiges oder verzweigtes Hydroximinoalkyl, Alkoximinoalkyl, N-Alkyliminoalkyl oder N,N-Dialkylhydrazonoalkyl mit jeweils 1 bis 4 Kohlenstoffatomen in den einzelnen Alkylteilen, für jeweils geradkettiges oder verzweigtes Alkoxy oder Alkylthio mit jeweils 1 bis 4 Kohlenstoffatomen, für jeweils geradkettiges oder verzweigtes Halogenalkoxy oder Halogenalkylthio mit jeweils 1 bis 4 Kohlenstoffatomen und jeweils 1 bis 9 gleichen oder verschiedenen Halogenatomen, für jeweils geradkettiges oder verzweigtes Alkanoyl, Alkoxycarbonyl oder N,N-Dialkylcarbamoyl mit jeweils 1 bis 4 Kohlenstoffatomen in den einzelnen Alkylteilen oder für Heterocyclylcarbonyl steht, wobei als Heterocyclylrest ein gesättigter fünf- bis siebengliedriger N-verknüpfter Heterocyclus infrage kommt, der gegebenenfalls ein weiteres Heteroatom enthalten kann und der gegebenenfalls ein- bis vierfach durch Methyl und/oder Ethyl substituiert sein kann;

$R^2$ außerdem für jeweils gegebenenfalls einfach bis mehrfach, gleich oder verschieden im Arylteil mit jeweils 6 bis 10 Kohlenstoffatomen substituiertes N-Aryliminoalkyl, Aryloxy oder Arylthio steht, wobei der geradkettige oder verzweigte Alkylteil 1 bis 4 Kohlenstoffatome hat und wobei als Arylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen und/oder geradkettiges oder verzweigtes Alkyl mit 1 bis 4 Kohlenstoffatomen substituiertes Phenyl,

$R^3$ für geradkettiges oder verzweigtes Alkyl mit 1 bis 6 Kohlenstoffatomen oder für gegebenenfalls im Arylteil einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ genannten infrage kommen,

$R^4$ für Dialkylamino mit jeweils 1 bis 6 Kohlenstoffatomen in den einzelnen geradkettigen oder verzweigten Alkylteilen steht oder für einen Rest $-Z-R^5$ steht,

X für Sauerstoff oder Schwefel steht und

Y für Sauerstoff, Schwefel oder für einen Rest

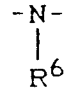

steht,

wobei

$R^5$ für geradkettiges oder verzweigtes Alkyl mit 1 bis 6 Kohlenstoffatomen oder für gegebenenfalls im Arylteil einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ genannten infrage kommen,

$R^6$ für Wasserstoff, für geradkettiges oder verzweigtes Alkyl mit 1 bis 6 Kohlenstoffatomen, für geradkettiges oder verzweigtes Alkanoyl mit 1 bis 6 Kohlenstoffatomen im Alkylteil oder für jeweils gegebenenfalls im Arylteil einfach bis mehrfach, gleich oder verschieden substituiertes Aralkyl mit 1 bis 6 Kohlenstoffatomen im geradkettigen oder verzweigten Alkylteil oder Aryl mit jeweils 6 bis 10 Kohlenstoffatomen im jeweiligen Arylteil steht, wobei als Substituenten im Arylteil jeweils die bei $R^1$ genannten infrage kommen und

Z für Sauerstoff oder Schwefel steht,

deren geometrische Isomere und deren Gemische,

dadurch gekennzeichnet, daß man

(a) substituierte Acrylsäureester der Formel (Ia),

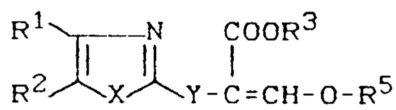

(Ia)

in welcher

$R^1$, $R^2$, $R^3$, $R^5$, X und Y die oben angegebene Bedeutung haben, erhält,

indem man Hydroxyacrylsäureester oder deren Alkalimetallsalze der Formel (II),

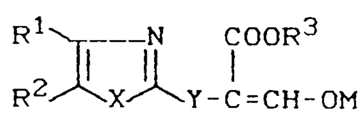

(II)

in welcher

M für Wasserstoff oder für ein Alkalimetallkation steht und

$R^1$, $R^2$, $R^3$, X und Y die oben angegebene Bedeutung haben,

mit Alkylierungsmitteln der Formel (III),

$R^5$-$E^1$ (III)

in welcher

$E^1$ für eine elektronenanziehende Abgangsgruppe steht und

$R^5$ die oben angegebene Bedeutung hat,

gegebenenfalls in Gegenwart eines Verdünnungsmittels und gegebenenfalls in Gegenwart eines Reaktionshilfsmittels umsetzt oder

(b) substituierte Acrylsäureester der Formel (Ib),

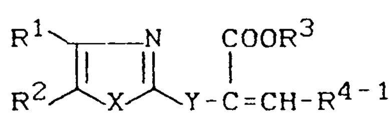

(Ib)

in welcher

$R^{4-1}$ für Dialkylamino steht und

$R^1$, $R^2$, $R^3$, X und Y die oben angegebene Bedeutung haben, erhält,

indem man substituierte Essigsäureester der Formel (IV),

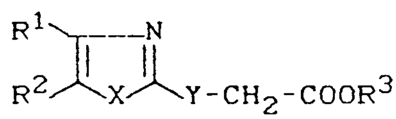

(IV)

in welcher
$R^1$, $R^2$, $R^3$, X und Y die oben angegebene Bedeutung haben,
mit Formamiden der Formel (Va),

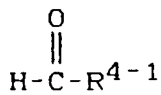
(Va)

in welcher
$R^{4-1}$ die oben angegebene Bedeutung hat,
oder mit deren Derivaten der Formel (Vb),

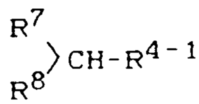
(Vb)

in welcher
$R^7$ und $R^8$ unabhängig voneinander jeweils für Alkoxy oder Dialkylamino stehen und
$R^{4-1}$ die oben angegebene Bedeutung hat,
gegebenenfalls in Gegenwart eines Verdünnungsmittel umsetzt oder
(c) substituierte Acrylsäureester der Formel (Ic),

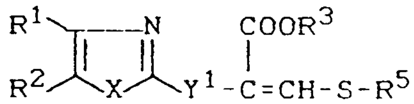
(Ic)

in welcher
$Y^1$ für Schwefel oder für einen Rest

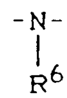

steht,
und
$R^1$, $R^2$, $R^3$, $R^5$, $R^6$ und X die oben angegebene Bedeutung haben, erhält,
indem man Oxalsäurederivate der Formel (VI),

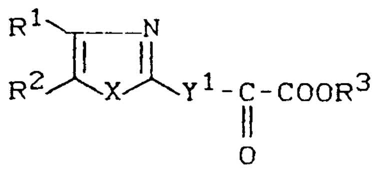
(VI)

in welcher
$R^1$, $R^2$, $R^3$, X und $Y^1$ die oben angegebene Bedeutung haben,
mit metallorganischen Verbindungen der Formel (VII),

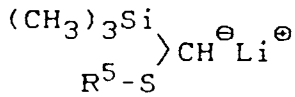 (VII)

in welcher
$R^5$ die oben angegebene Bedeutung hat,
gegebenenfalls in Gegenwart eines Verdünnungsmittels umsetzt oder
(d) substituierte Acrylsäureester der Formel (Id),

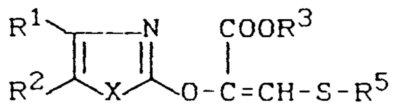 (Id)

in welcher
$R^1$, $R^2$, $R^3$, $R^5$ und X die oben angegebene Bedeutung haben, erhält,
indem man substituierte Acrylsäureester der Formel (VIII),

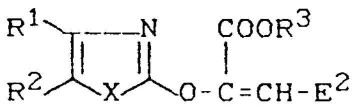 (VIII)

in welcher
$E^2$ für eine elektronenanziehende Abgangsgruppe steht und
$R^1$, $R^2$, $R^3$ und X die oben angegebene Bedeutung haben,
mit Thiolen der Formel (IX),

$R^5$-SH (IX)

in welcher
$R^5$ die oben angegebene Bedeutung hat,
gegebenenfalls in Gegenwart eines Verdünnungsmittels und gegebenenfalls in Gegenwart eines Reaktionshilfsmittels umsetzt.

**2.** Verfahren gemäß Anspruch 1, worin
$R^1$ für Wasserstoff, Methyl, Ethyl, n- oder i-Propyl,n-, i-, s- oder t-Butyl, für Allyl, n- oder i-Butenyl, für jeweils gegebenenfalls im Arylteil oder im Heteroarylteil ein- bis vierfach, gleich oder verschieden substituiertes Benzyl, Phenylethyl, Phenylethenyl, Phenyl, Naphthyl, Pyridyl, Thienyl oder Furyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy oder Benzyloxy,
$R^2$ für Chlor, Brom, Iod, Cyano, Nitro, Formyl, Chlormethyl, Brommethyl, Iodmethyl, Methoxymethyl, Ethoxymethyl, n- oder i-Propoxymethyl, Methylthiomethyl, Ethylthiomethyl, n- oder i-Propylthiomethyl, für Hydroximinomethyl, Hydroximinoethyl, Hydroximinopropyl, für Methoximinomethyl, Ethoximinomethyl, n- oder i-Propoximinomethyl, Methoximinoethyl, Ethoximinoethyl, n- oder i-Propoximinoethyl, für Methyliminomethyl, Ethyliminomethyl, n-oder i-Propyliminomethyl, für Methyliminoethyl, Ethyliminoethyl, n- oder i-Propyliminoethyl, für N,N-Dimethylhydrazonomethyl, N,N-Diethylhydrazonomethyl, N-Methyl-N-ethylhydrazonomethyl, N-Methyl-N-propylhydrazonomethyl, N,N-Dipropylhydrazonomethyl, N,N-Dimethylhydrazonoethyl, N,N-Diethylhydrazonoethyl, für Methoxy, Ethoxy, n- oder i-Propoxy, Methylthio, Ethylthio, n- oder i-Propylthio, für Trifluormethoxy, Difluormethoxy, Dichlorfluormethoxy, Difluorchlormethoxy, Trichlormethoxy, Trifluormethylthio, Trichlormethylthio, Difluormethylthio, Dichlorfluormethylthio, Difluorchlormethylthio, für Acetyl, Propionyl, Butyryl, Methoxycarbonyl, Ethoxycarbonyl, n-oder i-Propoxycarbonyl, N,N-Dimethylaminocarbonyl, N,N-Diethylaminocarbonyl, N,N-Dipropylaminocarbonyl, N-Methyl-N-ethylaminocarbonyl, N-Methyl-N-propyl-aminocarbonyl, für 1-Piperidinylcarbonyl, 4-Morpholinylcarbonyl oder für jeweils gegebenenfalls im Phenylteil ein- bis dreifach, gleich oder verschieden substituiertes N-Phenyliminomethyl, N-Phenyliminoethyl, Phenoxy oder Phenylthio steht,

wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/oder Ethyl substituiertes Phenyl,

$R^3$ für Methyl, Ethyl, n- oder i-Propyl, n-, i-, s-oder t-Butyl oder für gegebenenfalls einbis dreifach, gleich oder verschieden substituiertes Benzyl steht, wobei als Substituenten die bei $R^1$ genannten infrage kommen,

$R^4$ für Dialkylamino mit jeweils 1 bis 4 Kohlenstoffatomen in den einzelnen geradkettigen oder verzweigten Alkylteilen steht oder für einen Rest -Z-$R^5$ steht,

X für Sauerstoff oder Schwefel steht und

Y für Sauerstoff, Schwefel oder für einen Rest

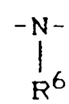

steht,

wobei

$R^5$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^1$ genannten infrage kommen;

$R^6$ für Wasserstoff, Methyl, Ethyl, n- oder i-Propyl, n-, i-, s- oder t-Butyl, für Acetyl, Propionyl, n- oder i-Butyryl oder für jeweils gegebenenfalls ein- bis dreifach, gleich oder verschieden substituiertes Benzyl oder Phenyl steht, wobei als Substituenten die bei $R^1$ genannten infrage kommen und

Z für Sauerstoff oder Schwefel steht.

**3.** Verfahren gemäß Anspruch 1, worin

$R^1$ für Wasserstoff, Methyl, Ethyl, n- oder i-Propyl oder für jeweils gegebenenfalls ein- bis vierfach, gleich oder verschieden substituiertes Phenyl oder Naphthyl steht,

wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls ein- oder zweifach, gleich oder verschieden durch Fluor, Chlor, Brom, Methyl und/oder Ethyl substituiertes Phenyl, Phenoxy, Benzyl oder Benzyloxy,

$R^2$ für Chlor, Brom, Cyano, Nitro, Formyl, Methoxymethyl, Ethoxymethyl, Methylthiomethyl, Hydroximinomethyl, Methoximinomethyl, Ethoximinomethyl, Methyliminomethyl, Ethyliminomethyl, N,N-Dimethylhydrazonomethyl, N,N-Diethylhydrazonomethyl, für Methoxy, Ethoxy, Methylthio, Ethylthio, Trifluormethylthio, Dichlorfluormethylthio, Difluorchlormethylthio, Trichlormethylthio, für Acetyl, Methoxycarbonyl, Ethoxycarbonyl, N,N-Dimethylaminocarbonyl, N,N-Diethylaminocarbonyl, N-Methyl-N-ethylaminocarbonyl oder für gegebenenfalls einfach oder zweifach, gleich oder verschieden substituiertes Phenylthio steht,

wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio,

$R^3$ für Methyl, Ethyl oder Benzyl steht,

$R^4$ für Dimethylamino, Diethylamino oder für einen Rest -Z-$R^5$ steht,

X für Sauerstoff oder Schwefel steht und

Y für Sauerstoff, Schwefel oder für einen Rest

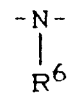

steht,

wobei

$R^5$ für Methyl, Ethyl, n- oder i-Propyl oder Benzyl steht,

$R^6$ für Wasserstoff, Methyl, Ethyl, Acetyl, Propionyl oder für jeweils gegebenenfalls einfach oder zweifach, gleich oder verschieden durch Fluor, Chlor, Brom, Methyl, Ethyl und/oder Trifluormethyl substituiertes Benzyl oder Phenyl steht und

Z für Sauerstoff oder Schwefel steht.

**4.** Verfahren gemäß Anspruch 1, worin

$R^1$ für Wasserstoff, Methyl, Ethyl, n- oder i-Propyl oder für gegebenenfalls einfach oder zweifach, gleich oder verschieden substituiertes Phenyl steht, wobei als Substituenten infrage kommen:
Fluor, Chlor, Brom, Cyano, Nitro, Methyl, Ethyl, Methoxy, Ethoxy, Methylthio, Trifluormethyl, Trifluormethoxy, Trifluormethylthio, Methoxycarbonyl, Ethoxycarbonyl, Methoximinomethyl, Cyclopentyl, 1,3-Propandiyl, Methoximinoethyl oder gegebenenfalls ein- oder zweifach, gleich oder verschieden durch Fluor, Chlor, Brom oder Methyl substituiertes Phenyl, Phenoxy, Benzyl oder Benzyloxy,

$R^2$ für Chlor, Brom, Cyano, Nitro, Formyl, Methoxymethyl, Methylthiomethyl, Methoxycarbonyl, Ethoxycarbonyl, Methylthio, Trifluormethylthio oder Methoximinomethyl steht,

$R^3$ für Methyl oder Ethyl steht,

$R^4$ für Methoxy, Ethoxy, Methylthio oder Dimethylamino steht,

X für Sauerstoff oder Schwefel steht und

Y für einen N-Methyl-Rest steht.

**5.** Schädlingsbekämpfungsmittel, gekennzeichnet durch einen Gehalt an mindestens einem substituierten Acrylsäureester der Formel (I) nach den Ansprüchen 1 bis 4.

**6.** Verwendung von substituierten Acrylsäureestern der Formel (I) nach den Ansprüchen 1 bis 4 zur Bekämpfung von Schädlingen.

**7.** Verfahren zur Bekämpfung von Schädlingen, dadurch gekennzeichnet, daß man substituierte Acrylsäureester der Formel (I) nach den Ansprüchen 1 bis 4 auf Schädlinge und/oder ihren Lebensraum einwirken läßt.

**8.** Verfahren zur Herstellung von Schädlingsbekämpfungsmitteln, dadurch gekennzeichnet, daß man substituierte Acrylsäureester der Formel (I) nach den Ansprüchen 1 bis 4 mit Streckmitteln und/oder oberflächenaktiven Mitteln vermischt.

**9.** Verfahren zur Herstellung von Hydroxyacrylsäureestern oder deren Alkalimetallsalzen der Formel (II)

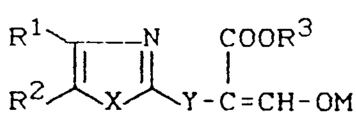

(II)

in welcher

$R^1$, $R^2$, $R^3$, X und Y die in Anspruch 1 angegebene Bedeutung haben und

M für Wasserstoff oder für ein Alkalimetallkation steht und

dadurch gekennzeichnet, daß man substituierte Essigsäureester der Formel (IV),

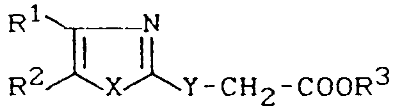

(IV)

in welcher

$R^1$, $R^2$, $R^3$, X und Y die oben angegebene Bedeutung haben,

mit Ameisensäureestern der Formel (X),

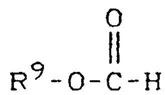

(X)

in welcher

$R^9$ für Alkyl steht,

gegebenenfalls in Gegenwart eines Verdünnungsmittels und gegebenenfalls in Gegenwart eines basischen Reaktionshilfsmittels bei Temperaturen zwischen -20 °C und +50 °C umsetzt.

**10.** Verfahren zur Herstellung von substituierten Essigsäureestern der Formel (IVa)

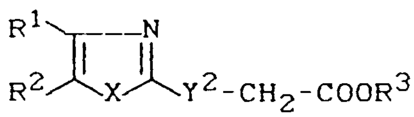

(IVa)

in welcher

$R^1$, $R^2$, $R^3$ und X die in Anspruch 1 angegebene Bedeutung haben und

$Y^2$ für Sauerstoff, Schwefel oder für einen N-Alkylrest steht,

dadurch gekennzeichnet, daß man Thiazol- oder Oxazolderivate der Formel (XIa),

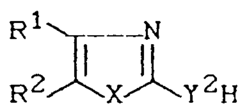

(XIa)

in welcher

$R^1$, $R^2$,X und $Y^2$ die oben angegebene Bedeutung haben,

oder Thiazol- oder Oxazolderivate der Formel (XIb),

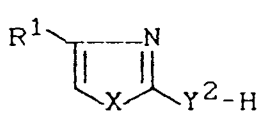

(XIb)

in welcher

$R^1$, X und $Y^2$ die oben angegebene Bedeutung haben,

mit Bromessigestern der Formel (XII),

$Br-CH_2-COOR^3$ (XII)

in welcher
$R^3$ die oben angegebene Bedeutung hat,
gegebenenfalls in Gegenwart eines Verdünnungsmittels und gegebenenfalls in Gegenwart eines Reaktionshilfsmittels bei Temperaturen zwischen - 20 °C und + 150 °C umsetzt und gegebenenfalls anschließend in 5-Position des Thiazol- bzw. Oxazolringes mit üblichen elektrophilen Substitutionsreaktionen substituiert oder bereits vorhandene funktionelle Gruppen in dieser Position mit Hilfe allgemein bekannter Verfahren derivatisiert.

**11.** Verfahren zur Herstellung von substituierten Acrylsäureestern der Formel (VIII)

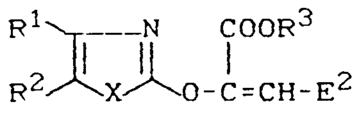

(VIII)

in welcher
$R^1$, $R^2$, $R^3$ und X die in Anspruch 1 angegebene Bedeutung haben und
$E^2$ für eine elektronenanziehende Abgangsgruppe steht,
dadurch gekennzeichnet, daß man Hydroxyacrylsäureester der Formel (IIb),

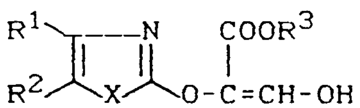

(IIb)

in welcher
$R^1$, $R^2$, $R^3$ und X die oben angegebene Bedeutung haben,
mit Säurechloriden der Formel (XIV),

$R^{10}-Cl$ (XIV)

in welcher
$R^{10}$ für einen Acyl- oder Sulfonylrest steht,
gegebenenfalls in Gegenwart eines Verdünnungsmittels und gegebenenfalls in Gegenwart eines Säurebindemittels bei Temperaturen zwischen -20 °C und +120 °C umsetzt.

**12.** Verwendung von Verbindungen der Formeln (II), (IVa) und (VIII) gemäß den Ansprüchen 9, 10 und 11 als Zwischenprodukte.

## Claims

**Claims for the following Contracting States : BE, CH, LI, DE, DK, FR, GB, IT, NL**

**1.** Substituted acrylic esters of the general formula (I)

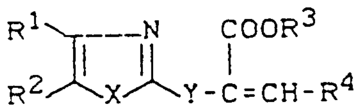

(I)

in which
$R^1$ represents hydrogen, or represents straight-chain or branched alkyl having 1 to 8 carbon atoms, or represents straight-chain or branched alkenyl having 2 to 8 carbon atoms, or

represents aralkyl having 1 to 6 carbon atoms in the straight-chain or branched alkyl moiety, aralkenyl having 2 to 6 carbon atoms in the straight-chain or branched alkenyl moiety or aryl having 6 to 10 carbon atoms in the respective aryl moiety, each of which is optionally monosubstituted to polysubstituted in the aryl moiety by identical or different substituents, suitable aryl substitutents in each case being:

halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 8 carbon atoms in the individual alkyl moieties, cycloalkyl having 3 to 7 carbon atoms, double-linked alkanediyl having 3 to 5 carbon atoms, or aryl, aralkyl, aryloxy or aralkyloxy, each of which has 6 to 10 carbon atoms in the aryl moiety and if appropriate 1 to 4 carbon atoms in the straight-chain or branched alkyl moiety and each of which is optionally monosubstituted to polysubstituted in the aryl moiety by identical or different substituents from the series comprising halogen, alkyl, alkoxy, alkylthio, halogenoalkyl, halogenoalkoxy or halogenoalkylthio, each having 1 to 4 carbon atoms and if appropriate 1 to 9 identical or different halogen atoms, or heteroarylalkyl or heteroaryl, each of which has 2 to 9 carbon atoms and 1 to 4 identical or different hetero atoms in the heteroaryl moiety and if appropriate 1 to 4 carbon atoms in the straight-chain or branched alkyl moiety and each of which is optionally monosubstituted to polysubstituted in the heteroaryl moiety by identical or different substituents from the series comprising halogen, alkyl, alkoxy, alkylthio, halogenoalkyl, halogenoalkoxy or halogenoalkylthio, each having 1 to 4 carbon atoms and if appropriate 1 to 9 identical or different halogen atoms;

$R^1$ furthermore represents a heteroaryl radical which has 2 to 9 carbon atoms and 1 to 4 identical or different hetero atoms and which is optionally monosubstituted to polysubstituted by identical or different substituents, possible substituents being the abovementioned aryl substituents,

$R^2$ represents fluorine, chlorine, bromine, iodine, cyano, nitro or formyl, or represents straight-chain or branched halogenoalkyl having 1 to 4 carbon atoms and 1 to 9 identical or different halogen atoms, or represents in each case straight-chain or branched alkoxyalkyl or alkylthioalkyl, each of which has 1 to 4 carbon atoms in the individual alkyl moieties, or represents in each case straight-chain or branched hydroximinoalkyl, alkoximinoalkyl, N-alkyliminoalkyl or N,N-dialkylhydrazonoalkyl, each of which has 1 to 4 carbon atoms in the individual alkyl moieties, or represents in each case straight-chain or branched alkoxy or alkylthio, each of which has 1 to 4 carbon atoms, or represents in each case straight-chain or branched halogenoalkoxy or halogenoalkylthio, each of which has 1 to 4 carbon atoms and each of which has 1 to 9 identical or different halogen atoms, or represents in each case straight-chain or branched alkanoyl, alkoxycarbonyl or N,N-dialkylcarbamoyl, each of which has 1 to 4 carbon atoms in the individual alkyl moieties, or represents heterocyclylcarbonyl, a possible heterocyclyl radical being a saturated five- to seven-membered N-linked heterocyclic ring which can optionally contain a further hetero atom and which can optionally be monosubstituted to tetrasubstituted by methyl and/or ethyl;

$R^2$ furthermore represents N-aryliminoalkyl, aryloxy or arylthio, each of which is optionally monosubstituted to polysubstituted in the aryl moiety by identical or different substituents, each of which has 6 to 10 carbon atoms, the straight-chain or branched alkyl moiety having 1 to 4 carbon atoms and possible aryl substituents in each case being: halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, or phenyl which is optionally monosubstituted to polysubstituted by identical or different substituents from the series comprising halogen and/or straight-chain or branched alkyl having 1 to 4 carbon atoms,

$R^3$ represents straight-chain or branched alkyl having 1 to 6 carbon atoms, or represents aralkyl which has 1 to 4 carbon atoms in the straight-chain or branched alkyl moiety and 6 to 10 carbon atoms in the aryl moiety and which is optionally monosubstituted to polysubstituted in the aryl moiety by identical or different substituents, possible aryl substituents being those mentioned in the case of $R^1$,

$R^4$ represents dialkylamino having in each case 1 to 6 carbon atoms in the individual straight-chain or branched alkyl moieties, or represents a radical -Z-$R^5$,

X represents oxygen or sulphur and

Y represents oxygen or sulphur or represents a radical

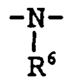

where

$R^5$ represents straight-chain or branched alkyl having 1 to 6 carbon atoms, or represents aralkyl which has 1 to 4 carbon atoms in the straight-chain or branched alkyl moiety and 6 to 10 carbon atoms in the aryl moiety and which is optionally monosubstituted to polysubstituted in the aryl moiety by identical or different substituents, possible aryl substituents being those mentioned in the case of $R^1$,

$R^6$ represents hydrogen, or represents straight-chain or branched alkyl having 1 to 6 carbon atoms, or represents straight-chain or branched alkanoyl having 1 to 6 carbon atoms in the alkyl moiety, or represents aralkyl which has 1 to 6 carbon atoms in the straight-chain or branched alkyl moiety or aryl having in each case 6 to 10 carbon atoms in the respective aryl moiety, each of these aralkyl or aryl moieties being optionally monosubstituted to polysubstituted in the aryl moiety by identical or different substituents, possible substituents in the aryl moiety in each case being those mentioned in the case of $R^1$, and

Z represents oxygen or sulphur, their geometric isomers and their mixtures.

**2.** Substituted acrylic esters of the formula (I) according to Claim 1, where

$R^1$ represents hydrogen, methyl, ethyl, n- or i-propyl or n-, i-, s- or t-butyl, or represents allyl or n-or i-butenyl, or represents benzyl, phenylethyl, phenylethenyl, phenyl, naphthyl, pyridyl, thienyl or furyl, each of which is optionally monosubstituted to tetrasubstituted in the aryl moiety or in the heteroaryl moiety by identical or different substituents, possible substituents in each case being:

fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl or 1,4-butanediyl, or phenyl, benzyl, phenoxy or benzyloxy, each of which is optionally monosubstituted to trisubstituted in the phenyl moiety by identical or different substituents from the series comprising fluorine, chlorine, bromine, methyl, ethyl, methoxy, ethoxy, methylthio, trifluoromethyl, difluoromethoxy, trifluoromethoxy and/or trifluoromethylthio,

$R^2$ represents chlorine, bromine, iodine, cyano, nitro, formyl, chloromethyl, bromomethyl, iodomethyl, methoxymethyl, ethoxymethyl, n- or i-propoxymethyl, methylthiomethyl, ethylthiomethyl or n- or i-propylthiomethyl, or represents hydroximinomethyl, hydroximinoethyl or hydroximinopropyl, or represents methoximinomethyl, ethoximinomethyl, n- or i-propoximinomethyl, methoximinoethyl, ethoximinoethyl or n- or i-propoximinoethyl, or represents methyliminomethyl, ethyliminomethyl or n- or i-propyliminomethyl, or represents methyliminoethyl, ethyliminoethyl or n- or i-propyliminoethyl, or represents N,N-dimethylhydrazonomethyl, N,N-diethylhydrazonomethyl, N-methyl-N-ethylhydrazonomethyl, N-methyl-N-propylhydrazonomethyl, N,N-dipropylhydrazonomethyl, N,N-dimethylhydrazonoethyl or N,N-diethylhydrazonoethyl, or represents methoxy, ethoxy, n- or i-propoxy, methylthio, ethylthio or n- or i-propylthio, or represents trifluoromethoxy, difluoromethoxy, dichlorofluoromethoxy, difluorochloromethoxy, trichloromethoxy, trifluoromethylthio, trichloromethylthio, difluoromethylthio, dichlorofluoromethylthio or difluorochloromethylthio or represents acetyl, propionyl, butyryl, methoxycarbonyl, ethoxycarbonyl, n- or i-propoxycarbonyl, N,N-dimethylaminocarbonyl, N,N-diethylaminocarbonyl, N,N-dipropylaminocarbonyl, N-methyl-N-ethylaminocarbonyl or N-methyl-N-propylaminocarbonyl, or represents 1-piperidinylcarbonyl or 4-morpholinylcarbonyl, or represents N-phenyliminomethyl, N-phenyliminoethyl, phenoxy or phenylthio, each of which is optionally monosubstituted to trisubstituted in the

phenyl moiety by identical or different substituents, possible substituents in each case being: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, trifluoromethylthio, methoxycarbonyl, ethoxycarbonyl, methoximinomethyl, ethoximinomethyl, methoximinoethyl or ethoximinoethyl, or phenyl which is optionally monosubstituted to trisubstituted by identical or different substituents from the series comprising fluorine, chlorine, bromine, methyl and/or ethyl,

$R^3$ represents methyl, ethyl, n- or i-propyl or n-, i-, s- or t-butyl, or represents benzyl which is optionally monosubstituted to trisubstituted by identical or different substituents, possible substituents being those mentioned in the case of $R^1$,

$R^4$ represents dialkylamino having in each case 1 to 4 carbon atoms in the individual straight-chain or branched alkyl moieties, or represents a radical $-Z-R^5$,

X represents oxygen or sulphur and

Y represents oxygen or sulphur, or represents a radical

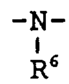

where

$R^5$ represents methyl, ethyl, n- or i-propyl or n-, i-, s- or t-butyl, or represents benzyl which is optionally monosubstituted to trisubstituted by identical or different substituents, possible substituents being those mentioned in the case of $R^1$;

$R^6$ represents hydrogen, methyl, ethyl, n- or i-propyl or n-, i-, s- or t-butyl, or represents acetyl, propionyl or n- or i-butyryl, or represents benzyl or phenyl, each of which is optionally monosubstituted to trisubstituted by identical or different substituents, possible substituents being those mentioned in the case of $R^1$, and

Z represents oxygen or sulphur.

**3.** Substituted acrylic esters of the formula (I) according to Claim 1, where

$R^1$ represents hydrogen, methyl, ethyl, or n- or i-propyl, or represents phenyl or naphthyl, each of which is optionally monosubstituted to tetrasubstituted by identical or different substituents, possible substituents in each case being: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl or 1,4-butanediyl, or phenyl, phenoxy, benzyl or benzyloxy, each of which is optionally monosubstituted or disubstituted by identical or different substituents from the series comprising fluorine, chlorine, bromine, methyl and/or ethyl,

$R^2$ represents chlorine, bromine, cyano, nitro, formyl, methoxymethyl, ethoxymethyl, methylthiomethyl, hydroximinomethyl, methoximinomethyl, ethoximinomethyl, methyliminomethyl, ethyliminomethyl, N,N-dimethylhydrazonomethyl or N,N-diethylthydrazonomethyl, or represents methoxy, ethoxy, methylthio, ethylthio, trifluoromethylthio, dichlorofluoromethylthio, difluorochloromethylthio or trichloromethylthio, or represents acetyl, methoxycarbonyl, ethoxycarbonyl, N,N-dimethylaminocarbonyl, N,N-diethylaminocarbonyl or N-methyl-N-ethylaminocarbonyl, or represents phenylthio which is optionally monosubstituted or disubstituted by identical or different substituents, possible substituents being: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy or trifluoromethylthio,

$R^3$ represents methyl, ethyl or benzyl,

$R^4$ represents dimethylamino or diethylamino, or represents a radical $-Z-R^5$,

X represents oxygen or sulphur and

Y represents oxygen or sulphur, or represents a radical

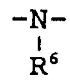

where

$R^5$ represents methyl, ethyl, n- or i-propyl or benzyl,

$R^6$ represents hydrogen, methyl, ethyl, acetyl or propionyl, or represents benzyl or phenyl, each of which is optionally monosubstituted or disubstituted by identical or different substituents from the series comprising fluorine, chlorine, bromine, methyl, ethyl and/or trifluoromethyl, and

Z represents oxygen or sulphur.

**4.** Substituted acrylic esters of the formula (I) according to Claim 1, where

$R^1$ represents hydrogen, methyl, ethyl or n- or i-propyl, or represents phenyl which is optionally monosubstituted or disubstituted by identical or different substituents, possible substituents being: fluorine, chlorine, bromine, cyano, nitro, methyl, ethyl, methoxy, ethoxy, methylthio, trifluoromethyl, trifluoromethoxy, trifluoromethylthio, methoxycarbonyl, ethoxycarbonyl, methoximinomethyl, cyclopentyl, 1,3-propanediyl, methoximinoethyl or phenyl, phenoxy, benzyl or benzyloxy, each of which is optionally monosubstituted or disubstituted by identical or different substituents from the series comprising fluorine, chlorine, bromine or methyl,

$R^2$ represents chlorine, bromine, cyano, nitro, formyl, methoxymethyl, methylthiomethyl, methoxycarbonyl, ethoxycarbonyl, methylthio, trifluoromethylthio or methoximinomethyl,

$R^3$ represents methyl or ethyl,

$R^4$ represents methoxy, ethoxy, methylthio or dimethylamino,

X represents oxygen or sulphur and

Y represents an N-methyl radical.

**5.** Process for the preparation of substituted acrylic esters of the general formula (I)

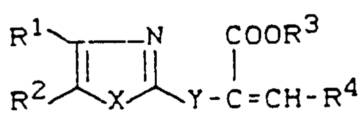

(I)

in which

$R^1$, $R^2$, $R^3$, $R^4$, X and Y have the meaning given in Claim 1, characterized in that

(a) substituted acrylic esters of the formula (Ia)

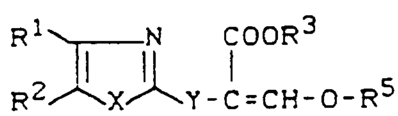

(Ia)

in which

$R^1$, $R^2$, $R^3$, $R^5$, X and Y have the abovementioned meaning,

are obtained by reacting hydroxyacrylic esters or their alkali metal salts of formula (II)

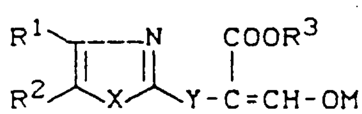

(II)

in which

M represents hydrogen or represents an alkali metal cation and

$R^1$, $R^2$, $R^3$, X and Y have the abovementioned meaning,

with alkylating agents of the formula (III)

$R^5$-$E^1$ (III)

in which
$E^1$ represents an electron-withdrawing leaving group and
$R^5$ has the abovementioned meaning,
if appropriate in the presence of a diluent and if appropriate in the presence of a reaction auxiliary, or
(b) substituted acryic esters of the formula (Ib)

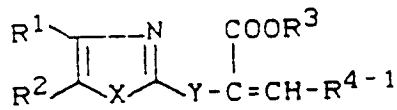

(Ib)

in which
$R^{4-1}$ represents dialkylamino and
$R^1$, $R^2$, $R^3$, X and Y have the abovementioned meaning,
are obtained by reacting substituted acetic esters of the formula (IV)

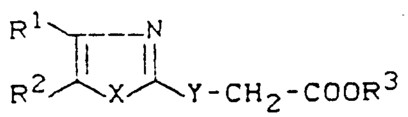

(IV)

in which
$R^1$, $R^2$, $R^3$, X and Y have the abovementioned meaning,
with formamides of the formula (Va)

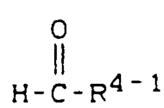

(Va)

in which
$R^{4-1}$ has the abovementioned meaning,
or with their derivatives of the formula (Vb)

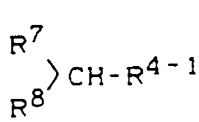

(Vb)

in which
$R^7$ and $R^8$ independently of one another in each case represent alkoxy or dialkylamino and
$R^{4-1}$ has the abovementioned meaning,
if appropriate in the presence of a diluent, or
(c) substituted acrylic esters of the formula (Ic)

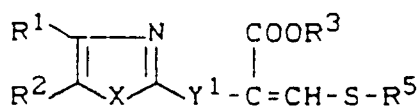

(Ic)

in which

$Y^1$ represents sulphur or represents a radical

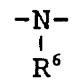

and

$R^1$, $R^2$, $R^3$, $R^5$, $R^6$ and X have the abovementioned meaning,

are obtained by reacting oxalic acid derivatives of the formula (VI)

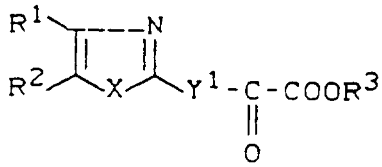

(VI)

in which

$R^1$, $R^2$, $R^3$, X and $Y^1$ have the abovementioned meaning,

with organometal compounds of the formula (VII)

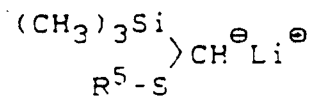

(VII)

in which

$R^5$ has the abovementioned meaning,

if appropriate in the presence of a diluent, or

(d) substituted acrylic esters of the formula (Id)

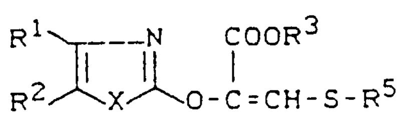

(Id)

in which

$R^1$, $R^2$, $R^3$, $R^5$ and X have the abovementioned meaning,

are obtained by reacting substituted acrylic esters of the formula (VIII)

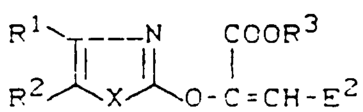

(VIII)

in which

$E^2$ represents an electron-withdrawing leaving group and

$R^1$, $R^2$, $R^3$ and X have the abovementioned meaning,

with thiols of the formula (IX)

$R^5$-SH (IX)

in which
$R^5$ has the abovementioned meaning,
if appropriate in the presence of a diluent and if appropriate in the presence of an reaction auxiliary.

**6.** Pesticides, characterized in that they contain at least one substituted acrylic ester of the formula (I) according to Claim 1 to 4.

**7.** Use of substituted acrylic esters of the formula (I) according to Claims 1 to 4 for combating pests.

**8.** Method of combating pests, characterized in that substituted acrylic esters of the formula (I) according to Claims 1 to 4 are allowed to act on pests and/or their environment.

**9.** Process for the preparation of pesticides, characterized in that substituted acrylic esters of the formula (I) according to Claims 1 to 4 are mixed with extenders and/or surface-active agents.

**10.** Hydroxyacrylic esters or their alkali metal salts of the formula (II)

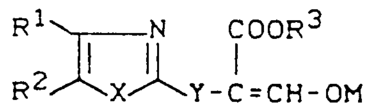

(II)

in which
$R^1$, $R^2$, $R^3$, X and Y have the meaning given in Claim 1 and
M represents hydrogen or represents an alkali metal cation.

**11.** Process for the preparation of hydroxyacrylic esters or their alkali metal salts of the formula (II)

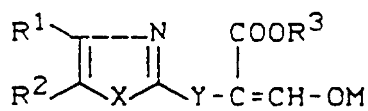

(II)

in which
$R^1$, $R^2$, $R^3$, X and Y have the meaning given in claim 10,
characterized in that substituted acetic esters of the formula (IV)

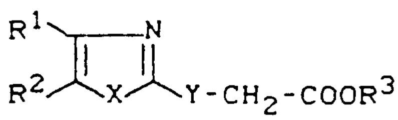

(IV)

in which
$R^1$, $R^2$, $R^3$, X and Y have the abovementioned meaning,
are reacted with formic esters of the formula (X)

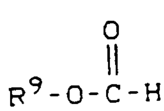

(X)

in which
$R^9$ represents alkyl,
if appropriate in the presence of a diluent and if appropriate in the presence of a basic reaction auxiliary at temperatures between -20 °C and +50 °C.

**12.** Substituted acetic esters of the formula (IVa)

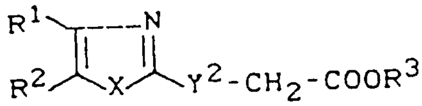

(IVa)

in which

$R^1$, $R^2$, $R^3$ and X have the meaning given in claim 1 and

$Y^2$ represents oxygen or sulphur, or represents an N-alkyl radical,

**13.** Process for the preparation of substituted acetic esters of the formula (IVa)

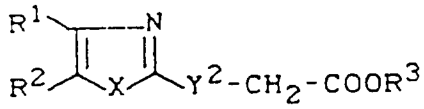

(IVa)

in which

$R^1$, $R^2$, $R^3$, $Y^2$ and X have the meaning given in Claim 12,

characterized in that the thiazole or oxazole derivatives of the formula (XIa)

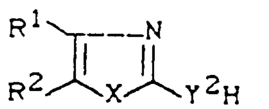

(XIa)

in which

$R^1$, $R^2$, X and $Y^2$ have the abovementioned meaning,

or thiazole or oxazole derivatives of the formula (XIb)

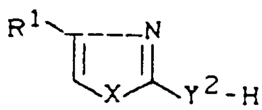

(XIb)

in which

$R^1$, X and $Y^2$ have the abovementioned meaning,

are reacted with bromoacetic esters of the formula (XII)

$Br-CH_2-COOR^3$ (XII)

in which

$R^3$ has the abovementioned meaning,

if appropriate in the presence of a diluent and if appropriate in the presence of a reaction auxiliary at temperatures between -20 °C and +150 °C, and, if appropriate, the products are subsequently substituted in the 5-position of the thiazole or oxazole ring using customary electrophilic substitution reactions, or derivatives are formed of the functional groups which are already present in this position with the aid of generally known processes.

**14.** Substituted acrylic esters of the formula (VIII)

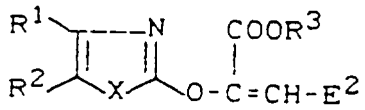

(VIII)

in which

$R^1$, $R^2$, $R^3$ and X have the meaning given in Claim 1, and

$E^2$ represents an electron-withdrawing leaving group.

**15.** Process for the preparation of substituted acrylic esters of the formula (VIII)

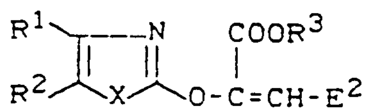

(VIII)

in which

$R^1$, $R^2$, $R^3$, X and $E^2$ have the meaning given in Claim 14,

characterized in that hydroxyacrylic esters of the formula (IIb)

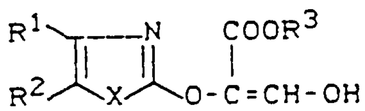

(IIb)

in which

$R^1$, $R^2$, $R^3$ and X have the abovementioned meaning,

are reacted with acid chlorides of the formula (XIV)

$R^{10}$-Cl (XIV)

in which

$R^{10}$ represents an acyl or sulphonyl radical,

if appropriate in the presence of a diluent and if appropriate in the presence of an acid-binding agent at temperatures between -20 °C and +120 °C.

**16.** Use of compounds of the formulae (II), (IVa) and (VIII) according to Claims 10, 12 and 14 as intermediates.

**Claims for the following Contracting State : ES**

**1.** Process for the preparation of substituted acrylic esters of the general formula (I)

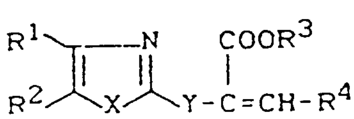

(I)

in which

$R^1$ represents hydrogen, or represents straight-chain or branched alkyl having 1 to 8 carbon atoms, or represents straight-chain or branched alkenyl having 2 to 8 carbon atoms, or represents aralkyl having 1 to 6 carbon atoms in the straight-chain or branched alkyl moiety,

aralkenyl having 2 to 6 carbon atoms in the straight-chain or branched alkenyl moiety or aryl having 6 to 10 carbon atoms in the respective aryl moiety, each of which is optionally monosubstituted to polysubstituted in the aryl moiety by identical or different substituents, suitable aryl substitutents in each case being:
halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 8 carbon atoms in the individual alkyl moieties, cycloalkyl having 3 to 7 carbon atoms, double-linked alkanediyl having 3 to 5 carbon atoms, or aryl, aralkyl, aryloxy or aralkyloxy, each of which has 6 to 10 carbon atoms in the aryl moiety and if appropriate 1 to 4 carbon atoms in the straight-chain or branched alkyl moiety and each of which is optionally monosubstituted to polysubstituted in the aryl moiety by identical or different substituents from the series comprising halogen, alkyl, alkoxy, alkylthio, halogenoalkyl, halogenoalkoxy or halogenoalkylthio, each having 1 to 4 carbon atoms and if appropriate 1 to 9 identical or different halogen atoms, or heteroarylalkyl or heteroaryl, each of which has 2 to 9 carbon atoms and 1 to 4 identical or different hetero atoms in the heteroaryl moiety and if appropriate 1 to 4 carbon atoms in the straight-chain or branched alkyl moiety and each of which is optionally monosubstituted to polysubstituted in the heteroaryl moiety by identical or different substituents from the series comprising halogen, alkyl, alkoxy, alkylthio, halogenoalkyl, halogenoalkoxy or halogenoalkylthio, each having 1 to 4 carbon atoms and if appropriate 1 to 9 identical or different halogen atoms;

$R^1$ furthermore represents a heteroaryl radical which has 2 to 9 carbon atoms and 1 to 4 identical or different hetero atoms and which is optionally monosubstituted to polysubstituted by identical or different substituents, possible substituents being the abovementioned aryl substituents,

$R^2$ represents fluorine, chlorine, bromine, iodine, cyano, nitro or formyl, or represents straight-chain or branched halogenoalkyl having 1 to 4 carbon atoms and 1 to 9 identical or different halogen atoms, or represents in each case straight-chain or branched alkoxyalkyl or alkylthioalkyl, each of which has 1 to 4 carbon atoms in the individual alkyl moieties, or represents in each case straight-chain or branched hydroximinoalkyl, alkoximinoalkyl, N-alkyliminoalkyl or N,N-dialkylhydrazonoalkyl, each of which has 1 to 4 carbon atoms in the individual alkyl moieties, or represents in each case straight-chain or branched alkoxy or alkylthio, each of which has 1 to 4 carbon atoms, or represents in each case straight-chain or branched halogenoalkoxy or halogenoalkylthio, each of which has 1 to 4 carbon atoms and each of which has 1 to 9 identical or different halogen atoms, or represents in each case straight-chain or branched alkanoyl, alkoxycarbonyl or N,N-dialkylcarbamoyl, each of which has 1 to 4 carbon atoms in the individual alkyl moieties, or represents heterocyclylcarbonyl, a possible heterocyclyl radical being a saturated five- to seven-membered N-linked heterocyclic ring which can optionally contain a further hetero atom and which can optionally be monosubstituted to tetrasubstituted by methyl and/or ethyl;

$R^2$ furthermore represents N-aryliminoalkyl, aryloxy or arylthio, each of which is optionally monosubstituted to polysubstituted in the aryl moiety by identical or different substituents, each of which has 6 to 10 carbon atoms, the straight-chain or branched alkyl moiety having 1 to 4 carbon atoms and possible aryl substituents in each case being: halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, or phenyl which is optionally monosubstituted to polysubstituted by identical or different substituents from the series comprising halogen and/or straight-chain or branched alkyl having 1 to 4 carbon atoms,

$R^3$ represents straight-chain or branched alkyl having 1 to 6 carbon atoms, or represents aralkyl which has 1 to 4 carbon atoms in the straight-chain or branched alkyl moiety and 6 to 10 carbon atoms in the aryl moiety and which is optionally monosubstituted to polysubstituted in the aryl moiety by identical or different substituents, possible aryl substituents being those mentioned in the case of $R^1$,

$R^4$ represents dialkylamino having in each case 1 to 6 carbon atoms in the individual straight-chain or branched alkyl moieties, or represents a radical -Z-$R^5$,

X represents oxygen or sulphur and

Y represents oxygen or sulphur or represents a radical

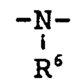

where

$R^5$ represents straight-chain or branched alkyl having 1 to 6 carbon atoms, or represents aralkyl which has 1 to 4 carbon atoms in the straight-chain or branched alkyl moiety and 6 to 10 carbon atoms in the aryl moiety and which is optionally monosubstituted to polysubstituted in the aryl moiety by identical or different substituents, possible aryl substituents being those mentioned in the case of $R^1$,

$R^6$ represents hydrogen, or represents straight-chain or branched alkyl having 1 to 6 carbon atoms, or represents straight-chain or branched alkanoyl having 1 to 6 carbon atoms in the alkyl moiety, or represents aralkyl which has 1 to 6 carbon atoms in the straight-chain or branched alkyl moiety or aryl having in each case 6 to 10 carbon atoms in the respective aryl moiety, each of these aralkyl or aryl moieties being optionally monosubstituted to polysubstituted in the aryl moiety by identical or different substituents, possible substituents in the aryl moiety in each case being those mentioned in the case of $R^1$, and

Z represents oxygen or sulphurm, their geometric isomers and their mixtures, characterized in that

(a) substituted acrylic esters of the formula (Ia)

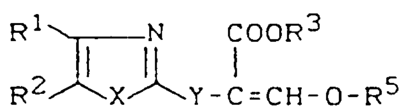

(Ia)

in which

$R^1$, $R^2$, $R^3$, $R^5$, X and Y have the abovementioned meaning,

are obtained by reacting hydroxyacrylic esters or their alkali metal salts of formula (II)

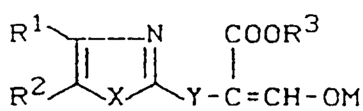

(II)

in which

M represents hydrogen or represents an alkali metal cation and

$R^1$, $R^2$, $R^3$, X and Y have the abovementioned meaning,

with alkylating agents of the formula (III)

$R^5$-$E^1$ (III)

in which

$E^1$ represents an electron-withdrawing leaving group and

$R^5$ has the abovementioned meaning,

if appropriate in the presence of a diluent and if appropriate in the presence of a reaction auxiliary, or

(b) substituted acryic esters of the formula (Ib)

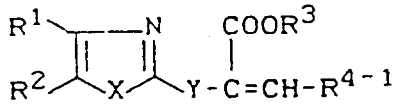

(Ib)

in which

$R^{4-1}$ represents dialkylamino and

$R^1$, $R^2$, $R^3$, X and Y have the abovementioned meaning,

are obtained by reacting substituted acetic esters of the formula (IV)

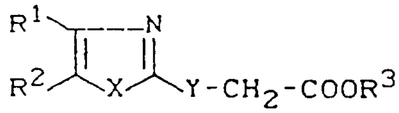

(IV)

in which

$R^1$, $R^2$, $R^3$, X and Y have the abovementioned meaning,

with formamides of the formula (Va)

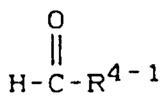

(Va)

in which

$R^{4-1}$ has the abovementioned meaning,

or with their derivatives of the formula (Vb)

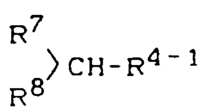

(Vb)

in which

$R^7$ and $R^8$ independently of one another in each case represent alkoxy or dialkylamino and

$R^{4-1}$ has the abovementioned meaning,

if appropriate in the presence of a diluent, or

(c) substituted acrylic esters of the formula (Ic)

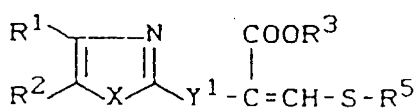

(Ic)

in which

$Y^1$ represents sulphur or represents a radical

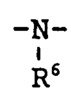

and

$R^1$, $R^2$, $R^3$, $R^5$, $R^6$ and X have the abovementioned meaning,

are obtained by reacting oxalic acid derivatives of the formula (VI)

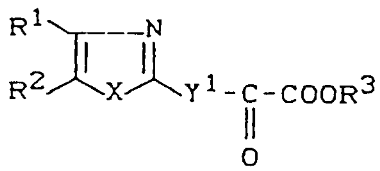

(VI)

in which

$R^1$, $R^2$, $R^3$, X and $Y^1$ have the abovementioned meaning,

with organometal compounds of the formula (VII)

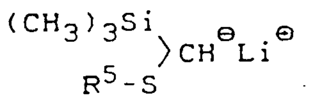

(VII)

in which

$R^5$ has the abovementioned meaning,

if appropriate in the presence of a diluent, or

(d) substituted acrylic esters of the formula (Id)

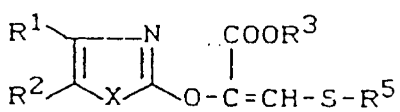

(Id)

in which

$R^1$, $R^2$, $R^3$, $R^5$ and X have the abovementioned meaning,

are obtained by reacting substituted acrylic esters of the formula (VIII)

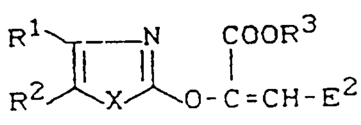

(VIII)

in which

$E^2$ represents an electron-withdrawing leaving group and

$R^1$, $R^2$, $R^3$ and X have the abovementioned meaning,

with thiols of the formula (IX)

$R^5$-SH (IX)

in which

$R^5$ has the abovementioned meaning,

if appropriate in the presence of a diluent and if appropriate in the presence of an reaction auxiliary.

**2.** Process according to Claim 1, where

$R^1$ represents hydrogen, methyl, ethyl, n- or i-propyl or n-, i-, s- or t-butyl, or represents allyl or n-or i-butenyl, or represents benzyl, phenylethyl, phenylethenyl, phenyl, naphthyl, pyridyl, thienyl or furyl, each of which is optionally monosubstituted to tetrasubstituted in the aryl

moiety or in the heteroaryl moiety by identical or different substituents, possible substituents in each case being:

fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl or 1,4-butanediyl, or phenyl, benzyl, phenoxy or benzyloxy, each of which is optionally monosubstituted to trisubstituted in the phenyl moiety by identical or different substituents from the series comprising fluorine, chlorine, bromine, methyl, ethyl, methoxy, ethoxy, methylthio, trifluoromethyl, difluoromethoxy, trifluoromethoxy and/or trifluoromethylthio,

$R^2$ represents chlorine, bromine, iodine, cyano, nitro, formyl, chloromethyl, bromomethyl, iodomethyl, methoxymethyl, ethoxymethyl, n- or i-propoxymethyl, methylthiomethyl, ethylthiomethyl or n- or i-propylthiomethyl, or represents hydroximinomethyl, hydroximinoethyl or hydroximinopropyl, or represents methoximinomethyl, ethoximinomethyl, n- or i-propoximinomethyl, methoximinoethyl, ethoximinoethyl or n- or i-propoximinoethyl, or represents methyliminomethyl, ethyliminomethyl or n- or i-propyliminomethyl, or represents methyliminoethyl, ethyliminoethyl or n- or i-propyliminoethyl, or represents N,N-dimethylhydrazonomethyl, N,N-diethylhydrazonomethyl, N-methyl-N-ethylhydrazonomethyl, N-methyl-N-propylhydrazonomethyl, N,N-dipropylhydrazonomethyl, N,N-dimethylhydrazonoethyl or N,N-diethylhydrazonoethyl, or represents methoxy, ethoxy, n- or i-propoxy, methylthio, ethylthio or n-or i-propylthio, or represents trifluoromethoxy, difluoromethoxy, dichlorofluoromethoxy, difluorochloromethoxy, trichloromethoxy, trifluoromethylthio, trichloromethylthio, difluoromethylthio, dichlorofluoromethylthio or difluorochloromethylthio or represents acetyl, propionyl, butyryl, methoxycarbonyl, ethoxycarbonyl, n- or i-propoxycarbonyl, N,N-dimethylaminocarbonyl, N,N-diethylaminocarbonyl, N,N-dipropylaminocarbonyl, N-methyl-N-ethylaminocarbonyl or N-methyl-N-propylaminocarbonyl, or represents 1-piperidinylcarbonyl or 4-morpholinylcarbonyl, or represents N-phenyliminomethyl, N-phenyliminoethyl, phenoxy or phenylthio, each of which is optionally monosubstituted to trisubstituted in the phenyl moiety by identical or different substituents, possible substituents in each case being: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, trifluoromethylthio, methoxycarbonyl, ethoxycarbonyl, methoximinomethyl, ethoximinomethyl, methoximinoethyl or ethoximinoethyl, or phenyl which is optionally monosubstituted to trisubstituted by identical or different substituents from the series comprising fluorine, chlorine, bromine, methyl and/or ethyl,

$R^3$ represents methyl, ethyl, n- or i-propyl or n-, i-, s- or t-butyl, or represents benzyl which is optionally monosubstituted to trisubstituted by identical or different substituents, possible substituents being those mentioned in the case of $R^1$,

$R^4$ represents dialkylamino having in each case 1 to 4 carbon atoms in the individual straight-chain or branched alkyl moieties, or represents a radical $-Z-R^5$,

X represents oxygen or sulphur and

Y represents oxygen or sulphur, or represents a radical

$$-\underset{\displaystyle R^6}{\underset{|}{N}}-$$

where

$R^5$ represents methyl, ethyl, n- or i-propyl or n-, i-, s- or t-butyl, or represents benzyl which is optionally monosubstituted to trisubstituted by identical or different substituents, possible substituents being those mentioned in the case of $R^1$;

$R^6$ represents hydrogen, methyl, ethyl, n- or i-propyl or n-, i-, s- or t-butyl, or represents acetyl, propionyl or n- or i-butyryl, or represents benzyl or phenyl, each of which is optionally monosubstituted to trisubstituted by identical or different substituents, possible substituents being those mentioned in the case of $R^1$, and

Z represents oxygen or sulphur.

**3.** Process according to Claim 1, where

$R^1$ represents hydrogen, methyl, ethyl, or n- or i-propyl, or represents phenyl or naphthyl, each of which is optionally monosubstituted to tetrasubstituted by identical or different substituents, possible substituents in each case being: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl or 1,4-butanediyl, or phenyl, phenoxy, benzyl or benzyloxy, each of which is optionally monosubstituted or disubstituted by identical or different substituents from the series comprising fluorine, chlorine, bromine, methyl and/or ethyl,

$R^2$ represents chlorine, bromine, cyano, nitro, formyl, methoxymethyl, ethoxymethyl, methylthiomethyl, hydroximinomethyl, methoximinomethyl, ethoximinomethyl, methyliminomethyl, ethyliminomethyl, N,N-dimethylhydrazonomethyl or N,N-diethylthydrazonomethyl, or represents methoxy, ethoxy, methylthio, ethylthio, trifluoromethylthio, dichlorofluoromethylthio, difluorochloromethylthio or trichloromethylthio, or represents acetyl, methoxycarbonyl, ethoxycarbonyl, N,N-dimethylaminocarbonyl, N,N-diethylaminocarbonyl or N-methyl-N-ethylaminocarbonyl, or represents phenylthio which is optionally monosubstituted or disubstituted by identical or different substituents, possible substituents being: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy or trifluoromethylthio,

$R^3$ represents methyl, ethyl or benzyl,

$R^4$ represents dimethylamino or diethylamino, or represents a radical -Z-$R^5$,

X represents oxygen or sulphur and

Y represents oxygen or sulphur, or represents a radical

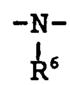

where

$R^5$ represents methyl, ethyl, n- or i-propyl or benzyl,

$R^6$ represents hydrogen, methyl, ethyl, acetyl or propionyl, or represents benzyl or phenyl, each of which is optionally monosubstituted or disubstituted by identical or different substituents from the series comprising fluorine, chlorine, bromine, methyl, ethyl and/or trifluoromethyl, and

Z represents oxygen or sulphur.

**4.** Process according to Claim 1, where

$R^1$ represents hydrogen, methyl, ethyl or n- or i-propyl, or represents phenyl which is optionally monosubstituted or disubstituted by identical or different substituents, possible substituents being: fluorine, chlorine, bromine, cyano, nitro, methyl, ethyl, methoxy, ethoxy, methylthio, trifluoromethyl, trifluoromethoxy, trifluoromethylthio, methoxycarbonyl, ethoxycarbonyl, methoximinomethyl, cyclopentyl, 1,3-propanediyl, methoximinoethyl or phenyl, phenoxy, benzyl or benzyloxy, each of which is optionally monosubstituted or disubstituted by identical or different substituents from the series comprising fluorine, chlorine, bromine or methyl,

$R^2$ represents chlorine, bromine, cyano, nitro, formyl, methoxymethyl, methylthiomethyl, methoxycarbonyl, ethoxycarbonyl, methylthio, trifluoromethylthio or methoximinomethyl,

$R^3$ represents methyl or ethyl,

$R^4$ represents methoxy, ethoxy, methylthio or dimethylamino,

X represents oxygen or sulphur and

Y represents an N-methyl radical.

**5.** Pesticides, characterized in that they contain at least one substituted acrylic ester of the formula (I) according to Claim 1 to 4.

**6.** Use of substituted acrylic esters of the formula (I) according to Claims 1 to 4 for combating pests.

**7.** Method of combating pests, characterized in that substituted acrylic esters of the formula (I) according to Claims 1 to 4 are allowed to act on pests and/or their environment.

**8.** Process for the preparation of pesticides, characterized in that substituted acrylic esters of the formula (I) according to Claims 1 to 4 are mixed with extenders and/or surface-active agents.

**9.** Process for the preparation of hydroxyacrylic esters or their alkali metal salts of the formula (II)

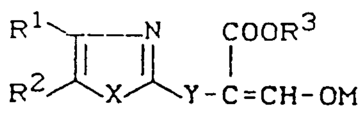

(II)

in which
$R^1$, $R^2$, $R^3$, X and Y have the meaning given in claim 1 and
M represents hydrogen or represents an alkali metal cation
characterized in that substituted acetic esters of the formula (IV)

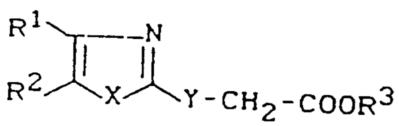

(IV)

in which
$R^1$, $R^2$, $R^3$, X and Y have the abovementioned meaning,
are reacted with formic esters of the formula (X)

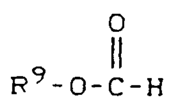

(X)

in which
$R^9$ represents alkyl,
if appropriate in the presence of a diluent and if appropriate in the presence of a basic reaction auxiliary at temperatures between -20 °C and +50 °C.

**10.** Process for the preparation of substituted acetic esters of the formula (IVa)

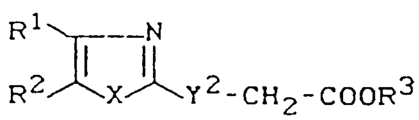

(IVa)

in which
$R^1$, $R^2$, $R^3$, $Y^2$ and X have the meaning given in Claim 1 and
$Y^2$ represents oxygen or sulphur, or represents an N-alkyl radical,
characterized in that the thiazole or oxazole derivatives of the formula (XIa)

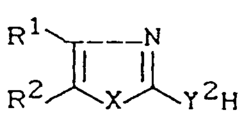

(XIa)

in which

$R^1$, $R^2$, X and $Y^2$ have the abovementioned meaning,

or thiazole or oxazole derivatives of the formula (XIb)

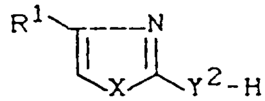

(XIb)

in which

$R^1$, X and $Y^2$ have the abovementioned meaning,

are reacted with bromoacetic esters of the formula (XII)

$Br\text{-}CH_2\text{-}COOR^3$ (XII)

in which

$R^3$ has the abovementioned meaning,

if appropriate in the presence of a diluent and if appropriate in the presence of a reaction auxiliary at temperatures between -20 °C and +150 °C, and, if appropriate, the products are subsequently substituted in the 5-position of the thiazole or oxazole ring using customary electrophilic substitution reactions, or derivatives are formed of the functional groups which are already present in this position with the aid of generally known processes.

**11.** Process for the preparation of substituted acrylic esters of the formula (VIII)

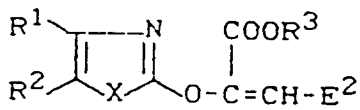

(VIII)

in which

$R^1$, $R^2$, $R^3$ and X have the meaning given in Claim 1 and

$E^2$ represents an electron-withdrawing leaving group,

characterized in that hydroxyacrylic esters of the formula (IIb)

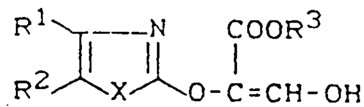

(IIb)

in which

$R^1$, $R^2$, $R^3$ and X have the abovementioned meaning,

are reacted with acid chlorides of the formula (XIV)

$R^{10}\text{-}Cl$ (XIV)

in which

$R^{10}$ represents an acyl or sulphonyl radical,

if appropriate in the presence of a diluent and if appropriate in the presence of an acid-binding agent at temperatures between -20 °C and +120 °C.

**12.** Use of compounds of the formulae (II), (IVa) and (VIII) according to Claims 9, 10 and 11 as intermediates.

**Revendications**

**Revendications pour les Etats contractants suivants : BE, CH, LI, DE, DK, FR, GB, IT, NL**

**1.** Esters d'acides acryliques substitués, de formule générale (I)

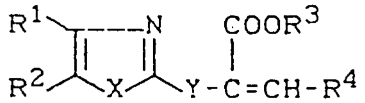

(I)

dans laquelle

$R^1$ représente de l'hydrogène, un groupe alkyle à chaîne droite ou ramifiée ayant 1 à 8 atomes de carbone, un groupe alcényle à chaîne droite ou ramifiée ayant 2 à 8 atomes de carbone, un groupe aralkyle ayant 1 à 6 atomes de carbone dans la partie alkyle à chaîne droite ou ramifiée, un groupe aralcényle ayant 2 à 6 atomes de carbone dans la partie alcényle à chaîne droite ou ramifiée ou un groupe aryle, avec chacun 6 à 10 atomes de carbone dans la partie aryle et portant chacun le cas échéant dans la partie aryle un ou plusieurs substituants identiques ou différents, en considérant dans chaque cas comme substituants de la partie aryle :
un halogène, un radical cyano, nitro, un radical alkyle, alkoxy ou alkylthio ayant chacun 1 à 4 atomes de carbone et étant chacun linéaire ou ramifié, un groupe halogénalkyle, halogénalkoxy ou halogénalkylthio ayant chacun 1 à 4 atomes de carbone et 1 à 9 atomes d'halogènes identiques ou différents et étant chacun linéaire ou ramifié, un groupe alkoxycarbonyle ou alkoximinoalkyle ayant chacun 1 à 8 atomes de carbone dans les parties alkyle individuelles et étant chacun linéaire ou ramifié, un groupe cycloalkyle ayant 3 à 7 atomes de carbone, un groupe alcanediyle à deux liaisons ayant 3 à 5 atomes de carbone, un groupe aryle, un groupe aralkyle, un groupe aryloxy ou un groupe aralkoxy ayant chacun 6 à 10 atomes de carbone dans la partie aryle et le cas échéant 1 à 4 atomes de carbone dans la partie alkyle linéaire ou ramifiée, portant chacun le cas échéant dans la partie aryle un ou plusieurs substituants, identiques ou différents, halogéno, alkyle, alkoxy, alkylthio, halogénalkyle, halogénalkoxy ou halogénalkylthio ayant chacun 1 à 4 atomes de carbone et le cas échéant 1 à 9 atomes d'halogènes identiques ou différents ou un groupe hétéroarylalkyle ou un groupe hétéroaryle ayant chacun 2 à 9 atomes de carbone et 1 à 4 hétéroatomes identiques ou différents dans la partie hétéroaryle et le cas échéant 1 à 4 atomes de carbone dans la partie alkyle à chaîne droite ou ramifiée, portant chacun le cas échéant dans la partie hétéroaryle un ou plusieurs substituants, identiques ou différents, halogéno, alkyle, alkoxy, alkylthio, halogénalkyle, halogénalkoxy ou halogénalkylthio ayant chacun 1 à 4 atomes de carbone et le cas échéant 1 à 9 atomes d'halogènes identiques ou différents ;

$R^1$ représente en outre un reste hétéroaryle de 2 à 9 atomes de carbone et 1 à 4 hétéroatomes identiques ou différents, portant le cas échéant un ou plusieurs substituants identiques ou différents, en considérant comme substituants les substituants de la partie aryle mentionnés ci-dessus,

$R^2$ représente du fluor, du chlore, du brome, de l'iode, un groupe cyano, nitro, formyle, un groupe halogénalkyle à chaîne droite ou ramifié ayant 1 à 4 atomes de carbone et 1 à 9 atomes d'halogènes identiques ou différents, un groupe alkoxyalkyle ou un groupe alkylthioalkyle linéaire ou ramifié portant chacun 1 à 4 atomes de carbone dans les parties alkyle individuelles, un groupe hydroxyminoalkyle, un groupe alkoximinoalkyle, un groupe N-alkyliminoalkyle ou un groupe N,N-dialkylhydrazonoalkyle ayant chacun 1 à 4 atomes de carbone dans les parties alkyle individuelles, chacun étant linéaire ou ramifié, un groupe alkoxy ou alkylthio linéaire ou ramifié ayant chacun 1 à 4 atomes de carbone, un groupe halogénalkoxy ou un groupe halogénalkylthio, linéaire ou ramifié, ayant chacun 1 à 4 atomes de carbone et chacun 1 à 9 atomes d'halogènes identiques ou différents, un groupe alcanoyle, alkoxycarbonyle ou N,N-dialkylcarbamoyle linéaire ou ramifié ayant chacun 1 à 4 atomes de carbone dans les parties alkyle individuelles ou un groupe hétérocyclylcarbonyle, en considérant comme reste hétérocyclyle un hétérocycle saturé pentagonal à heptagonal lié par de l'azote, qui peut contenir éventuellement un autre hétéroatome et qui peut porter le cas échéant 1 à 4 substituants méthyle et/ou éthyle ;

$R^2$ représente en outre un groupe N-aryliminoalkyle, aryloxy ou arylthio portant chacun le cas échéant un ou plusieurs substituants identiques ou différents dans la partie aryle avec chacun 6 à 10 atomes de carbone, la partie alkyle linéaire ou ramifiée ayant 1 à 4 atomes de carbone et en considérant dans chaque cas comme substituants de la partie aryle : un halogène, un radical cyano, nitro, un radical alkyle, alkoxy ou alkylthio linéaire ou ramifié ayant chacun 2 à 4 atomes de carbone, un radical halogénalkyle, halogénalkoxy ou halogénalkylthio linéaire ou ramifié ayant chacun 1 à 4 atomes de carbone et 1 à 9 atomes d'halogènes identiques ou différents, un groupe alkoxycarbonyle ou un groupe alkoximinoalkyle linéaire ou ramifié ayant chacun 1 à 4 atomes de carbone dans les parties alkyle individuelles, un groupe phényle, portant éventuellement un ou plusieurs substituants, identiques ou différents, halogéno et/ou alkyle linéaire ou ramifié ayant 1 à 4 atomes de carbone,

$R^3$ est un groupe alkyle linéaire ou ramifié ayant 1 à 6 atomes de carbone ou un groupe aralkyle ayant 1 à 4 atomes de carbone dans la partie alkyle linéaire ou ramifiée et 6 à 10 atomes de carbone dans la partie aryle, portant le cas échéant dans la partie aryle un ou plusieurs substituants identiques ou différents, en considérant comme substituants de la partie aryle ceux qui ont été mentionnés dans le cas de $R^1$,

$R^4$ est un groupe dialkylamino ayant 1 à 6 atomes de carbone dans les parties alkyle individuelles linéaires ou ramifiées ou représente un reste -Z-$R^5$,

X est de l'oxygène ou du soufre et

Y est de l'oxygène, du soufre ou un reste

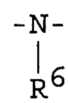

$R^5$ est un groupe alkyle à chaîne droite ou ramifiée ayant 1 à 6 atomes de carbone ou un groupe aralkyle ayant 1 à 4 atomes de carbone dans la partie alkyle linéaire ou ramifiée et 6 à 10 atomes de carbone dans la partie aryle, portant le cas échéant dans la partie aryle un ou plusieurs substituants identiques ou différents, en considérant comme substituants de la partie aryle ceux qui ont été mentionnés dans le cas de $R^1$,

$R^6$ représente de l'hydrogène, un groupe alkyle linéaire ou ramifié ayant 1 à 6 atomes de carbone, un groupe alcanoyle linéaire ou ramifié ayant 1 à 6 atomes de carbone dans la partie alkyle ou un groupe aralkyle portant éventuellement dans la partie aryle un ou plusieurs substituants identiques ou différents et ayant 1 à 6 atomes de carbone dans la partie alkyle linéaire ou ramifiée ou un groupe aryle, avec chacun dans la partie aryle correspondante 6 à 10 atomes de carbone, en considérant comme substituants de la partie aryle chacun des substituants mentionnés dans le cas de $R^1$ et

Z représente de l'oxygène ou du soufre,

leurs isomères géométriques et leurs mélanges.

**2.** Esters d'acides acryliques substitués, de formule (I) suivant la revendication 1, dans laquelle

$R^1$ représente de l'hydrogène, un groupe méthyle, éthyle, n-propyle, isopropyle, n-butyle, isobutyle, sec.-butyle, tertio-butyle, allyle, n-butényle ou isobutényle, un groupe benzyle, phényléthyle, phényléthényle, phényle, naphtyle, pyridyle, thiényle ou furyle portant chacun le cas échéant dans la partie aryle ou dans la partie hétéroaryle 1 à 4 substituants identiques ou différents, en considérant dans chaque cas comme substituants :
le fluor, le chlore, le brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, trifluorométhyle, trifluorométhoxy, difluorométhoxy, trifluorométhylthio, méthoxycarbonyle, éthoxycarbonyle, méthoximinométhyle, éthoximinométhyle, méthoximinoéthyle, éthoximinoéthyle, cyclopentyle, cyclohexyle, 1,3-propanediyle, 1,4-butanediyle ou un groupe phényle, benzyle, phénoxy ou benzyloxy portant chacun le cas échéant dans la partie phényle 1 à 3 substituants, identiques ou différents, fluoro, chloro, bromo, méthyle, éthyle, méthoxy, éthoxy, méthylthio, trifluorométhyle, difluorométhoxy, trifluorométhoxy et/ou trifluorométhylthio,

$R^2$ représente du chlore, du brome, de l'iode, un groupe cyano, nitro, formyle, chlorométhyle, bromométhyle, iodométhyle, méthoxyméthyle, éthoxyméthyle, n-propoxyméthyle, isopropoxyméthyle, méthylthiométhyle, éthylthiométhyle, n-propylthiométhyle, isopropylthiométhyle, hydroximinométhyle, hydroximinoéthyle, hydroximinopropyle, méthoximinométhyle, éthoximinométhyle, n-propoximinométhyle, isopropoximinométhyle, méthoximinoéthyle, éthoximinoéthyle, n-propoximinoéthyle, isopropoximinoéthyle, méthyliminométhyle, éthyliminométhyle, n-propyliminométhyle, isopropyliminométhyle, méthyliminoéthyle, éthyliminoéthyle, n-propyliminoéthyle, isopropyliminoéthyle, N,N-diméthylhydrazonométhyle, N,N-diéthylhydrazonométhyle, N-méthyl-N-éthylhydrazonométhyle, N-méthyl-N-propylhydrazonométhyle, N,N-dipropylhydrazonométhyle, N,N-diméthylhydrazonoéthyle, N,N-diéthylhydrazonoéthyle, méthoxy, éthoxy, n-propoxy, isopropoxy, méthylthio, éthylthio, n-propylthio, isopropylthio, trifluorométhoxy, difluorométhoxy, dichlorofluorométhoxy, difluorochlorométhoxy, trichlorométhoxy, trifluorométhylthio, trichlorométhylthio, difluorométhylthio, dichlorofluorométhylthio, difluorochlorométhylthio, acétyle, propionyle, butyryle, méthoxycarbonyle, éthoxycarbonyle, n-propoxycarbonyle, isopropoxycarbonyle, N,N-diméthylaminocarbonyle, N,N-diéthylaminocarbonyle, N,N-dipropylaminocarbonyle, N-méthyl-N-éthylaminocarbonyle, N-méthyl-N-propylaminocarbonyle, 1-pipéridinylcarbonyle, 4-morpholinylcarbonyle ou un groupe N-phényliminométhyle, N-phényliminoéthyle, phénoxy ou phénylthio portant chacun le cas échéant dans la partie phényle 1 à 3 substituants identiques ou différents, en considérant dans chaque cas comme substituants : le fluor, le chlore, le brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, trifluorométhyle, trifluorométhoxy, trifluorométhylthio, méthoxycarbonyle, éthoxycarbonyle, méthoximinométhyle, éthoximinométhyle, méthoximinoéthyle, éthoximinoéthyle ou un groupe phényle portant le cas échéant 1 à 3 substituants fluoro, chloro, bromo, méthyle et/ou éthyle identiques ou différents,

$R^3$ est un groupe méthyle, éthyle, n-propyle, isopropyle, n-butyle, isobutyle, sec.-butyle ou tertio-butyle ou un groupe benzyle portant le cas échéant 1 à 3 substituants identiques ou différents, en considérant comme substituants ceux qui ont été mentionnés pour $R^1$,

$R^4$ est un groupe dialkylamino ayant 1 à 4 atomes de carbone dans chacune des parties alkyle individuelles linéaires ou ramifiées ou un reste -Z-$R^5$,

X est de l'oxygène ou du soufre et

Y est de l'oxygène, du soufre ou un reste

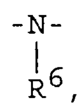

$R^5$ est un groupe méthyle, éthyle, n-propyle, isopropyle, n-butyle, isobutyle, sec.-butyle, tertio-butyle ou un reste benzyle portant le cas échéant 1 à 3 substituants identiques ou différents, en considérant comme substituants ceux qui ont été mentionnés pour $R^1$ ;

$R^6$ est de l'hydrogène, un groupe méthyle, éthyle, n-propyle, isopropyle, n-butyle, isobutyle, sec.-butyle, tertio-butyle, acétyle, propionyle, n-butyryle, isobutyryle ou un reste benzyle ou phényle portant chacun le cas échéant 1 à 3 substituants identiques ou différents, en considérant comme substituants ceux qui ont été mentionnés pour $R^1$ et

Z est de l'oxygène ou du soufre.

**3.** Esters d'acides acryliques substitués de formule (I) suivant la revendication 1, dans laquelle

$R^1$ représente de l'hydrogène, un groupe méthyle, éthyle, n-propyle, isopropyle ou un groupe phényle ou naphtyle portant chacun le cas échéant 1 à 4 substituants identiques ou différents, en considérant dans chaque cas comme subtituants : le fluor, le chlore, le brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, trifluorométhyle, trifluorométhoxy, difluorométhoxy, trifluorométhylthio, méthoxycarbonyle, éthoxycarbonyle, méthoximinométhyle, éthoximinométhyle, méthoximinoéthyle, éthoximinoéthyle, cyclopentyle, cyclohexyle, 1,3-propanediyle, 1,4-butanediyle ou un reste phényle, phénoxy, benzyle ou benzyloxy portant chacun le cas échéant 1 ou 2 substituants, identiques ou différents, fluoro, chloro, bromo, méthyle et/ou éthyle,

$R^3$ représente du chlore, du brome, un radical cyano, nitro, formyle, méthoxyméthyle, éthoxyméthyle, méthylthiométhyle, hydroximinométhyle, méthoximinométhyle, éthoximinométhyle, méthyliminométhyle, éthyliminométhyle, N,N-diméthylhydrazonométhyle, N,N-diéthylhydrazonométhyle, méthoxy, éthoxy, méthylthio, éthylthio, trifluorométhylthio, dichlorofluorométhylthio, difluorochlorométhylthio, trichlorométhylthio, acétyle, méthoxycarbonyle, éthoxycarbonyle, N,N-diméthylaminocarbonyle, N,N-diéthylaminocarbonyle, N-méthyl-N-éthylaminocarbonyle ou un reste phénylthio portant le cas échéant 1 ou 2 substituants identiques ou différents, en considérant comme substituants : le fluor, le chlore, le brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, trifluorométhyle, trifluorométhoxy, trifluorométhylthio,

$R^3$ est un groupe méthyle, éthyle ou benzyle,
$R^4$ est un groupe diméthylamino, diéthylamino ou un reste $-Z-R^5$,
X est de l'oxygène ou du soufre et
Y est de l'oxygène, du soufre ou un reste

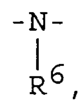

$R^5$ est un groupe méthyle, éthyle, n-propyle, isopropyle ou benzyle,
$R^6$ est de l'hydrogène, un groupe méthyle, éthyle, acétyle, propionyle, ou un groupe benzyle ou phényle portant chacun le cas échéant un ou deux substituants fluoro, chloro, bromo, méthyle, éthyle et/ou trifluorométhyle, identiques ou différents et
Z est de l'oxygène ou du soufre.

**4.** Esters d'acides acryliques substitués de formule (I) suivant la revendication 1, dans laquelle
$R^1$ représente de l'hydrogène, un groupe méthyle, éthyle, n-propyle, isopropyle ou un groupe phényle portant le cas échéant un ou deux substituants identiques ou différents, en considérant comme substituants :
le fluor, le chlore, le brome, un radical cyano, nitro, méthyle, éthyle, méthoxy, éthoxy, méthylthio, trifluorométhyle, trifluorométhoxy, trifluorométhylthio, méthoxycarbonyle, éthoxycarbonyle, méthoximinométhyle, cyclopentyle, 1,3-propanediyle, méthoximinométhyle ou un groupe phényle, phénoxy, benzyle ou benzyloxy portant le cas échéant un ou deux substituants fluoro, chloro, bromo ou méthyle identiques ou différents,
$R^2$ est du chlore, du brome, un groupe cyano ou nitro, formyle, méthoxyméthyle, méthylthiométhyle, méthoxycarbonyle, éthoxycarbonyle, méthylthio, trifluorométhylthio ou méthoximinométhyle,
$R^3$ est un groupe méthyle ou éthyle,
$R^4$ est un groupe méthoxy, éthoxy, méthylthio, ou diméthylamino,
X est de l'oxygène ou du soufre et
Y est un reste N-méthyle.

**5.** Procédé de production d'esters d'acides acryliques substitués de formule générale (I)

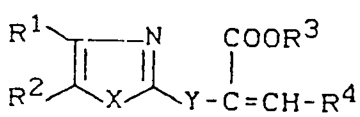

( I )

dans laquelle
$R^1$, $R^2$, $R^3$, $R^4$, X et Y ont la définition indiquée dans la revendication 1, caractérisé en ce que :
(a) on obtient des esters d'acides acryliques substitués de formule (Ia)

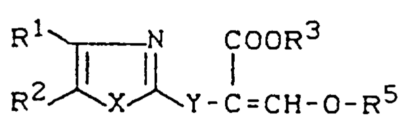

( I a )

dans laquelle

$R^1$, $R^2$, $R^3$, $R^5$, X et Y ont la définition indiquée ci-dessus, en faisant réagir des esters d'acides hydroxyacryliques ou leurs sels de métaux alcalins de formule (II)

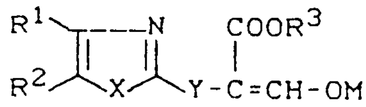

(II)

dans laquelle

M est de l'hydrogène ou un cation de métal alcalin et

$R^1$, $R^2$, $R^3$, X et Y ont la définition indiquée ci-dessus,

avec des agents d'alkylation de formule (III)

$R^5$-$E^1$ (III)

dans laquelle

$E^1$ est un groupe partant attirant les électrons et

$R^5$ a la définition indiquée ci-dessus,

éventuellement en présence d'un diluant et en la présence éventuelle d'une substance auxiliaire de réaction, ou bien

(b) on obtient des esters d'acides acryliques substitués de formule (Ib)

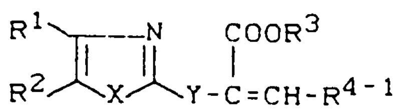

(Ib)

dans laquelle

$R^{4-1}$ est un groupe dialkylamino et

$R^1$, $R^2$, $R^3$, X et Y ont la définition indiquée ci-dessus,

en faisant réagir des esters d'acides acétiques substitués de formule (IV)

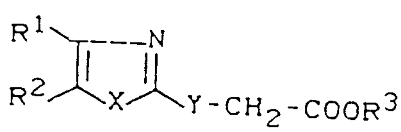

(IV)

dans laquelle

$R^1$, $R^2$, $R^3$, X et Y ont la définition indiquée ci-dessus,

avec des formamides de formule (Va)

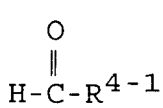

(Va)

dans laquelle

$R^{4-1}$ a la définition indiquée ci-dessus,

ou avec des dérivés de formule (Vb),

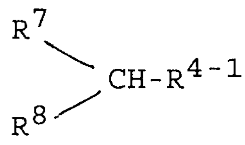

(Vb)

dans laquelle

$R^7$ et $R^8$ représentent chacun, indépendamment l'un de l'autre, un groupe alkoxy ou dialkylamino et

$R^{4-1}$ a la définition indiquée ci-dessus,

le cas échéant en présence d'un diluant, ou bien

(c) on obtient des esters d'acides acryliques substitués de formule (Ic)

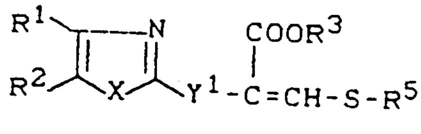

(Ic)

dans laquelle

$Y^1$ représente du soufre ou un reste

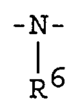

et

$R^1$, $R^2$, $R^3$, $R^5$, $R^6$ et X ont la définition indiquée ci-dessus,

en faisant réagir des dérivés d'acide oxalique de formule (VI)

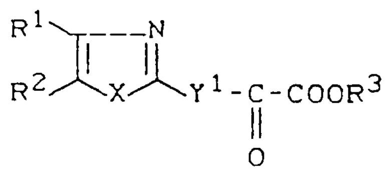

(VI)

dans laquelle

$R^1$, $R^2$, $R^3$, X et $Y^1$ ont la définition indiquée ci-dessus,

avec des composés organométalliques de formule (VII)

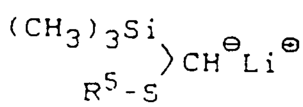

(VII)

dans laquelle

$R^5$ a la définition indiquée ci-dessus,

le cas échéant en présence d'un diluant, ou bien

(d) on obtient des esters d'acides acryliques substitués de formule (Id)

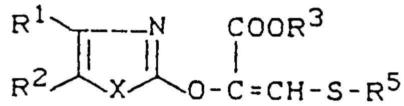

(Id)

dans laquelle

$R^1$, $R^2$, $R^3$, $R^5$ et X  ont la définition indiquée ci-dessus,

en faisant réagir des esters d'acides acryliques substitués de formule (VIII)

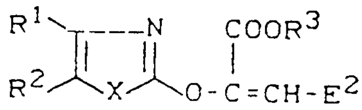

(VIII)

dans laquelle

$E^2$  est un groupe partant attirant les électrons et

$R^1$, $R^2$, $R^3$ et X  ont la définition indiquée ci-dessus,

avec des thiols de formule (IX)

$R^5$-SH  (IX)

dans laquelle

$R^5$  a la définition indiquée ci-dessus,

le cas échéant en présence d'un diluant et en la présence éventuelle d'une substance auxiliaire de réaction;

**6.** Compositions pesticides, caractérisées par une teneur en au moins un ester d'acide acrylique substitué de formule (I) suivant les revendications 1 à 4.

**7.** Utilisation d'esters d'acides acryliques substitués de formule (I) suivant les revendications 1 à 4 pour combattre des parasites.

**8.** Procédé pour combattre des parasites, caractérisé en ce qu'on fait agir sur des parasites et/ou sur leur milieu des esters d'acides acryliques substitués de formule (I) suivant les revendications 1 à 4.

**9.** Procédé de préparation de compositions pesticides, caractérisé en ce qu'on mélange des esters d'acides acryliques substitués de formule (I) suivant les revendications 1 à 4 avec des diluants et/ou des agents tensio-actifs.

**10.** Esters d'acides hydroxyacryliques ou leurs sels de métaux alcalins de formule (II)

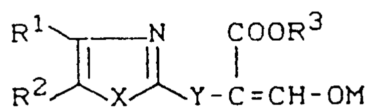

(II)

dans laquelle

$R^1$, $R^2$, $R^3$, X et Y  ont la définition indiquée dans la revendication 1 et

M  est de l'hydrogène ou un cation de métal alcalin.

**11.** Procédé de production d'esters d'acides hydroxyacryliques ou de leurs sels de métaux alcalins de formule (II)

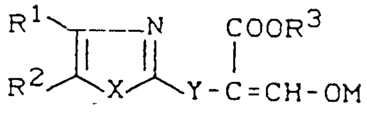

(II)

dans laquelle

$R^1$, $R^2$, $R^3$, X, Y et M  ont la définition indiquée dans la revendication 10.

caractérisé en ce qu'on fait réagir des esters d'acides acétiques substitués de formule (IV)

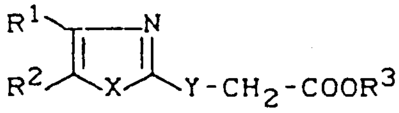

(IV)

dans laquelle

$R^1$, $R^2$, $R^3$, X et Y ont la définition indiquée ci-dessus,

avec des esters d'acide formique de formule (X)

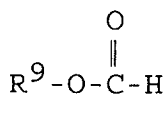

(X)

dans laquelle

$R^9$ est un groupe alkyle,

le cas échéant en présence d'un diluant et en la présence éventuelle d'une substance auxiliaire basique de réaction, à des températures comprises entre -20 °C et +50 °C.

**12.** Esters d'acides acétiques substitués de formule (IVa)

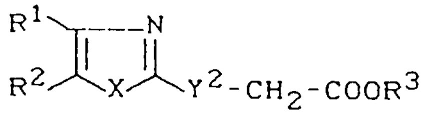

(IVa)

dans laquelle

$R^1$, $R^2$, $R^3$ et X ont la définition indiquée dans la revendication 1 et

$Y^2$ représente de l'oxygène, du soufre ou un reste N-alkyle.

**13.** Procédé de production d'esters d'acides acétiques substitués de formule (IVa)

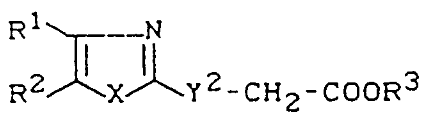

(IVa)

dans laquelle

$R^1$, $R^2$, $R^3$, $Y^2$ et X ont la définition indiquée dans la revendication 12,

caractérisé en ce qu'on fait réagir des dérivés de thiazole ou d'oxazole de formule (XIa)

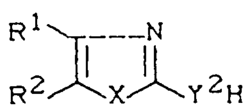

(XIa)

dans laquelle

$R^1$, $R^2$, X et $Y^2$ ont la définition indiquée ci-dessus,

ou des dérivés de thiazole ou d'oxazole de formule (XIb)

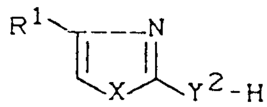

(XIb)

dans laquelle

$R^1$, X et $Y^2$ ont la définition indiquée ci-dessus

avec des esters bromacétiques de formule (XII)

$Br-CH_2-COOR^3$ (XII)

dans laquelle

$R^3$ a la définition indiquée ci-dessus,

le cas échéant en présence d'un diluant et en la présence éventuelle d'une substance auxiliaire de réaction à des températures comprises entre -20 °C et +150 °C puis, le cas échéant, on effectue leur substitution dans la position 5 du noyau de thiazole ou d'oxazole avec des réactions classiques de substitution électrophile ou on dérivatise à l'aide de procédés généralement connus des groupes fonctionnels déjà présents dans cette position.

**14.** Esters d'acides acryliques substitués de formule (VIII)

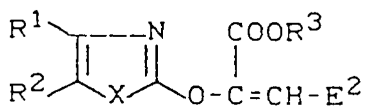

(VIII)

dans laquelle

$R^1$, $R^2$, $R^3$ et X ont la définition indiquée dans la revendication 1 et

$E^2$ est un groupe partant attirant les électrons.

**15.** Procédé de production d'esters d'acides acryliques substitués de formule (VIII)

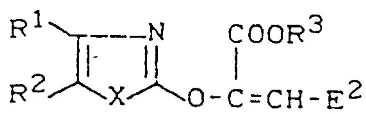

(VIII)

dans laquelle

$R^1$, $R^2$, $R^3$, X et $E^2$ ont la définition indiquée dans la revendication 14,

caractérisé en ce qu'on fait réagir des esters d'acides hydroxyacryliques de formule (IIb)

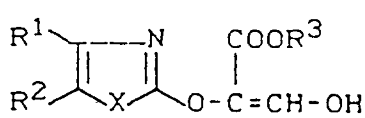

(IIb)

dans laquelle

$R^1$, $R^2$, $R^3$ et X ont la définition indiquée ci-dessus,

avec des chlorures d'acides de formule (XIV)

$R^{10}-Cl$ (XIV)

dans laquelle

$R^{10}$ est un reste acyle ou sulfonyle,

le cas échéant en présence d'un diluant et en la présence éventuelle d'un accepteur d'acide, à des températures comprises entre -20 °C et +120 °C.

**16.** Utilisation de composés de formules (II), (IVa) et (VIII) suivant les revendications 10, 12 et 14 comme produits intermédiaires.

**Revendications pour l'Etat contractant suivant : ES**

**1.** Procédé de production d'esters d'acides acryliques substitués de formule générale (I)

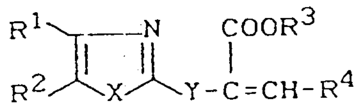

(I)

dans laquelle

$R^1$ représente de l'hydrogène, un groupe alkyle à chaîne droite ou ramifiée ayant 1 à 8 atomes de carbone, un groupe alcényle à chaîne droite ou ramifiée ayant 2 à 8 atomes de carbone, un groupe aralkyle ayant 1 à 6 atomes de carbone dans la partie alkyle à chaîne droite ou ramifiée, un groupe aralcényle ayant 2 à 6 atomes de carbone dans la partie alcényle à chaîne droite ou ramifiée ou un groupe aryle, avec chacun 6 à 10 atomes de carbone dans la partie aryle et portant chacun le cas échéant dans la partie aryle un ou plusieurs substituants identiques ou différents, en considérant dans chaque cas comme substituants de la partie aryle :
un halogène, un radical cyano, nitro, un radical alkyle, alkoxy ou alkylthio ayant chacun 1 à 4 atomes de carbone et étant chacun linéaire ou ramifié, un groupe halogénalkyle, halogénalkoxy ou halogénalkylthio ayant chacun 1 à 4 atomes de carbone et 1 à 9 atomes d'halogènes identiques ou différents et étant chacun linéaire ou ramifié, un groupe alkoxycarbonyle ou alkoximinoalkyle ayant chacun 1 à 8 atomes de carbone dans les parties alkyle individuelles et étant chacun linéaire ou ramifié, un groupe cycloalkyle ayant 3 à 7 atomes de carbone, un groupe alcanediyle à deux liaisons ayant 3 à 5 atomes de carbone, un groupe aryle, un groupe aralkyle, un groupe aryloxy ou un groupe aralkoxy ayant chacun 6 à 10 atomes de carbone dans la partie aryle et le cas échéant 1 à 4 atomes de carbone dans la partie alkyle linéaire ou ramifiée, portant chacun le cas échéant dans la partie aryle un ou plusieurs substituants, identiques ou différents, halogéno, alkyle, alkoxy, alkylthio, halogénalkyle, halogénalkoxy ou halogénalkylthio ayant chacun 1 à 4 atomes de carbone et le cas échéant 1 à 9 atomes d'halogènes identiques ou différents ou un groupe hétéroarylalkyle ou un groupe hétéroaryle ayant chacun 2 à 9 atomes de carbone et 1 à 4 hétéroatomes identiques ou différents dans la partie hétéroaryle et le cas échéant 1 à 4 atomes de carbone dans la partie alkyle à chaîne droite ou ramifiée, portant chacun le cas échéant dans la partie hétéroaryle un ou plusieurs substituants, identiques ou différents, halogéno, alkyle, alkoxy, alkylthio, halogénalkyle, halogénalkoxy ou halogénalkylthio ayant chacun 1 à 4 atomes de carbone et le cas échéant 1 à 9 atomes d'halogènes identiques ou différents ;

$R^1$ représente en outre un reste hétéroaryle de 2 à 9 atomes de carbone et 1 à 4 hétéroatomes identiques ou différents, portant le cas échéant un ou plusieurs substituants identiques ou différents, en considérant comme substituants les substituants de la partie aryle mentionnés ci-dessus,

$R^2$ représente du fluor, du chlore, du brome, de l'iode, un groupe cyano, nitro, formyle, un groupe halogénalkyle à chaîne droite ou ramifié ayant 1 à 4 atomes de carbone et 1 à 9 atomes d'halogènes identiques ou différents, un groupe alkoxyalkyle ou un groupe alkylthioalkyle linéaire ou ramifié portant chacun 1 à 4 atomes de carbone dans les parties alkyle individuelles, un groupe hydroxyminoalkyle, un groupe alkoximinoalkyle, un groupe N-alkyliminoalkyle ou un groupe N,N-dialkylhydrazonoalkyle ayant chacun 1 à 4 atomes de carbone dans les parties alkyle individuelles, chacun étant linéaire ou ramifié, un groupe alkoxy ou alkylthio linéaire ou ramifié ayant chacun 1 à 4 atomes de carbone, un groupe halogénalkoxy ou un groupe halogénalkylthio, linéaire ou ramifié, ayant chacun 1 à 4 atomes de carbone et chacun 1 à 9 atomes d'halogènes identiques ou différents, un groupe alcanoyle, alkoxycarbonyle ou N,N-dialkylcarbamoyle linéaire ou ramifié ayant chacun 1 à 4 atomes de carbone dans les parties alkyle individuelles ou un groupe hétérocyclylcarbonyle, en considérant comme reste hétérocyclyle un hétérocycle saturé pentagonal à heptagonal lié par de l'azote, qui peut contenir éventuellement un autre hétéroatome et qui peut porter le cas échéant 1 à 4 substituants méthyle et/ou éthyle ;

$R^2$ représente en outre un groupe N-aryliminoalkyle, aryloxy ou arylthio portant chacun le cas échéant un ou plusieurs substituants identiques ou différents dans la partie aryle avec chacun 6 à 10 atomes de carbone, la partie alkyle linéaire ou ramifiée ayant 1 à 4 atomes de carbone et en considérant dans chaque cas comme substituants de la partie aryle : un halogène, un radical cyano, nitro, un radical alkyle, alkoxy ou alkylthio linéaire ou ramifié ayant chacun 2 à 4 atomes de carbone, un radical halogénalkyle, halogénalkoxy ou halogénalkylthio linéaire ou ramifié ayant chacun 1 à 4 atomes de carbone et 1 à 9 atomes d'halogènes identiques ou différents, un groupe alkoxycarbonyle ou un groupe alkoximinoalkyle linéaire ou ramifié ayant chacun 1 à 4 atomes de carbone dans les parties alkyle individuelles, un groupe phényle, portant éventuellement un ou plusieurs substituants, identiques ou différents, halogéno et/ou alkyle linéaire ou ramifié ayant 1 à 4 atomes de carbone,

$R^3$ est un groupe alkyle linéaire ou ramifié ayant 1 à 6 atomes de carbone ou un groupe aralkyle ayant 1 à 4 atomes de carbone dans la partie alkyle linéaire ou ramifiée et 6 à 10 atomes de carbone dans la partie aryle, portant le cas échéant dans la partie aryle un ou plusieurs substituants identiques ou différents, en considérant comme substituants de la partie aryle ceux qui ont été mentionnés dans le cas de $R^1$,

$R^4$ est un groupe dialkylamino ayant 1 à 6 atomes de carbone dans les parties alkyle individuelles linéaires ou ramifiées ou représente un reste -Z-$R^5$,

X est de l'oxygène ou du soufre et

Y est de l'oxygène, du soufre ou un reste

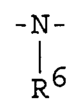

$R^5$ est un groupe alkyle à chaîne droite ou ramifiée ayant 1 à 6 atomes de carbone ou un groupe aralkyle ayant 1 à 4 atomes de carbone dans la partie alkyle linéaire ou ramifiée et 6 à 10 atomes de carbone dans la partie aryle, portant le cas échéant dans la partie aryle un ou plusieurs substituants identiques ou différents, en considérant comme substituants de la partie aryle ceux qui ont été mentionnés dans le cas de $R^1$,

$R^6$ représente de l'hydrogène, un groupe alkyle linéaire ou ramifié ayant 1 à 6 atomes de carbone, un groupe alcanoyle linéaire ou ramifié ayant 1 à 6 atomes de carbone dans la partie alkyle ou un groupe aralkyle portant éventuellement dans la partie aryle un ou plusieurs substituants identiques ou différents et ayant 1 à 6 atomes de carbone dans la partie alkyle linéaire ou ramifiée ou un groupe aryle, avec chacun dans la partie aryle correspondante 6 à 10 atomes de carbone, en considérant comme substituants de la partie aryle chacun des substituants mentionnés dans le cas de $R^1$ et

Z représente de l'oxygène ou du soufre,

leurs isomères géométriques et de leurs mélanges, caractérisé en ce que :

(a) on obtient des esters d'acides acryliques substitués de formule (Ia)

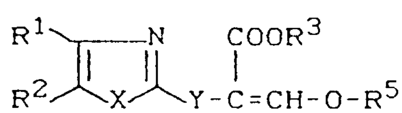

(I a)

dans laquelle

$R^1$, $R^2$, $R^3$, $R^5$, X et Y ont la définition indiquée ci-dessus,

en faisant réagir des esters d'acides hydroxyacryliques ou leurs sels de métaux alcalins de formule (II)

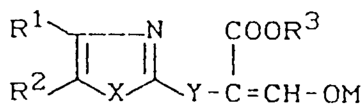 (II)

dans laquelle

M est de l'hydrogène ou un cation de métal alcalin et

$R^1$, $R^2$, $R^3$, X et Y ont la définition indiquée ci-dessus,

avec des agents d'alkylation de formule (III)

$R^5$-$E^1$ (III)

dans laquelle

$E^1$ est un groupe partant attirant les électrons et

$R^5$ a la définition indiquée ci-dessus,

éventuellement en présence d'un diluant et en la présence éventuelle d'une substance auxiliaire de réaction, ou bien

(b) on obtient des esters d'acides acryliques substitués de formule (Ib)

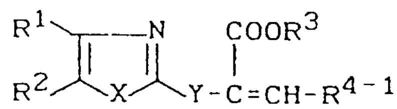 (Ib)

dans laquelle

$R^{4-1}$ est un groupe dialkylamino et

$R^1$, $R^2$, $R^3$, X et Y ont la définition indiquée ci-dessus,

en faisant réagir des esters d'acides acétiques substitués de formule (IV)

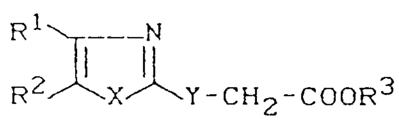 (IV)

dans laquelle

$R^1$, $R^2$, $R^3$, X et Y ont la définition indiquée ci-dessus,

avec des formamides de formule (Va)

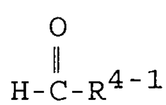 (Va)

dans laquelle

$R^{4-1}$ a la définition indiquée ci-dessus,

ou avec des dérivés de formule (Vb),

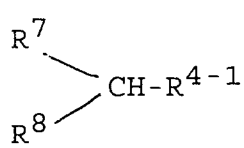 (Vb)

dans laquelle

$R^7$ et $R^8$ représentent chacun, indépendamment l'un de l'autre, un groupe alkoxy ou dialkylamino et

$R^{4-1}$ a la définition indiquée ci-dessus,

le cas échéant en présence d'un diluant, ou bien

(c) on obtient des esters d'acides acryliques substitués de formule (Ic)

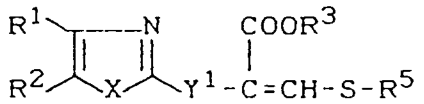

(Ic)

dans laquelle

$Y^1$ représente du soufre ou un reste

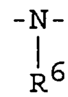

et

$R^1$, $R^2$, $R^3$, $R^5$, $R^6$ et X ont la définition indiquée ci-dessus,

en faisant réagir des dérivés d'acide oxalique de formule (VI)

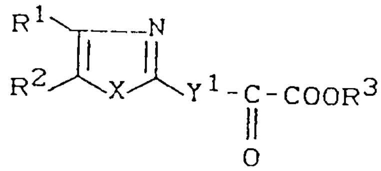

(VI)

dans laquelle

$R^1$, $R^2$, $R^3$, X et $Y^1$ ont la définition indiquée ci-dessus,

avec des composés organométalliques de formule (VII)

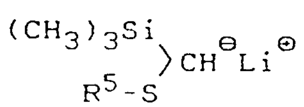

(VII)

dans laquelle

$R^5$ a la définition indiquée ci-dessus,

le cas échéant en présence d'un diluant, ou bien

(d) on obtient des esters d'acides acryliques substitués de formule (Id)

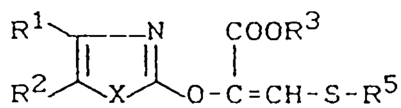

(Id)

dans laquelle

$R^1$, $R^2$, $R^3$, $R^5$ et X ont la définition indiquée ci-dessus,

en faisant réagir des esters d'acides acryliques substitués de formule (VIII)

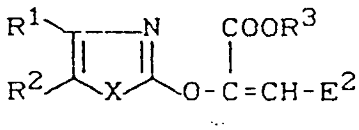

(VIII)

dans laquelle

$E^2$ est un groupe partant attirant les électrons et

$R^1$, $R^2$, $R^3$ et X ont la définition indiquée ci-dessus,

avec des thiols de formule (IX)

$R^5$-SH (IX)

dans laquelle

$R^5$ a la définition indiquée ci-dessus,

le cas échéant en présence d'un diluant et en la présence éventuelle d'une substance auxiliaire de réaction.

2. Procédé suivant la revendication 1, dans lequel

$R^1$ représente de l'hydrogène, un groupe méthyle, éthyle, n-propyle, isopropyle, n-butyle, isobutyle, sec.-butyle, tertio-butyle, allyle, n-butényle ou isobutényle, un groupe benzyle, phényléthyle, phényléthényle, phényle, naphtyle, pyridyle, thiényle ou furyle portant chacun le cas échéant dans la partie aryle ou dans la partie hétéroaryle 1 à 4 substituants identiques ou différents, en considérant dans chaque cas comme substituants : le fluor, le chlore, le brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, trifluorométhyle, trifluorométhoxy, difluorométhoxy, trifluorométhylthio, méthoxycarbonyle, éthoxycarbonyle, méthoximinométhyle, éthoximinométhyle, méthoximinoéthyle, éthoximinoéthyle, cyclopentyle, cyclohexyle, 1,3-propanediyle, 1,4-butanediyle ou un groupe phényle, benzyle, phénoxy ou benzyloxy portant chacun le cas échéant dans la partie phényle 1 à 3 substituants, identiques ou différents, fluoro, chloro, bromo, méthyle, éthyle, méthoxy, éthoxy, méthylthio, trifluorométhyle, difluorométhoxy, trifluorométhoxy et/ou trifluorométhylthio,

$R^2$ représente du chlore, du brome, de l'iode, un groupe cyano, nitro, formyle, chlorométhyle, bromométhyle, iodométhyle, méthoxyméthyle, éthoxyméthyle, n-propoxyméthyle, isopropoxyméthyle, méthylthiométhyle, éthylthiométhyle, n-propylthiométhyle, isopropylthiométhyle, hydroximinométhyle, hydroximinoéthyle, hydroximinopropyle, méthoximinométhyle, éthoximinométhyle, n-propoximinométhyle, isopropoximinométhyle, méthoximinoéthyle, éthoximinoéthyle, n-propoximinoéthyle, isopropoximinoéthyle, méthyliminométhyle, éthyliminométhyle, n-propyliminométhyle, isopropyliminométhyle, méthyliminoéthyle, éthyliminoéthyle, n-propyliminoéthyle, isopropyliminoéthyle, N,N-diméthylhydrazonométhyle, N,N-diéthylhydrazonométhyle, N-méthyl-N-éthylhydrazonométhyle, N-méthyl-N-propylhydrazonométhyle,N,N-dipropylhydrazonométhyle, N,N-diméthylhydrazonoéthyle, N,N-diéthylhydrazonoéthyle, méthoxy, éthoxy, n-propoxy, isopropoxy, méthylthio, éthylthio, n-propylthio, isopropylthio, trifluorométhoxy, difluorométhoxy, dichlorofluorométhoxy, difluorochlorométhoxy, trichlorométhoxy, trifluorométhylthio, trichlorométhylthio, difluorométhylthio, dichlorofluorométhylthio, difluorochlorométhylthio, acétyle, propionyle, butyryle, méthoxycarbonyle, éthoxycarbonyle, n-propoxycarbonyle, isopropoxycarbonyle, N,N-diméthylaminocarbonyle, N,N-diéthylaminocarbonyle, N,N-dipropylaminocarbonyle, N-méthyl-N-éthylaminocarbonyle, N-méthyl-N-propylaminocarbonyle, 1-pipéridinylcarbonyle, 4-morpholinylcarbonyle ou un groupe N-phényliminométhyle, N-phényliminoéthyle, phénoxy ou phénylthio portant chacun le cas échéant dans la partie phényle 1 à 3 substituants identiques ou différents, en considérant dans chaque cas comme substituants : le fluor, le chlore, le brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, trifluorométhyle, trifluorométhoxy, trifluorométhylthio, méthoxycarbonyle, éthoxycarbonyle, méthoximinométhyle, éthoximinométhyle, méthoximinoéthyle, éthoximinoéthyle ou un groupe phényle portant le cas échéant 1 à 3 substituants fluoro, chloro, bromo, méthyle et/ou éthyle identiques ou différents,

$R^3$ est un groupe méthyle, éthyle, n-propyle, isopropyle, n-butyle, isobutyle, sec.-butyle ou tertio-butyle ou un groupe benzyle portant le cas échéant 1 à 3 substituants identiques ou différents, en considérant comme substituants ceux qui ont été mentionnés pour $R^1$,

$R^4$ est un groupe dialkylamino ayant 1 à 4 atomes de carbone dans chacune des parties alkyle individuelles linéaires ou ramifiées ou un reste -Z-$R^5$,

X est de l'oxygène ou du soufre et

Y est de l'oxygène, du soufre ou un reste

$$-\underset{\displaystyle R^6}{\underset{|}{N}}- ,$$

$R^5$ est un groupe méthyle, éthyle, n-propyle, isopropyle, n-butyle, isobutyle, sec.-butyle, tertio-butyle ou un reste benzyle portant le cas échéant 1 à 3 substituants identiques ou différents, en considérant comme substituants ceux qui ont été mentionnés pour $R^1$ ;

$R^6$ est de l'hydrogène, un groupe méthyle, éthyle, n-propyle, isopropyle, n-butyle, isobutyle, sec.-butyle, tertio-butyle, acétyle, propionyle, n-butyryle, isobutyryle ou un reste benzyle ou phényle portant chacun le cas échéant 1 à 3 substituants identiques ou différents, en considérant comme substituants ceux qui ont été mentionnés pour $R^1$ et

Z est de l'oxygène ou du soufre.

**3.** Procédé suivant la revendication 1, dans lequel

$R^1$ représente de l'hydrogène, un groupe méthyle, éthyle, n-propyle, isopropyle ou un groupe phényle ou naphtyle portant chacun le cas échéant 1 à 4 substituants identiques ou différents, en considérant dans chaque cas comme subtituants : le fluor, le chlore, le brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, trifluorométhyle, trifluorométhoxy, difluorométhoxy, trifluorométhylthio, méthoxycarbonyle, éthoxycarbonyle, méthoximinométhyle, éthoximinométhyle, méthoximinoéthyle, éthoximinoéthyle, cyclopentyle, cyclohexyle, 1,3-propanediyle, 1,4-butanediyle ou un reste phényle, phénoxy, benzyle ou benzyloxy portant chacun le cas échéant 1 ou 2 substituants, identiques ou différents, fluoro, chloro, bromo, méthyle et/ou éthyle,

$R^3$ représente du chlore, du brome, un radical cyano, nitro, formyle, méthoxyméthyle, éthoxyméthyle, méthylthiométhyle, hydroximinométhyle, méthoximinométhyle, éthoximinométhyle, méthyliminométhyle, éthyliminométhyle, N,N-diméthylhydrazonométhyle, N,N-diéthylhydrazonométhyle, méthoxy, éthoxy, méthylthio, éthylthio, trifluorométhylthio, dichlorofluorométhylthio, difluorochlorométhylthio, trichlorométhylthio, acétyle, méthoxycarbonyle, éthoxycarbonyle, N,N-diméthylaminocarbonyle, N,N-diéthylaminocarbonyle, N-méthyl-N-éthylaminocarbonyle ou un reste phénylthio portant le cas échéant 1 ou 2 substituants identiques ou différents, en considérant comme substituants : le fluor, le chlore, le brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, trifluorométhyle, trifluorométhoxy, trifluorométhylthio,

$R^3$ est un groupe méthyle, éthyle ou benzyle,

$R^4$ est un groupe diméthylamino, diéthylamino ou un reste -Z-$R^5$,

X est de l'oxygène ou du soufre et

Y est de l'oxygène, du soufre ou un reste

$$-\underset{\displaystyle R^6}{\underset{|}{N}}- ,$$

$R^5$ est un groupe méthyle, éthyle, n-propyle, isopropyle ou benzyle,

$R^6$ est de l'hydrogène, un groupe méthyle, éthyle, acétyle, propionyle, ou un groupe benzyle ou phényle portant chacun le cas échéant un ou deux substituants fluoro, chloro, bromo, méthyle, éthyle et/ou trifluorométhyle, identiques ou différents et

Z est de l'oxygène ou du soufre.

**4.** Procédé suivant la revendication 1, dans lequel

$R^1$ représente de l'hydrogène, un groupe méthyle, éthyle, n-propyle, isopropyle ou un groupe phényle portant le cas échéant un ou deux substituants identiques ou différents, en considérant comme substituants : le fluor, le chlore, le brome, un radical cyano, nitro, méthyle, éthyle, méthoxy, éthoxy, méthylthio, trifluorométhyle, trifluorométhoxy, trifluorométhylthio, méthoxycarbonyle, éthoxycarbonyle, méthoximinométhyle, cyclopentyle, 1,3-propanediyle, méthoximinométhyle ou un groupe phényle, phénoxy, benzyle ou benzyloxy portant le cas échéant un ou deux substituants fluoro, chloro, bromo ou méthyle identiques ou différents,

$R^2$ est du chlore, du brome, un groupe cyano ou nitro, formyle, méthoxyméthyle, méthylthiométhyle, méthoxycarbonyle, éthoxycarbonyle, méthylthio, trifluorométhylthio ou méthoximinométhyle,

$R^3$ est un groupe méthyle ou éthyle,

$R^4$ est un groupe méthoxy, éthoxy, méthylthio, ou diméthylamino,

X est de l'oxygène ou du soufre et

Y est un reste N-méthyle.

**5.** Compositions pesticides, caractérisées par une teneur en au moins un ester d'acide acrylique substitué de formule (I) suivant les revendications 1 à 4.

**6.** Utilisation d'esters d'acides acryliques substitués de formule (I) suivant les revendications 1 à 4 pour combattre des parasites.

**7.** Procédé pour combattre des parasites, caractérisé en ce qu'on fait agir sur des parasites et/ou sur leur milieu des esters d'acides acryliques substitués de formule (I) suivant les revendications 1 à 4.

**8.** Procédé de préparation de compositions pesticides, caractérisé en ce qu'on mélange des esters d'acides acryliques substitués de formule (I) suivant les revendications 1 à 4 avec des diluants et/ou des agents tensio-actifs.

**9.** Procédé de production d'esters d'acides hydroxyacryliques ou de leurs sels de métaux alcalins de formule (II)

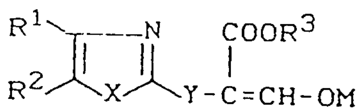

(II)

dans laquelle

$R^1$, $R^2$, $R^3$, X et Y ont la définition indiquée dans la revendication 1 et

M représente de l'hydrogène ou un cation de métal alcalin,

caractérisé en ce qu'on fait réagir des esters d'acides acétiques substitués de formule (IV)

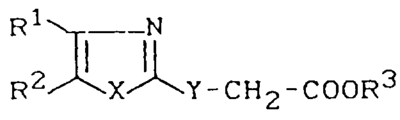

(IV)

dans laquelle

$R^1$, $R^2$, $R^3$, X et Y ont la définition indiquée ci-dessus,

avec des esters d'acide formique de formule (X)

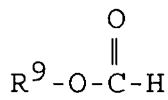 (X)

dans laquelle

$R^9$ est un groupe alkyle,

le cas échéant en présence d'un diluant et en la présence éventuelle d'une substance auxiliaire basique de réaction, à des températures comprises entre -20 ° C et + 50 ° C.

**10.** Procédé de production d'esters d'acides acétiques substitués de formule (IVa)

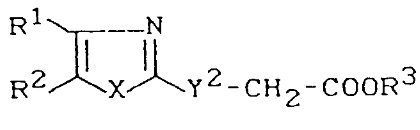 (IVa)

dans laquelle

$R^1$, $R^2$, $R^3$ et X ont la définition indiquée dans la revendication 1 et

$Y^2$ représente de l'oxygène, du soufre ou un reste N-alkyle,

caractérisé en ce qu'on fait réagir des dérivés de thiazole ou d'oxazole de formule (XIa)

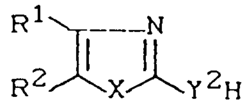 (XIa)

dans laquelle

$R^1$, $R^2$, X et $Y^2$ ont la définition indiquée ci-dessus,

ou des dérivés de thiazole ou d'oxazole de formule (XIb)

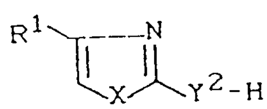 (XIb)

dans laquelle

$R^1$, X et $Y^2$ ont la définition indiquée ci-dessus

avec des esters bromacétiques de formule (XII)

$Br-CH_2-COOR^3$ (XII)

dans laquelle

$R^3$ a la définition indiquée ci-dessus,

le cas échéant en présence d'un diluant et en la présence éventuelle d'une substance auxiliaire de réaction à des températures comprises entre -20 ° C et + 150 ° C puis, le cas échéant, on effectue leur substitution dans la position 5 du noyau de thiazole ou d'oxazole avec des réactions classiques de substitution électrophile ou on dérivatise à l'aide de procédés généralement connus des groupes fonctionnels déjà présents dans cette position.

**11.** Procédé de production d'esters d'acides acryliques substitués de formule (VIII)

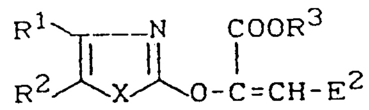

(VIII)

dans laquelle

$R^1$, $R^2$, $R^3$ et X ont la définition indiquée dans la revendication 1 et

$E^2$ est un groupe partant attirant les électrons,

caractérisé en ce qu'on fait réagir des esters d'acides hydroxyacryliques de formule (IIb)

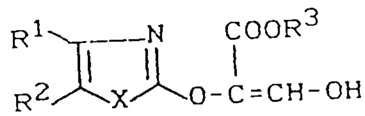

(IIb)

dans laquelle

$R^1$, $R^2$, $R^3$ et X ont la définition indiquée ci-dessus,

avec des chlorures d'acides de formule (XIV)

$R^{10}$-Cl (XIV)

dans laquelle

$R^{10}$ est un reste acyle ou sulfonyle,

le cas échéant en présence d'un diluant et en la présence éventuelle d'un accepteur d'acide, à des températures comprises entre -20 °C et +120 °C.

**12.** Utilisation de composés de formules (II), (IVa) et (VIII) suivant les revendications 9, 10 et 11 comme produits intermédiaires.